# EUROPEAN PATENT APPLICATION

(11) **EP 3 235 820 A1**
(43) Date of publication of application: **25.10.2017**
(21) Application number: 17170370.5
(22) Date of filing: 17.09.2015
(51) Int. Cl.: C07D 487/04, A61K 39/44, A61P 35/00

(54) **PYRROLOBENZODIAZEPINES AND ANTIBODY DISULFIDE CONJUGATES THEREOF**

(30) Priority: 17.09.2014 US 201462051387 P
(62) Divisional of application: 15772143.2
(71) Applicant: GENENTECH, INC., South San Francisco, CA 94080-4990 (US)
(72) Inventor: FLYGARE, John A., South San Francisco, CA California 94080-4990 (US); PILLOW, Thomas H., South San Francisco, CA California 94080-4990 (US)
(74) Representative: Watson, Robert James

(57) **Abstract**

A compound of formula I: 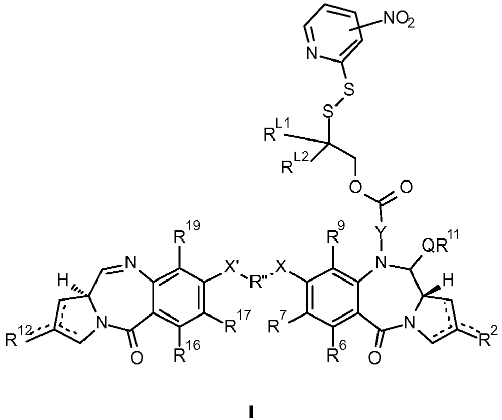 wherein Y is selected from a single bond, and a group of formulae A1 or A2: 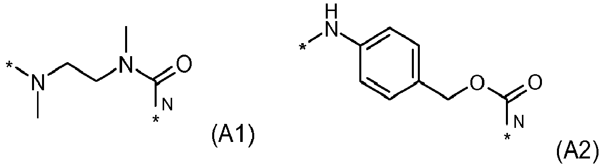 where N shows where the group binds to the N10 of the PBD moiety.

## Description

The present invention relates to pyrrolobenzodiazepines (PBDs), in particular pyrrolobenzodiazepines having a labile N10 protecting group, suitable to form a linker to a cell binding agent. The present invention also relates to certain conjugates made from these PBDs.

### Background to the invention

### Pyrrolobenzodiazepines

Some pyrrolobenzodiazepines (PBDs) have the ability to recognise and bond to specific sequences of DNA; the preferred sequence is PuGPu. The first PBD antitumour antibiotic, anthramycin, was discovered in 1965 (Leimgruber, et al., J. Am. Chem. Soc., 87, 5793-5795 (1965); Leimgruber, et al., J. Am. Chem. Soc., 87, 5791-5793 (1965)). Since then, a number of naturally occurring PBDs have been reported, and over 10 synthetic routes have been developed to a variety of analogues (Thurston, et al., Chem. Rev. 1994, 433-465 (1994); Antonow, D. and Thurston, D.E., Chem. Rev. 2011 111 (4), 2815-2864). Family members include abbeymycin (Hochlowski, et al., J. Antibiotics, 40, 145-148 (1987)), chicamycin (Konishi, et al., J. Antibiotics, 37, 200-206 (1984)), DC-81 (Japanese Patent 58-180 487; Thurston, et al., Chem. Brit., 26, 767-772 (1990); Bose, et al., Tetrahedron, 48, 751-758 (1992)), mazethramycin (Kuminoto, et al., J. Antibiotics, 33, 665-667 (1980)), neothramycins A and B (Takeuchi, et al., J. Antibiotics, 29, 93-96 (1976)), porothramycin (Tsunakawa, et al., J. Antibiotics, 41, 1366-1373 (1988)), prothracarcin (Shimizu, et al, J. Antibiotics, 29, 2492-2503 (1982); Langley and Thurston, J. Org. Chem., 52, 91-97 (1987)), sibanomicin (DC-102)(Hara, et al., J. Antibiotics, 41, 702-704 (1988); Itoh, et al., J. Antibiotics, 41, 1281-1284 (1988)), sibiromycin (Leber, et al., J. Am. Chem. Soc., 110, 2992-2993 (1988)) and tomamycin (Arima, et al., J. Antibiotics, 25, 437-444 (1972)). PBDs are of the general structure:

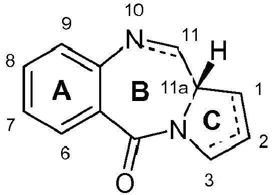

They differ in the number, type and position of substituents, in both their aromatic A rings and pyrrolo C rings, and in the degree of saturation of the C ring. In the B-ring there is either an imine (N=C), a carbinolamine(NH-CH(OH)), or a carbinolamine methyl ether (NH-CH(OMe)) at the N10-C11 position which is the electrophilic centre responsible for alkylating DNA. All of the known natural products have an (*S*)-configuration at the chiral C11a position which provides them with a right-handed twist when viewed from the C ring towards the A ring. This gives them the appropriate three-dimensional shape for isohelicity with the minor groove of B-form DNA, leading to a snug fit at the binding site (Kohn, In Antibiotics III. Springer-Verlag, New York, pp. 3-11 (1975); Hurley and Needham-VanDevanter, Acc. Chem. Res., 19, 230-237 (1986)). Their ability to form an adduct in the minor groove, enables them to interfere with DNA processing, hence their use as antitumour agents.

Dimeric PBD compounds bearing C2 aryl substituents are disclosed in WO 2005/085251, such as:

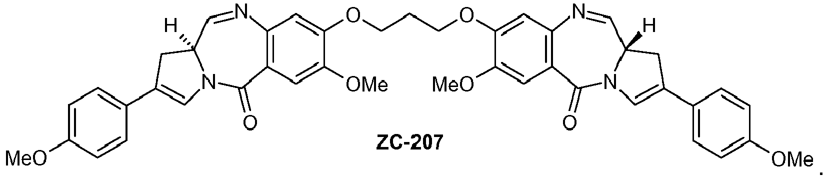

These compounds have been shown to be highly useful cytotoxic agents.

A particularly advantageous pyrrolobenzodiazepine compound is described by Gregson et al. (Chem. Commun. 1999, 797-798) as compound **1**, and by Gregson et al. (J. Med. Chem. 2001, 44, 1161-1174) as compound **4a.** This compound, also known as SJG-136, is shown below:

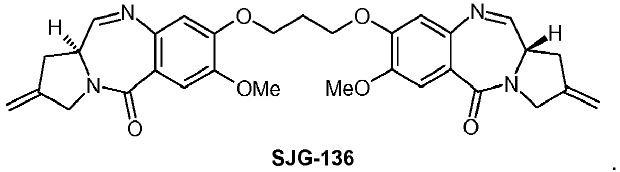

### Antibody-drug conjugates

Antibody therapy has been established for the targeted treatment of patients with cancer, immunological and angiogenic disorders (Carter, P. (2006) Nature Reviews Immunology 6:343-357). The use of antibody-drug conjugates (ADC), i.e. immunoconjugates, for the local delivery of cytotoxic or cytostatic agents, i.e. drugs to kill or inhibit tumor cells in the treatment of cancer, targets delivery of the drug moiety to tumors, and intracellular accumulation therein, whereas systemic administration of these unconjugated drug agents may result in unacceptable levels of toxicity to normal cells as well as the tumor cells sought to be eliminated (Xie et al (2006) Expert. Opin. Biol. Ther. 6(3):281-291; Kovtun et al (2006) Cancer Res. 66(6):3214-3121; Law et al (2006) Cancer Res. 66(4):2328-2337; Wu et al (2005) Nature Biotech. 23(9):1137-1145; Lambert J. (2005) Current Opin. in Pharmacol. 5:543-549; Hamann P. (2005) Expert Opin. Ther. Patents 15(9):1087-1103; Payne, G. (2003) Cancer Cell 3:207-212; Trail et al (2003) Cancer Immunol. Immunother. 52:328-337; Syrigos and Epenetos (1999) Anticancer Research 19:605-614).

Maximal efficacy with minimal toxicity is sought thereby. Efforts to design and refine ADC have focused on the selectivity of monoclonal antibodies (mAbs) as well as drug mechanism of action, drug-linking, drug/antibody ratio (loading), and drug-releasing properties (Junutula, et al., 2008b Nature Biotech., 26(8):925-932; Dornan et al (2009) Blood 114(13):2721-2729; US 7521541; US 7723485; WO2009/052249; McDonagh (2006) Protein Eng. Design & Sel. 19(7): 299-307; Doronina et al (2006) Bioconj. Chem. 17:114-124; Erickson et al (2006) Cancer Res. 66(8):1-8; Sanderson et al (2005) Clin. Cancer Res. 11:843-852; Jeffrey et al (2005) J. Med. Chem. 48:1344-1358; Hamblett et al (2004) Clin. Cancer Res. 10:7063-7070). Drug moieties may impart their cytotoxic and cytostatic effects by mechanisms including tubulin binding, DNA binding, or topoisomerase inhibition. Some cytotoxic drugs tend to be inactive or less active when conjugated to large antibodies or protein receptor ligands.

WO 2013/055987 discloses conjugates comprising a PBD dimer compound connected through the N10 position via a specific sulfur linker to a cell binding agent, having a general formula A:

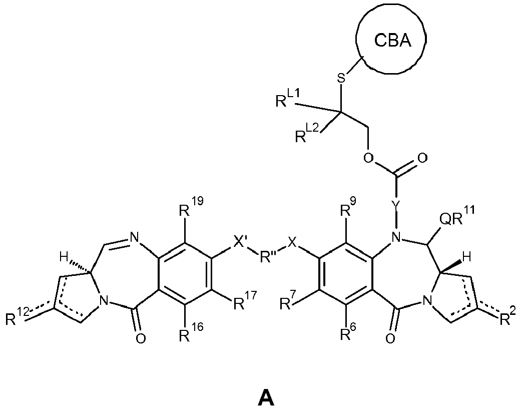

These conjugates were exemplified with A118C cysteine-engineered antibody mutants (THIOMAB™).

Drug linkers of formula D were disclosed as useful in the preparation of such conjugates:

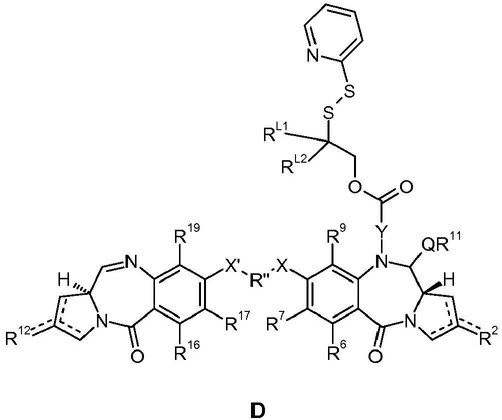

The present inventors have developed further drug linkers, which are useful in the synthesis of the conjugate compounds disclosed in WO 2013/055987.

### Summary of the Invention

In a general aspect the present invention provides drug linker compounds useful in the preparation of conjugates, the drug linkers comprising a PBD dimer compound connected through the N10 position via a specific sulfur linker to a nitro-pyridyl group, and methods of using the drug linker compounds to prepare conjugates.

In a first aspect, the present invention provides compounds of formula I: 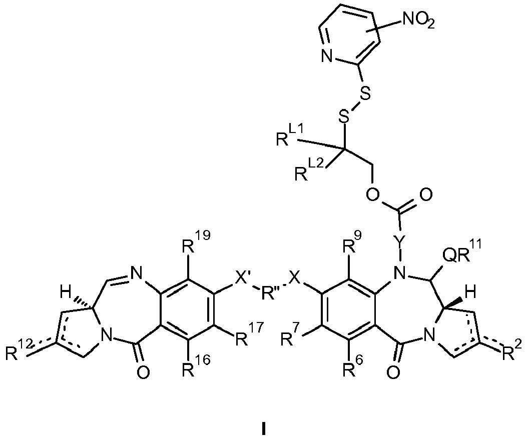 and salts and solvates thereof, wherein
the dotted lines indicate the optional presence of a double bond between C1 and C2 or C2 and C3;
R² is independently selected from H, OH, =O, =CH₂, CN, R, OR, =CH-R^{D}, =C(R^{D})₂, O-SO₂-R, CO₂R and COR, and optionally further selected from halo or dihalo;
where R^{D} is independently selected from R, CO₂R, COR, CHO, CO₂H, and halo;
R⁶ and R⁹ are independently selected from H, R, OH, OR, SH, SR, NH₂, NHR, NRR', NO₂, Me₃Sn and halo;
R⁷ is independently selected from H, R, OH, OR, SH, SR, NH₂, NHR, NRR', NO₂, Me₃Sn and halo;
Y is selected from a single bond, and a group of formulae A1 or A2: 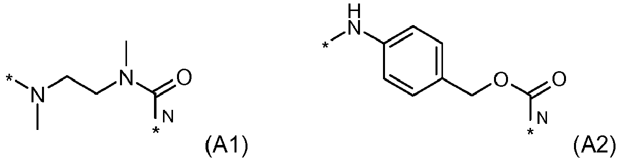 where N shows where the group binds to the N10 of the PBD moiety;
R^{L1} and R^{L2} are independently selected from H and methyl, or together with the carbon atom to which they are bound form a cyclopropylene group;
Q is independently selected from O, S and NH;
R¹¹ is either H, or R or, where Q is O, SO₃M, where M is a metal cation;
R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
wherein R¹², R¹⁶, R¹⁹ and R¹⁷ are as defined for R², R⁶, R⁹ and R⁷ respectively;
wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted; and
X and X' are independently selected from O, S and N(H).

Thus formula I is selected from the following formulae Ia, Ib and Ic, depending on Y:

| Y | I |
|---|---|
| Single bond | 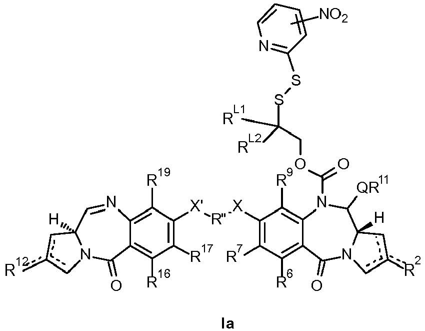 |
| 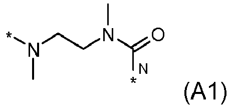 | 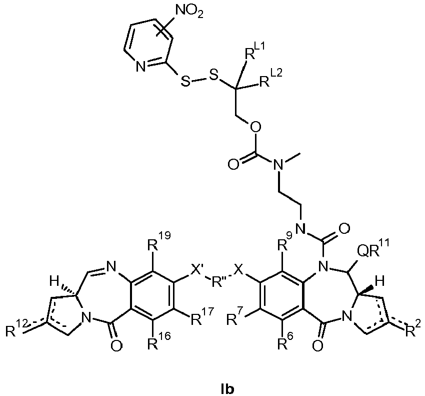 |
| 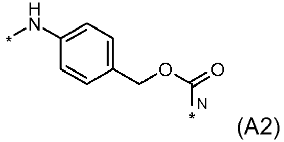 | 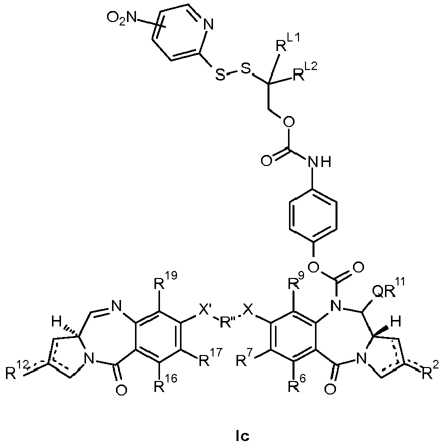 |

In one embodiment, the present invention provides compounds of formula II: 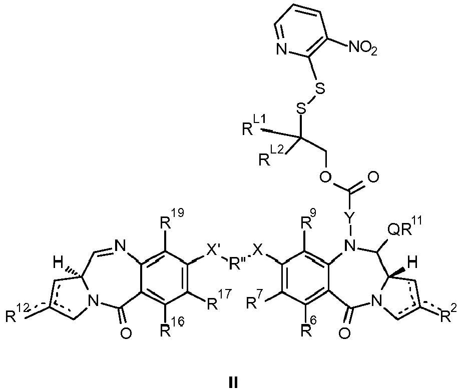 and salts and solvates thereof, wherein the dotted lines indicate the optional presence of a double bond between C1 and C2 or C2 and C3;
and all substituents are as defined above.

In another embodiment, the present invention provides novel compounds of formula III: 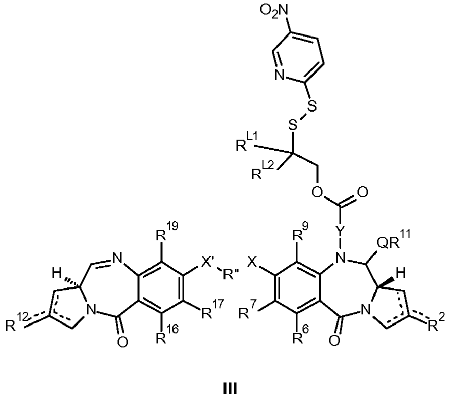 and salts and solvates thereof, wherein the dotted lines indicate the optional presence of a double bond between C1 and C2 or C2 and C3;
and all substituents are as defined above.

These drug linker may exhibit various advantages over those disclosed in WO 2013/055987, such as being easier to conjugate to a cell binding agent. Such ease of conjugation can relate to more rapid and higher yield. Without being limited to a particular mechanism or effect, the nitro group on the pyridyl ring of formula I compounds provides an electron-withdrawing effect which accelerates reaction with a cysteine thiol of a cysteine-engineered antibody. Where the cysteine thiol has been introduced at a hindered or less-reactive site on the antibody, the compounds of formula I provide more efficient conjugation relative to a corresponding unsubstituted pyridyl analog of a compound of formula I.

In a second aspect, the present invention provides methods of making conjugate compounds of formula A from drug linkers of the first aspect of the invention, by reacting a compound of the first aspect of the invention with a cell binding agent, wherein formula A is: 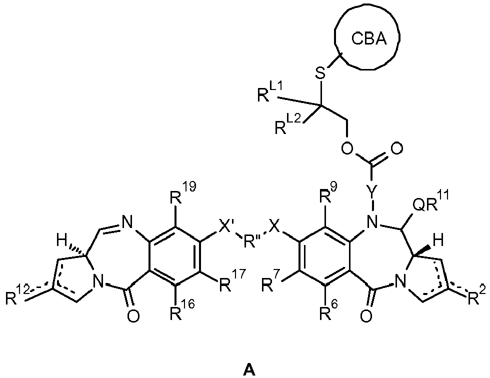
the dotted lines indicate the optional presence of a double bond between C1 and C2 or C2 and C3;
CBA represents a cell binding agent;
and the remaining groups are as defined in the first aspect of the invention.

In compounds of formula A: 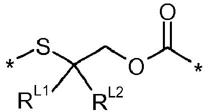 is the sulfur linking group.

In the compounds above, the 5-membered rings represented by 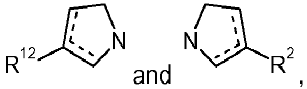 may be replaced by a ring selected from: 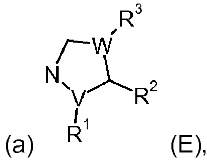 where R² with either of R¹ or R³, together with the carbon atoms of the C ring to which they are attached, form an optionally substituted benzene ring;
V and W are each selected from (CH₂)ₙ, O, S, NR, CHR, and CRR' where n is 1, 2 or 3, except that V is C when R¹ and R², together with the carbon atoms of the C ring to which they are attached, form an optionally substituted benzene ring, and W is C when R³ and R², together with the carbon atoms of the C ring to which they are attached, form an optionally substituted benzene ring; and 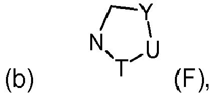 where T is selected from CH₂, NR, CO, BH, SO, and SO₂;
U is selected from CH₂, NR, O and S;
Y is (CH₂)ₙ, where n is 1, 2, 3 or 4;
except that T, U and Y are not all CH₂.

A third aspect of the present invention provides conjugates of formula A1: 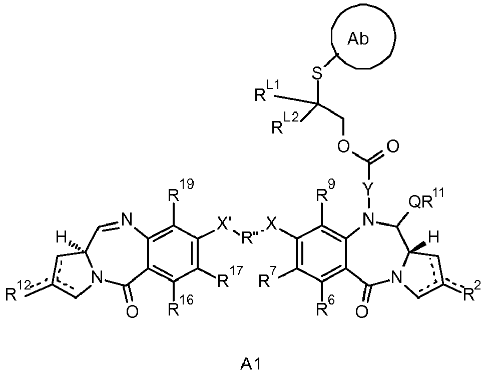
the dotted lines indicate the optional presence of a double bond between C1 and C2 or C2 and C3;
Ab represents a cysteine-engineered antibody mutant (THIOMAB™) selected from the group consisiting of:
   (a) LC K149C cysteine-engineered antibody mutant (THIOMAB™);
   (b) HC A140C cysteine-engineered antibody mutant (THIOMAB™);
   (c) LC V205C cysteine-engineered antibody mutant (THIOMAB™); and
   (d) HC S239C cysteine-engineered antibody mutant (THIOMAB™); and the remaining groups are as defined in the first aspect of the invention.

A fourth aspect of the present invention provides the use of a conjugate of the first aspect of the invention in a method of medical treatment. The fourth aspect also provides a pharmaceutical composition comprising a conjugate of the first aspect, and a pharmaceutically acceptable excipient.

A fifth aspect of the present invention provides a conjugate of the first aspect of the invention or a pharmaceutical composition of the fourth aspect of the invention for use in a method of treatment of a proliferative disease. The fifth aspect also provides the use of a conjugate of the first aspect in a method of manufacture of a medicament for the treatment of a proliferative disease, and a method of treating a mammal having a proliferative disease, comprising administering an effective amount of a conjugate of the first aspect or a pharmaceutical composition of the fourth aspect.

### Detailed Description of the Invention

The present invention provides a compound (drug-linker) comprising a PBD dimer connected through the N10 position on one of the PBD moieties via the specified linker to leaving group, wherein the pyridine ring is substituted with a nitro group.

The present invention also provides a method of preparing a conjugate from drug-linker compounds, the method comprising the step of reacting a cell binding agent with a drug-linker compound. In some embodiments, the cell binding agent is an antibody.

The conjugates so formed can deliver a PBD compound to a preferred site in a subject. The conjugate allows the release of an active PBD compound that does not retain any part of the linker. There is no stub present that could affect the reactivity of the PBD compound.

### Preferences

The following preferences may apply to all aspects of the invention as described above, or may relate to a single aspect. The preferences may be combined together in any combination.

### Preferred Drug Linkers

In a first aspect, the present invention provides dug linkers for use in the preparation of the conjugate compounds described herein.

Preferred intermediates are described below, and correspond closely to the preferred conjugates described herein.

In one embodiment, the compound is a dimer wherein each of the PBD moieties has a C2 methylene group i.e. each R² is =CH₂.

In another embodiment, the compound is a dimer wherein each of the monomers has a C2 aryl group i.e. each R² is optionally substituted C₅₋₂₀ aryl, and there is a double bond between C2 and C3 in each PBD moiety.

### C2 Alkylene

In one embodiment, the compound is: 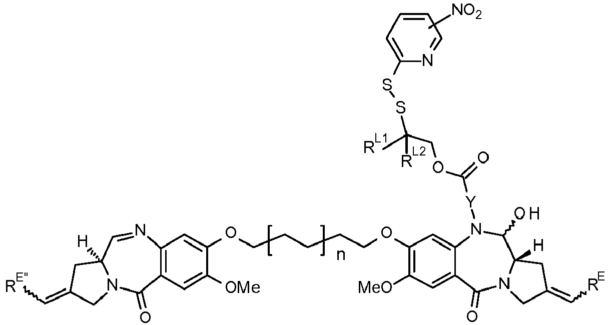 more preferably: 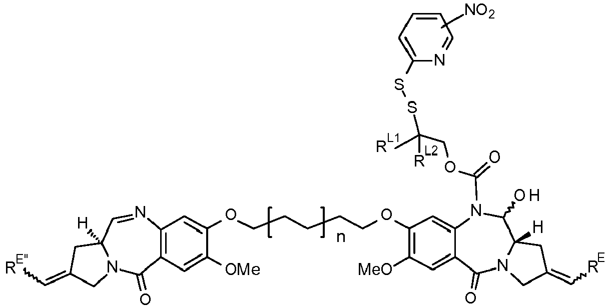
where n is 0 or 1; and
Y, R^{L1} and R^{L2} are as previously defined, and R^{E} and R^{E"} are each independently selected from H or R^{D}.

For each of the compounds above, the following preferences may apply, where appropriate:
n is 0;
n is 1;
R^{E} is H;
R^{E} is R^{D}, where R^{D} is optionally substituted alkyl;
R^{E} is R^{D}, where R^{D} is methyl;
R^{L1} and R^{L2} are H;
R^{L1} and R^{L2} are Me.

### C2 Aryl

In one embodiment, the compound is: 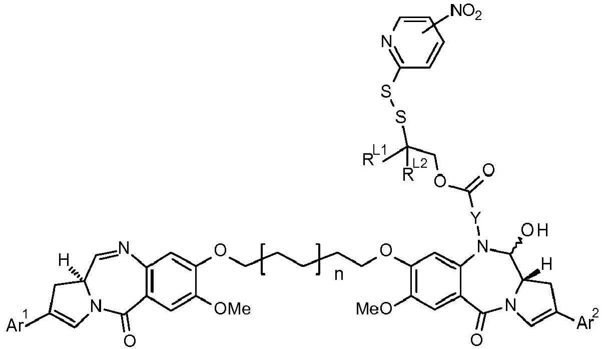 more preferably: 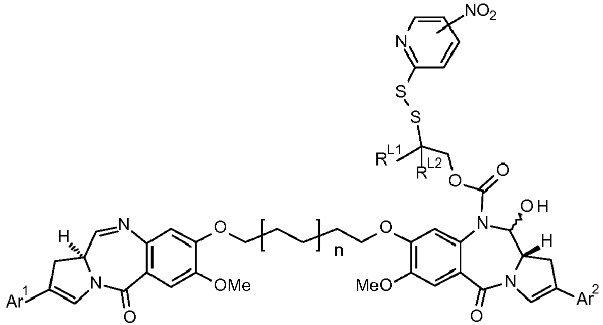
wherein Y, R^{L1} and R^{L2} are as previously defined;
Ar¹ and Ar² are each independently optionally substituted C₅₋₂₀ aryl, and
n is 0 or 1. Ar¹ and Ar² may be the same or different.

In one embodiment, Ar¹ and Ar² in each of the embodiments above are each independently selected from optionally substituted phenyl, furanyl, thiophenyl and pyridyl.

In one embodiment, Ar¹ and Ar² in each of the embodiments above is optionally substituted phenyl.

In one embodiment, Ar¹ and Ar² in each of the embodiments above is optionally substituted thien-2-yl or thien-3-yl.

In one embodiment, Ar¹ and Ar² in each of the embodiments above is optionally substituted quinolinyl or isoquinolinyl.

The quinolinyl or isoquinolinyl group may be bound to the PBD core through any available ring position. For example, the quinolinyl may be quinolin-2-yl, quinolin-3-yl, quinolin-4yl, quinolin-5-yl, quinolin-6-yl, quinolin-7-yl and quinolin-8-yl. Of these quinolin-3-yl and quinolin-6-yl may be preferred. The isoquinolinyl may be isoquinolin-1-yl, isoquinolin-3-yl, isoquinolin-4yl, isoquinolin-5-yl, isoquinolin-6-yl, isoquinolin-7-yl and isoquinolin-8-yl. Of these isoquinolin-3-yl and isoquinolin-6-yl may be preferred.

### C2 Vinyl

In one embodiment, the compound is: 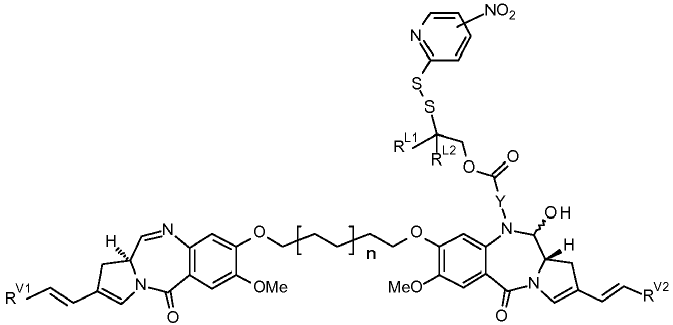 more preferably: 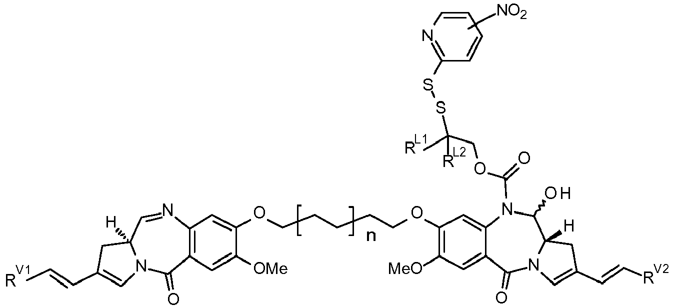 wherein Y, R^{L1} and R^{L2} are as previously defined, R^{V1} and R^{V2} are independently selected from H, methyl, ethyl and phenyl (which phenyl may be optionally substituted with fluoro, particularly in the 4 position) and C₅₋₆ heterocyclyl, and n is 0 or 1. R^{V1} and R^{V2} may be the same or different.

In some of the above embodiments, R^{V1} and R^{V2} may be independently selected from H, phenyl, and 4-fluorophenyl.

In some of the above embodiments, the pyridyl ring is monosubstituted in the 3-position with -NO₂ (meta relative to the disulfide).

In some of the above embodiments, the pyridyl ring is monosubstituted in the 5-position with -NO₂ (para relative to the disulfide).

The first aspect of the present invention provides compounds of formula (D) comprising a 2-mercaptopyridine leaving group. The pyridine ring of the leaving group is substituted at one or more positions with a nitro group. The conjugates are produced more efficiently from intermediates which have a nitro-substituted pyridyl compared to corresponding unsubstituted intermediates.

It has been observed that a nitro substituent is particularly effective at providing the enhanced reactivity of the intermediates required to more efficiently prepare the antibody-drug conjugates.

In one embodiment, the ring is mono-substituted at the 5-position with -NO₂ (para- to the disulfide), for example as in the following compound:

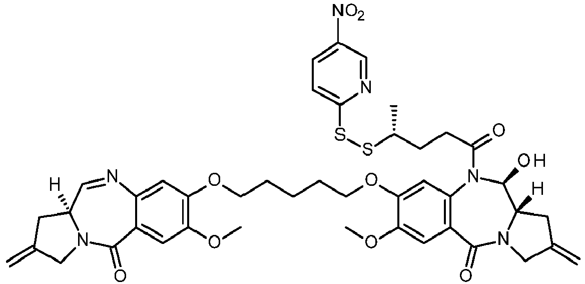

In another embodiment, the ring is mono-substituted at the 3-position with -NO₂ (meta- to the disulfide), for example as in the following compound:

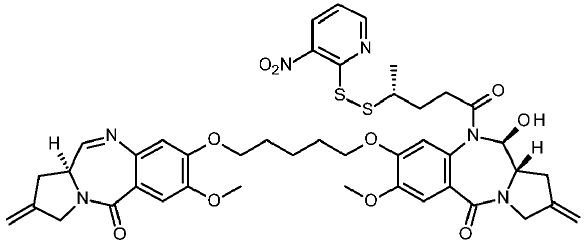

In other embodiments, the ring is poly-substituted with nitro groups. For example, there may be an -NO₂ substituent in both the 3- and 5-position (i.e. both meta- and para-substituted relative to the disulfide).

### Double Bond

In one embodiment, there is no double bond present between C1 and C2, and C2 and C3.

In one embodiment, the dotted lines indicate the optional presence of a double bond between C2 and C3, as shown below:

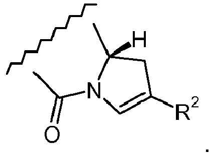

In one embodiment, a double bond is present between C2 and C3 when R² is C₅₋₂₀ aryl or C₁₋₁₂ alkyl.

In one embodiment, the dotted lines indicate the optional presence of a double bond between C1 and C2, as shown below:

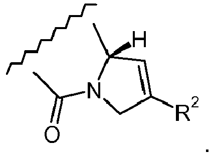

In one embodiment, a double bond is present between C1 and C2 when R² is C₅₋₂₀ aryl or C₁₋₁₂ alkyl.

### R²

In one embodiment, R² is independently selected from H, OH, =O, =CH₂, CN, R, OR, =CH-R^{D}, =C(R^{D})₂, O-SO₂-R, CO₂R and COR, and optionally further selected from halo or dihalo. In one embodiment, R² is independently selected from H, OH, =O, =CH₂, CN, R, OR, =CH-R^{D}, =C(R^{D})₂, O-SO₂-R, CO₂R and COR.

In one embodiment, R² is independently selected from H, =O, =CH₂, R, =CH-R^{D}, and =C(R^{D})₂.

In one embodiment, R² is independently H.

In one embodiment, R² is independently =O.

In one embodiment, R² is independently =CH₂.

In one embodiment, R² is independently =CH-R^{D}. Within the PBD compound, the group =CH-R^{D} may have either configuration shown below:

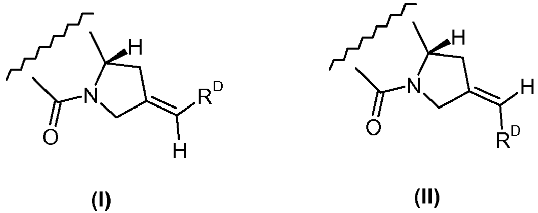

In one embodiment, the configuration is configuration (I).

In one embodiment, R² is independently =C(R^{D})₂.

In one embodiment, R² is independently =CF₂.

In one embodiment, R² is independently R.

In one embodiment, R² is independently optionally substituted C₅₋₂₀ aryl.

In one embodiment, R² is independently optionally substituted C₁₋₁₂ alkyl.

In one embodiment, R² is independently optionally substituted C₅₋₂₀ aryl.

In one embodiment, R² is independently optionally substituted C₅₋₇ aryl.

In one embodiment, R² is independently optionally substituted C₈₋₁₀ aryl.

In one embodiment, R² is independently optionally substituted phenyl.

In one embodiment, R² is independently optionally substituted thienyl.

In one embodiment, R² is independently optionally substituted naphthyl.

In one embodiment, R² is independently optionally substituted pyridyl.

In one embodiment, R² is independently optionally substituted quinolinyl or isoquinolinyl.

In one embodiment, R² bears one to three substituent groups, with 1 and 2 being more preferred, and singly substituted groups being most preferred. The substituents may be any position.

Where R² is a C₅₋₇ aryl group, a single substituent is preferably on a ring atom that is not adjacent the bond to the remainder of the compound, i.e. it is preferably β or γ to the bond to the remainder of the compound. Therefore, where the C₅₋₇ aryl group is phenyl, the substituent is preferably in the meta- or para- positions, and more preferably is in the para-position.

In one embodiment, R² is selected from: 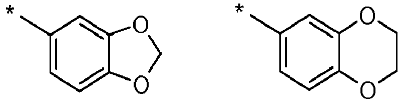 where the asterisk indicates the point of attachment.

Where R² is a C₈₋₁₀ aryl group, for example quinolinyl or isoquinolinyl, it may bear any number of substituents at any position of the quinoline or isoquinoline rings. In some embodiments, it bears one, two or three substituents, and these may be on either the proximal and distal rings or both (if more than one substituent).

In one embodiment, where R² is optionally substituted, the substituents are selected from those substituents given in the substituent section below.

Where R is optionally substituted, the substituents are preferably selected from:
Halo, Hydroxyl, Ether, Formyl, Acyl, Carboxy, Ester, Acyloxy, Amino, Amido, Acylamido, Aminocarbonyloxy, Ureido, Nitro, Cyano and Thioether.

In one embodiment, where R or R² is optionally substituted, the substituents are selected from the group consisting of R, OR, SR, NRR', NO₂, halo, CO₂R, COR, CONH₂, CONHR, and CONRR'.

Where R² is C₁₋₁₂ alkyl, the optional substituent may additionally include C₃₋₂₀ heterocyclyl and C₅₋₂₀ aryl groups.

Where R² is C₃₋₂₀ heterocyclyl, the optional substituent may additionally include C₁₋₁₂ alkyl and C₅₋₂₀ aryl groups.

Where R² is C₅₋₂₀ aryl groups, the optional substituent may additionally include C₃₋₂₀ heterocyclyl and C₁₋₁₂ alkyl groups.

It is understood that the term "alkyl" encompasses the sub-classes alkenyl and alkynyl as well as cycloalkyl. Thus, where R² is optionally substituted C₁₋₁₂ alkyl, it is understood that the alkyl group optionally contains one or more carbon-carbon double or triple bonds, which may form part of a conjugated system. In one embodiment, the optionally substituted C₁₋₁₂ alkyl group contains at least one carbon-carbon double or triple bond, and this bond is conjugated with a double bond present between C1 and C2, or C2 and C3. In one embodiment, the C₁₋₁₂ alkyl group is a group selected from saturated C₁₋₁₂ alkyl, C₂₋₁₂ alkenyl, C₂₋₁₂ alkynyl and C₃₋₁₂ cycloalkyl.

If a substituent on R² is halo, it is preferably F or Cl, more preferably F.

If a substituent on R² is ether, it may in some embodiments be an alkoxy group, for example, a C₁₋₇ alkoxy group (e.g. methoxy, ethoxy) or it may in some embodiments be a C₅₋₇ aryloxy group (e.g. phenoxy, pyridyloxy, furanyloxy).

If a substituent on R² is C₁₋₇ alkyl, it may preferably be a C₁₋₄ alkyl group (e.g. methyl, ethyl, propyl, butyl).

If a substituent on R² is C₃₋₇ heterocyclyl, it may in some embodiments be C₆ nitrogen containing heterocyclyl group, e.g. morpholino, thiomorpholino, piperidinyl, piperazinyl. These groups may be bound to the rest of the PBD moiety via the nitrogen atom. These groups may be further substituted, for example, by C₁₋₄ alkyl groups.

If a substituent on R² is bis-oxy-C₁₋₃ alkylene, this is preferably bis-oxy-methylene or bis-oxyethylene.

Particularly preferred substituents for R² include methoxy, ethoxy, fluoro, chloro, cyano, bis-oxy-methylene, methyl-piperazinyl, morpholino and methyl-thienyl.

Particularly preferred substituted R² groups include, but are not limited to, 4-methoxy-phenyl, 3-methoxyphenyl, 4-ethoxy-phenyl, 3-ethoxy-phenyl, 4-methyl-phenyl, 4-fluoro-phenyl, 4-chloro-phenyl, 3,4-bisoxymethylene-phenyl, 4-methylthienyl, 4-cyanophenyl, 4-phenoxyphenyl, quinolin-3-yl and quinolin-6-yl, isoquinolin-3-yl and isoquinolin-6-yl, 2-thienyl, 2-furanyl, methoxynaphthyl, and naphthyl.

In one embodiment, R² is halo or dihalo. In one embodiment, R² is -F or -F₂, which substituents are illustrated below as (III) and (IV) respectively:

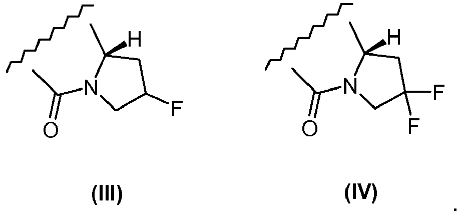

In some embodiments, it is preferred that there is either a double bond between C2 and C3 or the C2 substituent is bound to the PBD ring by a double bond (i.e. that the C atom at C2 is a sp² centre)

### R^{D}

In one embodiment, R^{D} is independently selected from R, CO₂R, COR, CHO, CO₂H, and halo.

In one embodiment, R^{D} is independently R.

In one embodiment, R^{D} is independently halo.

### R⁶

In one embodiment, R⁶ is independently selected from H, R, OH, OR, SH, SR, NH₂, NHR, NRR', NO₂, Me₃Sn- and Halo.

In one embodiment, R⁶ is independently selected from H, OH, OR, SH, NH₂, NO₂ and Halo.

In one embodiment, R⁶ is independently selected from H and Halo.

In one embodiment, R⁶ is independently H.

In one embodiment, R⁶ and R⁷ together form a group -O-(CH₂)ₚ-O-, where p is 1 or 2.

### R⁷

R⁷ is independently selected from H, R, OH, OR, SH, SR, NH₂, NHR, NRR', NO₂, Me₃Sn and halo.

In one embodiment, R⁷ is independently OR.

In one embodiment, R⁷ is independently OR^{7A}, where R^{7A} is independently optionally substituted C₁₋₆ alkyl.

In one embodiment, R^{7A} is independently optionally substituted saturated C₁₋₆ alkyl.

In one embodiment, R^{7A} is independently optionally substituted C₂₋₄ alkenyl.

In one embodiment, R^{7A} is independently Me.

In one embodiment, R^{7A} is independently CH₂Ph.

In one embodiment, R^{7A} is independently allyl.

### R⁹

In one embodiment, R⁹ is independently selected from H, R, OH, OR, SH, SR, NH₂, NHR, NRR', NO₂, Me₃Sn- and Halo.

In one embodiment, R⁹ is independently H.

In one embodiment, R⁹ is independently R or OR.

### Linking group

The linking group is removable from the N10 position of the PBD moiety in the conjugate of formula A to leave an N10-C11 imine bond, a carbinolamine, a substituted carbinolamine, where QR¹¹ is OSO₃M, a bisulfite adduct, a thiocarbinolamine, a substituted thiocarbinolamine, a substituted carbinalamine as illustrated below: 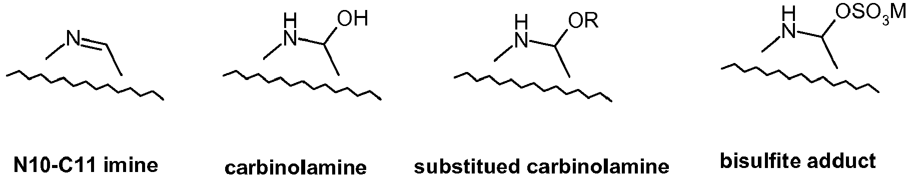 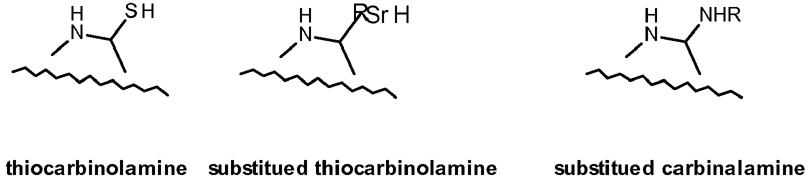 where R and M are as defined for the conjugates of the invention.

In one embodiment, the linking group is removable from the N10 position of the PBD moiety to leave an N10-C11 imine bond.

The specified link between the PBD dimer and the cell binding agent, e.g. antibody, in the present invention is preferably stable extracellularly. Before transport or delivery into a cell, the antibody-drug conjugate (ADC) is preferably stable and remains intact, i.e. the antibody remains linked to the drug moiety. The linkers are stable outside the target cell and may be cleaved at some efficacious rate inside the cell. An effective linker will: (i) maintain the specific binding properties of the antibody; (ii) allow intracellular delivery of the conjugate or drug moiety; (iii) remain stable and intact, i.e. not cleaved, until the conjugate has been delivered or transported to its targeted site; and (iv) maintain a cytotoxic, cell-killing effect or a cytostatic effect of the PBD drug moiety. Stability of the ADC may be measured by standard analytical techniques such as mass spectroscopy, HPLC, and the separation/analysis technique LC/MS.

Delivery of the PBD compounds is achieved at the desited activation site of the conjugates of formula A by the action of an enzyme on the linking group. The S of the conjugate of formula A is linked by a disulfide bond to a free S (active thiol) on the cell binding agent.

The linking group may be cleavable by the action of an enzyme. In one embodiment, the enzyme is a thioreductase.

Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by introducing one, two, three, four, or more cysteine residues (e.g., preparing mutant antibodies comprising one or more non-native cysteine amino acid residues). US 7521541 teaches engineering antibodies by introduction of reactive cysteine amino acids.

R^{L1} and R^{L2} are selected from H and methyl, or together with the carbon atom to which they are bound form a cyclopropylene group. In some embodiments, both are H. In other embodiment, both are methyl. In further embodiments, one is H and the other is methyl; in these embodiments, the carbon atom to which they are bound is a chiral centre.

In some embodiments, Y is a single bond.

In other embodiments, Y is

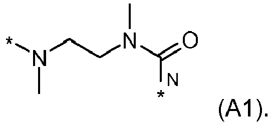

In further embodiments, Y is

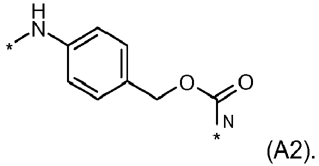

### Q

In one embodiment, Q is selected from O, S, or N(H).

Preferably, Q is O.

### R¹¹

In one embodiment, R¹¹ is either H, or R or, where Q is O, SO₃M, where M is a metal cation.

In one embodiment, R¹¹ is H.

In one embodiment, R¹¹ is R.

In one embodiment, where Q is O, R¹¹ is SO₃M, where M is a metal cation. The cation may be Na⁺.

### Cell Binding Agent

The compounds of the first aspect of the invention are useful for reaction with a cell binding agent to produce a conjugate compound. The method of the second aspect of the present invention involves the reaction of a cell binding agent with a compound of the first aspect.

A cell binding agent may be of any kind, and include peptides and non-peptides. These can include antibodies or a fragment of an antibody that contains at least one binding site, lymphokines, hormones, growth factors, nutrient-transport molecules, or any other cell binding molecule or substance.

The term "antibody" herein is used in the broadest sense and specifically covers monoclonal antibodies, polyclonal antibodies, dimers, multimers, multispecific antibodies (e.g., bispecific antibodies), and antibody fragments, so long as they exhibit the desired biological activity (Miller et al (2003) Jour. of Immunology 170:4854-4861). Antibodies may be murine, human, humanized, chimeric, or derived from other species. An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, *i.e.,* a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g. IgG, IgE, IgM, IgD, and IgA), class (e.g. IgG1, IgG2, IgG3, IgG4, IgA1 and IgA2) or subclass of immunoglobulin molecule. The immunoglobulins can be derived from any species, including human, murine, or rabbit origin.

"Antibody fragments" comprise a portion of a full length antibody, generally the antigen binding or variable region thereof. Examples of antibody fragments include Fab, Fab', F(ab')₂, and Fv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The term "monoclonal antibody" as used herein refers to an antibody obtained from a population of substantially homogeneous antibodies, i.e. the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al (1975) Nature 256:495, or may be made by recombinant DNA methods (see, US 4816567). The monoclonal antibodies may also be isolated from phage antibody libraries using the techniques described in Clackson et al (1991) Nature, 352:624-628; Marks et al (1991) J. Mol. Biol., 222:581-597.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US 4816567; and Morrison et al (1984) Proc. Natl. Acad. Sci. USA, 81:6851-6855). Chimeric antibodies include "primatized" antibodies comprising variable domain antigen-binding sequences derived from a non-human primate (e.g. Old World Monkey or Ape) and human constant region sequences.

An "intact antibody" herein is one comprising a VL and VH domains, as well as a light chain constant domain (CL) and heavy chain constant domains, CH1, CH2 and CH3. The constant domains may be native sequence constant domains (e.g. human native sequence constant domains) or amino acid sequence variant thereof. The intact antibody may have one or more "effector functions" which refer to those biological activities attributable to the Fc region (a native sequence Fc region or amino acid sequence variant Fc region) of an antibody. Examples of antibody effector functions include C1 q binding; complement dependent cytotoxicity; Fc receptor binding; antibody-dependent cell-mediated cytotoxicity (ADCC); phagocytosis; and down regulation of cell surface receptors such as B cell receptor and BCR.

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), e.g., IgG1, IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known.

Examples of cell binding agents include those agents described for use in WO 2007/085930, which is incorporated herein.

The cell binding agent may be, or comprise, a polypeptide. The polypeptide may be a cyclic polypeptide. The cell binding agent may be antibody. Thus, in one embodiment, the method of the present invention provides an antibody-drug conjugate (ADC).

### Drug loading

The drug loading is the average number of PBD drugs per antibody. Drug loading may range from 1 to 8 drugs (D) per antibody (Ab), i.e. where 1, 2, 3, 4, 5, 6, 7, and 8 drug moieties are covalently attached to the antibody. Compositions of ADC include collections of antibodies conjugated with a range of drugs, from 1 to 8. The average number of drugs per antibody in preparations of ADC from conjugation reactions may be characterized by conventional means such as mass spectroscopy, ELISA assay, electrophoresis, and HPLC. The quantitative distribution of ADC in terms of p may also be determined. By ELISA, the averaged value of p in a particular preparation of ADC may be determined (Hamblett et al (2004) Clin. Cancer Res. 10:7063-7070; Sanderson et al (2005) Clin. Cancer Res. 11:843-852). However, the distribution of p (drug) values is not discernible by the antibody-antigen binding and detection limitation of ELISA. Also, ELISA assay for detection of antibody-drug conjugates does not determine where the drug moieties are attached to the antibody, such as the heavy chain or light chain fragments, or the particular amino acid residues. In some instances, separation, purification, and characterization of homogeneous ADC where p is a certain value from ADC with other drug loadings may be achieved by means such as reverse phase HPLC or electrophoresis.

For some antibody-drug conjugates, p may be limited by the number of attachment sites on the antibody. For example, an antibody may have only one or several cysteine thiol groups, or may have only one or several sufficiently reactive thiol groups through which a linker may be attached. Higher drug loading, e.g. p >5, may cause aggregation, insolubility, toxicity, or loss of cellular permeability of certain antibody-drug conjugates.

Typically, less than the theoretical maximum of drug moieties are conjugated to an antibody during a conjugation reaction. An antibody may contain, for example, many lysine residues that do not react with the drug-linker intermediate (D-L) or linker reagent. Only the most reactive lysine groups may react with an amine-reactive linker reagent. Also, only the most reactive cysteine thiol groups may react with a thiol-reactive linker reagent. Generally, antibodies do not contain many, if any, free and reactive cysteine thiol groups which may be linked to a drug moiety. Most cysteine thiol residues in the antibodies of the compounds exist as disulfide bridges and must be reduced with a reducing agent such as dithiothreitol (DTT) or TCEP, under partial or total reducing conditions. The loading (drug/antibody ratio) of an ADC may be controlled in several different manners, including: (i) limiting the molar excess of drug-linker intermediate (D-L) or linker reagent relative to antibody, (ii) limiting the conjugation reaction time or temperature, and (iii) partial or limiting reductive conditions for cysteine thiol modification.

Cysteine amino acids may be engineered at reactive sites in an antibody and which do not form intrachain or intermolecular disulfide linkages (Junutula, et al., 2008b Nature Biotech., 26(8):925-932; Dornan et al (2009) Blood 114(13):2721-2729; US 7521541; US 7723485; WO2009/052249, Shen et al (2012) Nature Biotech., 30(2):184-191; Junutula et al (2008) Jour of Immun. Methods 332:41-52). The engineered cysteine thiols may react with linker reagents or the drug-linker reagents of the present invention which have thiol-reactive, electrophilic groups such as maleimide or alpha-halo amides to form ADC with cysteine engineered antibodies (THIOMAB™) and the PBD drug moieties. The location of the drug moiety can thus be designed, controlled, and known. The drug loading can be controlled since the engineered cysteine thiol groups typically react with thiol-reactive linker reagents or drug-linker reagents in high yield. Engineering an IgG antibody to introduce a cysteine amino acid by substitution at a single site on the heavy or light chain gives two new cysteines on the symmetrical antibody. A drug loading near 2 can be achieved and near homogeneity of the conjugation product ADC.

Where more than one nucleophilic or electrophilic group of the antibody reacts with a drug-linker intermediate, or linker reagent followed by drug moiety reagent, then the resulting product is a mixture of ADC compounds with a distribution of drug moieties attached to an antibody, e.g. 1, 2, 3, etc. Liquid chromatography methods such as polymeric reverse phase (PLRP) and hydrophobic interaction (HIC) may separate compounds in the mixture by drug loading value. Preparations of ADC with a single drug loading value (p) may be isolated, however, these single loading value ADCs may still be heterogeneous mixtures because the drug moieties may be attached, via the linker, at different sites on the antibody.

Thus the antibody-drug conjugate compositions described herein include mixtures of antibody-drug conjugate compounds where the antibody has one or more PBD drug moieties and where the drug moieties may be attached to the antibody at various amino acid residues.

In one embodiment, the average number of dimer pyrrolobenzodiazepine groups per cell binding agent is in the range 1 to 20. In some embodiments the range is selected from 1 to 8, 2 to 8, 2 to 6, 2 to 4, and 4 to 8.

In some embodiments, there is one dimer pyrrolobenzodiazepine groups per cell binding agent.

Cysteine-engineered antibody mutants (THIOMAB™) are described in WO 2006/034488 and WO 2011/156328, which are herein incorporated by reference.

### LC K149C cysteine-engineered antibody mutant (THIOMAB™)

The LC K149C cysteine-engineered antibody mutant is described generically in WO 2006/034488, and specifically in SEQ ID NO.:133 on page 57 of WO 2011/156328. K149C mutant is also described in WO 2013/093809 and US 2013/0066054.

In one aspect, the LC K149C cysteine-engineered antibody mutant comprises a Cλ polypeptide, or portion thereof, comprising the amino acid substitution K149C according to the numbering of Kabat.

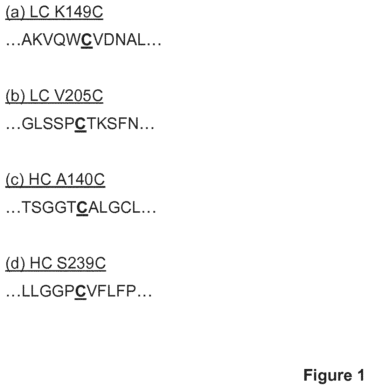

Figure 1a shows an example sequence in which the mutated residue (in bold & underlined) is shown in context of the five preceding and subsequent amino acids.

### LC V205C cysteine-engineered antibody mutant (THIOMAB™)

The LC V205C cysteine-engineered antibody mutant is described generically in WO 2006/034488, and specifically in SEQ ID NO.:145 on page 57 of WO 2011/156328. V205C mutant is also described in WO 2013/093809 and US 2013/0066054.

In one aspect, the LC V205C cysteine-engineered antibody mutant comprises a Cλ polypeptide, or portion thereof, comprising the amino acid substitution V205C according to the numbering of Kabat.

Figure 1b shows an example sequence in which the mutated residue (in bold & underlined) is shown in context of the five preceding and subsequent amino acids.

### HC A140C cysteine-engineered antibody mutant (THIOMAB™)

In one aspect, the HC 140C cysteine-engineered antibody mutant comprises a Cγ polypeptide, or portion thereof, comprising the amino acid substitution A140C according to the EU index of Kabat.

Figure 1c shows an example sequence in which the mutated residue (in bold & underlined) is shown in context of the five preceding and subsequent amino acids.

### HC S239C cysteine-engineered antibody mutant (THIOMAB™)

In one aspect, the HC S239C cysteine-engineered antibody mutant comprises a Cγ polypeptide, or portion thereof, comprising the amino acid substitution S239C according to the EU index of Kabat.

Figure 1d shows an example sequence in which the mutated residue (in bold & underlined) is shown in context of the five preceding and subsequent amino acids.

### Peptides

In one embodiment, the cell binding agent is a linear or cyclic peptide comprising 4-20, preferably 6-20, contiguous amino acid residues. In this embodiment, it is preferred that one cell binding agent is linked to one monomer or dimer pyrrolobenzodiazepine compound.

In one embodiment the cell binding agent comprises a peptide that binds integrin αᵥβ₆. The peptide may be selective for αᵥβ₆ over XYS.

In one embodiment the cell binding agent comprises the A20FMDV-Cys polypeptide. The A20FMDV-Cys has the sequence: NAVPNLRGDLQVLAQKVARTC. Alternatively, a variant of the A20FMDV-Cys sequence may be used wherein one, two, three, four, five, six, seven, eight, nine or ten amino acid residues is substituted with another amino acid residue.

In one embodiment the antibody is a monoclonal antibody; chimeric antibody; humanized antibody; fully human antibody; or a single chain antibody. One embodiment the antibody is a fragment of one of these antibodies having biological activity. Examples of such fragments include Fab, Fab', F(ab')₂ and Fv fragments.

In these embodiments, each antibody may be linked to one or several dimer pyrrolobenzodiazepine groups. The preferred ratios of pyrrolobenzodiazepine to cell binding agent are given above.

The antibody may be a domain antibody (DAB).

In one embodiment, the antibody is a monoclonal antibody.

Antibodies for use in the method of the present invention include those antibodies described in WO 2005/082023 which is incorporated herein. Particularly preferred are those antibodies for tumour-associated antigens. Examples of those antigens known in the art include, but are not limited to, those tumour-associated antigens set out in WO 2005/082023. See, for instance, pages 41-55.

The conjugates described herein are designed to target tumour cells via their cell surface antigens. The antigens are usually normal cell surface antigens which are either over-expressed or expressed at abnormal times. Ideally the target antigen is expressed only on proliferative cells (preferably tumour cells), however this is rarely observed in practice. As a result, target antigens are usually selected on the basis of differential expression between proliferative and healthy tissue.

Antibodies have been raised to target specific tumour related antigens including: Cripto, CD30, CD19, CD33, Glycoprotein NMB, CanAg, Her2 (ErbB2/Neu), CD56 (NCAM), CD22 (Siglec2), CD33 (Siglec3), CD79, CD138, PSCA, PSMA (prostate specific membrane antigen), BCMA, CD20, CD70, E-selectin, EphB2, Melanotransferin, Muc16 and TMEFF2.

Tumor-associated antigens (TAA) are known in the art, and can prepared for use in generating antibodies using methods and information which are well known in the art. In attempts to discover effective cellular targets for cancer diagnosis and therapy, researchers have sought to identify transmembrane or otherwise tumor-associated polypeptides that are specifically expressed on the surface of one or more particular type(s) of cancer cell as compared to on one or more normal non-cancerous cell(s). Often, such tumor-associated polypeptides are more abundantly expressed on the surface of the cancer cells as compared to on the surface of the non-cancerous cells. The identification of such tumor-associated cell surface antigen polypeptides has given rise to the ability to specifically target cancer cells for destruction via antibody-based therapies.

Examples of TAA include, but are not limited to, TAA (1)-(53) listed below. For convenience, information relating to these antigens, all of which are known in the art, is listed below and includes names, alternative names, Genbank accession numbers and primary reference(s), following nucleic acid and protein sequence identification conventions of the National Center for Biotechnology Information (NCBI). Nucleic acid and protein sequences corresponding to TAA (1)-(53) are available in public databases such as GenBank. Tumor-associated antigens targeted by antibodies include all amino acid sequence variants and isoforms possessing at least about 70%, 80%, 85%, 90%, or 95% sequence identity relative to the sequences identified in the cited references, or which exhibit substantially the same biological properties or characteristics as a TAA having a sequence found in the cited references. For example, a TAA having a variant sequence generally is able to bind specifically to an antibody that binds specifically to the TAA with the corresponding sequence listed. The sequences and disclosure in the reference specifically recited herein are expressly incorporated by reference.

### TUMOR-ASSOCIATED ANTIGENS (1)-(53):

(1) BMPR1 B (bone morphogenetic protein receptor-type IB, Genbank accession no. NM_001203) ten Dijke,P., et al Science 264 (5155):101-104 (1994), Oncogene 14 (11):1377-1382 (1997)); WO2004/063362 (Claim 2); WO2003/042661 (Claim 12); US2003/134790-A1 (Page 38-39); WO2002/102235 (Claim 13; Page 296); WO2003/055443 (Page 91-92); WO2002/99122 (Example 2; Page 528-530); WO2003/029421 (Claim 6); WO2003/024392 (Claim 2; Fig 112); WO2002/98358 (Claim 1; Page 183); WO2002/54940 (Page 100-101); WO2002/59377 (Page 349-350); WO2002/30268 (Claim 27; Page 376); WO2001/48204 (Example; Fig 4); NP_001194 bone morphogenetic protein receptor, type IB /pid=NP_001194.1. Cross-references: MIM:603248; NP_001194.1; AY065994
(2) E16 (LAT1, SLC7A5, Genbank accession no. NM_003486) Biochem. Biophys. Res. Commun. 255 (2), 283-288 (1999), Nature 395 (6699):288-291 (1998), Gaugitsch, H.W., et al (1992) J. Biol. Chem. 267 (16):11267-11273); WO2004/048938 (Example 2); WO2004/032842 (Example IV); WO2003/042661 (Claim 12); WO2003/016475 (Claim 1); WO2002/78524 (Example 2); WO2002/99074 (Claim 19; Page 127-129); WO2002/86443 (Claim 27; Pages 222, 393); WO2003/003906 (Claim 10; Page 293); WO2002/64798 (Claim 33; Page 93-95); WO2000/14228 (Claim 5; Page 133-136); US2003/224454 (Fig 3); WO2003/025138 (Claim 12; Page 150); NP_003477 solute carrier family 7 (cationic amino acid transporter, y+system), member 5 /pid=NP_003477.3 - Homo sapiens; Cross-references: MIM:600182; NP_003477.3; NM_015923; NM_003486_1
(3) STEAP1 (six transmembrane epithelial antigen of prostate, Genbank accession no. NM_012449); Cancer Res. 61 (15), 5857-5860 (2001), Hubert, R.S., et al (1999) Proc. Natl. Acad. Sci. U.S.A. 96 (25):14523-14528); WO2004/065577 (Claim 6); WO2004/027049 (Fig 1L); EP1394274 (Example 11); WO2004/016225 (Claim 2); WO2003/042661 (Claim 12); US2003/157089 (Example 5); US2003/185830 (Example 5); US2003/064397 (Fig 2); WO2002/89747 (Example 5; Page 618-619); WO2003/022995 (Example 9; Fig 13A, Example 53; Page 173, Example 2; Fig 2A); NP_036581 six transmembrane epithelial antigen of the prostate; Cross-references: MIM:604415; NP_036581.1; NM_012449_1
(4) 0772P (CA125, MUC16, Genbank accession no. AF361486); J. Biol. Chem. 276 (29):27371-27375 (2001)); WO2004/045553 (Claim 14); WO2002/92836 (Claim 6; Fig 12); WO2002/83866 (Claim 15; Page 116-121); US2003/124140 (Example 16); Cross-references: GI:34501467; AAK74120.3; AF361486_1
(5) MPF (MPF, MSLN, SMR, megakaryocyte potentiating factor, mesothelin, Genbank accession no. NM_005823) Yamaguchi, N., et al Biol. Chem. 269 (2), 805-808 (1994), Proc. Natl. Acad. Sci. U.S.A. 96 (20):11531-11536 (1999), Proc. Natl. Acad. Sci. U.S.A. 93 (1):136-140 (1996), J. Biol. Chem. 270 (37):21984-21990 (1995)); WO2003/101283 (Claim 14); (WO2002/102235 (Claim 13; Page 287-288); WO2002/101075 (Claim 4; Page 308-309); WO2002/71928 (Page 320-321); WO94/10312 (Page 52-57); Cross-references: MIM:601051; NP_005814.2; NM_005823_1
(6) Napi3b (NAPI-3B, NPTIIb, SLC34A2, solute carrier family 34 (sodium phosphate), member 2, type II sodium-dependent phosphate transporter 3b, Genbank accession no. NM_006424) J. Biol. Chem. 277 (22):19665-19672 (2002), Genomics 62 (2):281-284 (1999), Feild, J.A., et al (1999) Biochem. Biophys. Res. Commun. 258 (3):578-582); WO2004/022778 (Claim 2); EP1394274 (Example 11); WO2002/102235 (Claim 13; Page 326); EP0875569 (Claim 1; Page 17-19); WO2001/57188 (Claim 20; Page 329); WO2004/032842 (Example IV); WO2001/75177 (Claim 24; Page 139-140); Cross-references: MIM:604217; NP_006415.1; NM_006424_1
(7) Sema 5b (FLJ10372, KIAA1445, Mm.42015, SEMA5B, SEMAG, Semaphorin 5b Hlog, sema domain, seven thrombospondin repeats (type 1 and type 1-like), transmembrane domain (TM) and short cytoplasmic domain, (semaphorin) 5B, Genbank accession no. AB040878); Nagase T., et al (2000) DNA Res. 7 (2):143-150); WO2004/000997 (Claim 1); WO2003/003984 (Claim 1); WO2002/06339 (Claim 1; Page 50); WO2001/88133 (Claim 1; Page 41-43, 48-58); WO2003/054152 (Claim 20); WO2003/101400 (Claim 11); Accession: Q9P283; EMBL; AB040878; BAA95969.1. Genew; HGNC:10737
(8) PSCA hlg (2700050C12Rik, C530008O16Rik, RIKEN cDNA 2700050C12, RIKEN cDNA 2700050C12 gene, Genbank accession no. AY358628); Ross et al (2002) Cancer Res. 62:2546-2553; US2003/129192 (Claim 2); US2004/044180 (Claim 12); US2004/044179 (Claim 11); US2003/096961 (Claim 11); US2003/232056 (Example 5); WO2003/105758 (Claim 12); US2003/206918 (Example 5); EP1347046 (Claim 1); WO2003/025148 (Claim 20); Cross-references: GI:37182378; AAQ88991.1; AY358628_1
(9) ETBR (Endothelin type B receptor, Genbank accession no. AY275463); Nakamuta M., et al Biochem. Biophys. Res. Commun. 177, 34-39, 1991; Ogawa Y., et al Biochem. Biophys. Res. Commun. 178, 248-255, 1991; Arai H., et al Jpn. Circ. J. 56, 1303-1307, 1992; Arai H., et al J. Biol. Chem. 268, 3463-3470, 1993; Sakamoto A., Yanagisawa M., et al Biochem. Biophys. Res. Commun. 178, 656-663, 1991; Elshourbagy N.A., et al J. Biol. Chem. 268, 3873-3879, 1993; Haendler B., et al J. Cardiovasc. Pharmacol. 20, s1-S4, 1992; Tsutsumi M., et al Gene 228, 43-49, 1999; Strausberg R.L., et al Proc. Natl. Acad. Sci. U.S.A. 99, 16899-16903, 2002; Bourgeois C., et al J. Clin. Endocrinol. Metab. 82, 3116-3123, 1997; Okamoto Y., et al Biol. Chem. 272, 21589-21596, 1997; Verheij J.B., et al Am. J. Med. Genet. 108, 223-225, 2002; Hofstra R.M.W., et al Eur. J. Hum. Genet. 5, 180-185, 1997; Puffenberger E.G., et al Cell 79, 1257-1266, 1994; Attie T., et al, Hum. Mol. Genet. 4, 2407-2409, 1995; Auricchio A., et al Hum. Mol. Genet. 5:351-354, 1996; Amiel J., et al Hum. Mol. Genet. 5, 355-357, 1996; Hofstra R.M.W., et al Nat. Genet. 12, 445-447, 1996; Svensson P.J., et al Hum. Genet. 103, 145-148, 1998; Fuchs S., et al Mol. Med. 7, 115-124, 2001; Pingault V., et al (2002) Hum. Genet. 111, 198-206; WO2004/045516 (Claim 1); WO2004/048938 (Example 2); WO2004/040000 (Claim 151); WO2003/087768 (Claim 1); WO2003/016475 (Claim 1); WO2003/016475 (Claim 1); WO2002/61087 (Fig 1); WO2003/016494 (Fig 6); WO2003/025138 (Claim 12; Page 144); WO2001/98351 (Claim 1; Page 124-125); EP0522868 (Claim 8; Fig 2); WO2001/77172 (Claim 1; Page 297-299); US2003/109676; US6518404 (Fig 3); US5773223 (Claim 1a; Col 31-34); WO2004/001004
(10) MSG783 (RNF124, hypothetical protein FLJ20315, Genbank accession no. NM_017763); WO2003/104275 (Claim 1); WO2004/046342 (Example 2); WO2003/042661 (Claim 12); WO2003/083074 (Claim 14; Page 61); WO2003/018621 (Claim 1); WO2003/024392 (Claim 2; Fig 93); WO2001/66689 (Example 6); Cross-references: LocusID:54894; NP_060233.2; NM_017763_1
(11) STEAP2 (HGNC_8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein, Genbank accession no. AF455138); Lab. Invest. 82 (11):1573-1582 (2002)); WO2003/087306; US2003/064397 (Claim 1; Fig 1); WO2002/72596 (Claim 13; Page 54-55); WO2001/72962 (Claim 1; Fig 4B); WO2003/104270 (Claim 11); WO2003/104270 (Claim 16); US2004/005598 (Claim 22); WO2003/042661 (Claim 12); US2003/060612 (Claim 12; Fig 10); WO2002/26822 (Claim 23; Fig 2); WO2002/16429 (Claim 12; Fig 10); Cross-references: GI:22655488; AAN04080.1; AF455138_1
(12) TrpM4 (BR22450, FLJ20041, TRPM4, TRPM4B, transient receptor potential cation channel, subfamily M, member 4, Genbank accession no. NM_017636); Xu, X.Z., et al Proc. Natl. Acad. Sci. U.S.A. 98 (19):10692-10697 (2001), Cell 109 (3):397-407 (2002), J. Biol. Chem. 278 (33):30813-30820 (2003)); US2003/143557 (Claim 4); WO2000/40614 (Claim 14; Page 100-103); WO2002/10382 (Claim 1; Fig 9A); WO2003/042661 (Claim 12); WO2002/30268 (Claim 27; Page 391); US2003/219806 (Claim 4); WO2001/62794 (Claim 14; Fig 1A-D); Cross-references: MIM:606936; NP_060106.2; NM_017636_1
(13) CRIPTO (CR, CR1, CRGF, CRIPTO, TDGF1, teratocarcinoma-derived growth factor, Genbank accession no. NP_003203 or NM_003212); Ciccodicola, A., et al EMBO J. 8 (7):1987-1991 (1989), Am. J. Hum. Genet. 49 (3):555-565 (1991)); US2003/224411 (Claim 1); WO2003/083041 (Example 1); WO2003/034984 (Claim 12); WO2002/88170 (Claim 2; Page 52-53); WO2003/024392 (Claim 2; Fig 58); WO2002/16413 (Claim 1; Page 94-95, 105); WO2002/22808 (Claim 2; Fig 1); US5854399 (Example 2; Col 17-18); US5792616 (Fig 2); Cross-references: MIM:187395; NP_003203.1; NM_003212_1
(14) CD21 (CR2 (Complement receptor 2) or C3DR (C3d/Epstein Barr virus receptor) or Hs.73792 Genbank accession no. M26004); Fujisaku et al (1989) J. Biol. Chem. 264 (4):2118-2125); Weis J.J., et al J. Exp. Med. 167, 1047-1066, 1988; Moore M., et al Proc. Natl. Acad. Sci. U.S.A. 84, 9194-9198, 1987; Barel M., et al Mol. Immunol. 35, 1025-1031, 1998; Weis J.J., et al Proc. Natl. Acad. Sci. U.S.A. 83, 5639-5643, 1986; Sinha S.K., et al (1993) J. Immunol. 150, 5311-5320; WO2004/045520 (Example 4); US2004/005538 (Example 1); WO2003/062401 (Claim 9); WO2004/045520 (Example 4); WO91/02536 (Fig 9.1-9.9); WO2004/020595 (Claim 1); Accession: P20023; Q13866; Q14212; EMBL; M26004; AAA35786.1.
(15) CD79b (CD79B, CD79β, IGb (immunoglobulin-associated beta), B29, Genbank accession no. NM_000626 or 11038674); Proc. Natl. Acad. Sci. U.S.A. (2003) 100 (7):4126-4131, Blood (2002) 100 (9):3068-3076, Muller et al (1992) Eur. J. Immunol. 22 (6):1621-1625); WO2004/016225 (claim 2, Fig 140); WO2003/087768, US2004/101874 (claim 1, page 102); WO2003/062401 (claim 9); WO2002/78524 (Example 2); US2002/150573 (claim 5, page 15); US5644033; WO2003/048202 (claim 1, pages 306 and 309); WO 99/58658, US6534482 (claim 13, Fig 17A/B); WO2000/55351 (claim 11, pages 1145-1146); Cross-references: MIM:147245; NP_000617.1; NM_000626_1
(16) FcRH2 (IFGP4, IRTA4, SPAP1A (SH2 domain containing phosphatase anchor protein 1a), SPAP1B, SPAP1C, Genbank accession no. NM_030764, AY358130); Genome Res. 13 (10):2265-2270 (2003), Immunogenetics 54 (2):87-95 (2002), Blood 99 (8):2662-2669 (2002), Proc. Natl. Acad. Sci. U.S.A. 98 (17):9772-9777 (2001), Xu, M.J., et al (2001) Biochem. Biophys. Res. Commun. 280 (3):768-775; WO2004/016225 (Claim 2); WO2003/077836; WO2001/38490 (Claim 5; Fig 18D-1-18D-2); WO2003/097803 (Claim 12); WO2003/089624 (Claim 25); Cross-references: MIM:606509; NP_110391.2; NM_030764_1
(17) HER2 (ErbB2, Genbank accession no. M11730); Coussens L., et al Science (1985) 230(4730):1132-1139); Yamamoto T., et al Nature 319, 230-234, 1986; Semba K., et al Proc. Natl. Acad. Sci. U.S.A. 82, 6497-6501, 1985; Swiercz J.M., et al J. Ce// Biol. 165, 869-880, 2004; Kuhns J.J., et al J. Biol. Chem. 274, 36422-36427, 1999; Cho H.-S., et al Nature 421, 756-760, 2003; Ehsani A., et al (1993) Genomics 15, 426-429; WO2004/048938 (Example 2); WO2004/027049 (Fig 1I); WO2004/009622; WO2003/081210; WO2003/089904 (Claim 9); WO2003/016475 (Claim 1); US2003/118592; WO2003/008537 (Claim 1); WO2003/055439 (Claim 29; Fig 1A-B); WO2003/025228 (Claim 37; Fig 5C); WO2002/22636 (Example 13; Page 95-107); WO2002/12341 (Claim 68; Fig 7); WO2002/13847 (Page 71-74); WO2002/14503 (Page 114-117); WO2001/53463 (Claim 2; Page 41-46); WO2001/41787 (Page 15); WO2000/44899 (Claim 52; Fig 7); WO2000/20579 (Claim 3; Fig 2); US5869445 (Claim 3; Col 31-38); WO9630514 (Claim 2; Page 56-61); EP1439393 (Claim 7); WO2004/043361 (Claim 7); WO2004/022709; WO2001/00244 (Example 3; Fig 4); Accession: P04626; EMBL; M11767; AAA35808.1. EMBL; M11761; AAA35808.1
(18) NCA (CEACAM6, Genbank accession no. M18728); Barnett T., et al Genomics 3, 59-66, 1988; Tawaragi Y., et al Biochem. Biophys. Res. Commun. 150, 89-96, 1988; Strausberg R.L., et al Proc. Natl. Acad. Sci. U.S.A. 99:16899-16903, 2002; WO2004/063709; EP1439393 (Claim 7); WO2004/044178 (Example 4); WO2004/031238; WO2003/042661 (Claim 12); WO2002/78524 (Example 2); WO2002/86443 (Claim 27; Page 427); WO2002/60317 (Claim 2); Accession: P40199; Q14920; EMBL; M29541; AAA59915.1. EMBL; M18728
(19) MDP (DPEP1, Genbank accession no. BC017023); Proc. Natl. Acad. Sci. U.S.A. 99 (26):16899-16903 (2002)); WO2003/016475 (Claim 1); WO2002/64798 (Claim 33; Page 85-87); JP05003790 (Fig 6-8); WO99/46284 (Fig 9); Cross-references: MIM:179780; AAH17023.1; BC017023_1
(20) IL20Rα (IL20Ra, ZCYTOR7, Genbank accession no. AF184971); Clark H.F., et al Genome Res. 13, 2265-2270, 2003; Mungall A.J., et al Nature 425, 805-811, 2003; Blumberg H., et al Cell 104, 9-19, 2001; Dumoutier L., et al J. Immunol. 167, 3545-3549, 2001; Parrish-Novak J., et al J. Biol. Chem. 277, 47517-47523, 2002; Pletnev S., et al (2003) Biochemistry 42:12617-12624; Sheikh F., et al (2004) J. Immunol. 172, 2006-2010; EP1394274 (Example 11); US2004/005320 (Example 5); WO2003/029262 (Page 74-75); WO2003/002717 (Claim 2; Page 63); WO2002/22153 (Page 45-47); US2002/042366 (Page 20-21); WO2001/46261 (Page 57-59); WO2001/46232 (Page 63-65); WO98/37193 (Claim 1; Page 55-59); Accession: Q9UHF4; Q6UWA9; Q96SH8; EMBL; AF184971; AAF01320.1.
(21) Brevican (BCAN, BEHAB, Genbank accession no. AF229053); Gary S.C., et al Gene 256, 139-147, 2000; Clark H.F., et al Genome Res. 13, 2265-2270, 2003; Strausberg R.L., et al Proc. Natl. Acad. Sci. U.S.A. 99, 16899-16903, 2002; US2003/186372 (Claim 11); US2003/186373 (Claim 11); US2003/119131 (Claim 1; Fig 52); US2003/119122 (Claim 1; Fig 52); US2003/119126 (Claim 1); US2003/119121 (Claim 1; Fig 52); US2003/119129 (Claim 1); US2003/119130 (Claim 1); US2003/119128 (Claim 1; Fig 52); US2003/119125 (Claim 1); WO2003/016475 (Claim 1); WO2002/02634 (Claim 1)
(22) EphB2R (DRT, ERK, Hek5, EPHT3, Tyro5, Genbank accession no. NM_004442); Chan, J. and Watt, V.M., Oncogene 6 (6), 1057-1061 (1991) Oncogene 10 (5):897-905 (1995), Annu. Rev. Neurosci. 21:309-345 (1998), Int. Rev. Cytol. 196:177-244 (2000)); WO2003042661 (Claim 12); WO200053216 (Claim 1; Page 41); WO2004065576 (Claim 1); WO2004020583 (Claim 9); WO2003004529 (Page 128-132); WO200053216 (Claim 1; Page 42); Cross-references: MIM:600997; NP_004433.2; NM_004442_1
(23) ASLG659 (B7h, Genbank accession no. AX092328); US2004/0101899 (Claim 2); WO2003104399 (Claim 11); WO2004000221 (Fig 3); US2003/165504 (Claim 1); US2003/124140 (Example 2); US2003/065143 (Fig 60); WO2002/102235 (Claim 13; Page 299); US2003/091580 (Example 2); WO2002/10187 (Claim 6; Fig 10); WO2001/94641 (Claim 12; Fig 7b); WO2002/02624 (Claim 13; Fig 1A-1B); US2002/034749 (Claim 54; Page 45-46); WO2002/06317 (Example 2; Page 320-321, Claim 34; Page 321-322); WO2002/71928 (Page 468-469); WO2002/02587 (Example 1; Fig 1); WO2001/40269 (Example 3; Pages 190-192); WO2000/36107 (Example 2; Page 205-207); WO2004/053079 (Claim 12); WO2003/004989 (Claim 1); WO2002/71928 (Page 233-234, 452-453); WO 01/16318
(24) PSCA (Prostate stem cell antigen precursor, Genbank accession no. AJ297436); Reiter R.E., et al Proc. Natl. Acad. Sci. U.S.A. 95, 1735-1740, 1998; Gu Z., et al Oncogene 19, 1288-1296, 2000; Biochem. Biophys. Res. Commun. (2000) 275(3):783-788; WO2004/022709; EP1394274 (Example 11); US2004/018553 (Claim 17); WO2003/008537 (Claim 1); WO2002/81646 (Claim 1; Page 164); WO2003/003906 (Claim 10; Page 288); WO2001/40309 (Example 1; Fig 17); US2001/055751 (Example 1; Fig 1b); WO2000/32752 (Claim 18; Fig 1); WO98/51805 (Claim 17; Page 97); WO98/51824 (Claim 10; Page 94); WO98/40403 (Claim 2; Fig 1B); Accession: 043653; EMBL; AF043498; AAC39607.1
(25) GEDA (Genbank accession No. AY260763); AAP14954 lipoma HMGIC fusion-partner-like protein /pid=AAP14954.1 - Homo sapiens (human); WO2003/054152 (Claim 20); WO2003/000842 (Claim 1); WO2003/023013 (Example 3, Claim 20); US2003/194704 (Claim 45); Cross-references: GI:30102449; AAP14954.1; AY260763_1
(26) BAFF-R (B cell -activating factor receptor, BLyS receptor 3, BR3, Genbank accession No. AF116456); BAFF receptor /pid=NP_443177.1 - Homo sapiens: Thompson, J.S., et al Science 293 (5537), 2108-2111 (2001); WO2004/058309; WO2004/011611; WO2003/045422 (Example; Page 32-33); WO2003/014294 (Claim 35; Fig 6B); WO2003/035846 (Claim 70; Page 615-616); WO2002/94852 (Col 136-137); WO2002/38766 (Claim 3; Page 133); WO2002/24909 (Example 3; Fig 3); Cross-references: MIM:606269; NP_443177.1; NM_052945_1; AF132600
(27) CD22 (B-cell receptor CD22-B isoform, BL-CAM, Lyb-8, Lyb8, SIGLEC-2, FLJ22814, Genbank accession No. AK026467); Wilson et al (1991) J. Exp. Med. 173:137-146; WO2003/072036 (Claim 1; Fig 1); Cross-references: MIM:107266; NP_001762.1; NM_001771_1
(28) CD79a (CD79A, CD79α, immunoglobulin-associated alpha, a B cell-specific protein that covalently interacts with Ig beta (CD79B) and forms a complex on the surface with Ig M molecules, transduces a signal involved in B-cell differentiation), pl: 4.84, MW: 25028 TM: 2 [P] Gene Chromosome: 19q13.2, Genbank accession No. NP_001774.10); WO2003/088808, US2003/0228319; WO2003/062401 (claim 9); US2002/150573 (claim 4, pages 13-14); WO99/58658 (claim 13, Fig 16); WO92/07574 (Fig 1); US5644033; Ha et al (1992) J. Immunol. 148(5):1526-1531; Müller et al (1992) Eur. J. Immunol.. 22:1621-1625; Hashimoto et al (1994) Immunogenetics 40(4):287-295; Preud'homme et al (1992) Clin. Exp. Immunol. 90(1):141-146; Yu et al (1992) J. Immunol. 148(2) 633-637; Sakaguchi et al (1988) EMBO J. 7(11):3457-3464
(29) CXCR5 (Burkitt's lymphoma receptor 1, a G protein-coupled receptor that is activated by the CXCL13 chemokine, functions in lymphocyte migration and humoral defense, plays a role in HIV-2 infection and perhaps development of AIDS, lymphoma, myeloma, and leukemia); 372 aa, pl: 8.54 MW: 41959 TM: 7 [P] Gene Chromosome: 11 q23.3, Genbank accession No. NP_001707.1); WO2004/040000; WO2004/015426; US2003/105292 (Example 2); US6555339 (Example 2); WO2002/61087 (Fig 1); WO2001/57188 (Claim 20, page 269); WO2001/72830 (pages 12-13); WO2000/22129 (Example 1, pages 152-153, Example 2, pages 254-256); WO99/28468 (claim 1, page 38); US5440021 (Example 2, col 49-52); WO94/28931 (pages 56-58); WO92/17497 (claim 7, Fig 5); Dobner et al (1992) Eur. J. Immunol. 22:2795-2799; Barella et al (1995) Biochem. J. 309:773-779
(30) HLA-DOB (Beta subunit of MHC class II molecule (Ia antigen) that binds peptides and presents them to CD4+ T lymphocytes); 273 aa, pl: 6.56, MW: 30820.TM: 1 [P] Gene Chromosome: 6p21.3, Genbank accession No. NP_002111.1); Tonnelle et al (1985) EMBO J. 4(11):2839-2847; Jonsson et al (1989) Immunogenetics 29(6):411-413; Beck et al (1992) J. Mol. Biol. 228:433-441; Strausberg et al (2002) Proc. Natl. Acad. Sci USA 99:16899-16903; Servenius et al (1987) J. Biol. Chem. 262:8759-8766; Beck et al (1996) J. Mol. Biol. 255:1-13; Naruse et al (2002) Tissue Antigens 59:512-519; WO99/58658 (claim 13, Fig 15); US6153408 (Col 35-38); US5976551 (col 168-170); US6011146 (col 145-146); Kasahara et al (1989) Immunogenetics 30(1):66-68; Larhammar et al (1985) J. Biol. Chem. 260(26):14111-14119
(31) P2X5 (Purinergic receptor P2X ligand-gated ion channel 5, an ion channel gated by extracellular ATP, may be involved in synaptic transmission and neurogenesis, deficiency may contribute to the pathophysiology of idiopathic detrusor instability); 422 aa), pi: 7.63, MW: 47206 TM: 1 [P] Gene Chromosome: 17p13.3, Genbank accession No. NP_002552.2); Le *et al* (1997) *FEBS Lett.* 418(1-2):195-199; WO2004/047749; WO2003/072035 (claim 10); Touchman et al (2000) Genome Res. 10:165-173; WO2002/22660 (claim 20); WO2003/093444 (claim 1); WO2003/087768 (claim 1); WO2003/029277 (page 82)
(32) CD72 (B-cell differentiation antigen CD72, Lyb-2); 359 aa, pi: 8.66, MW: 40225, TM: 1 [P] Gene Chromosome: 9p13.3, Genbank accession No. NP_001773.1); WO2004042346 (claim 65); WO2003/026493 (pages 51-52, 57-58); WO2000/75655 (pages 105-106); Von Hoegen et al (1990) J. Immunol. 144(12):4870-4877; Strausberg et al (2002) Proc. Natl. Acad. Sci USA 99:16899-16903.
(33) LY64 (Lymphocyte antigen 64 (RP105), type I membrane protein of the leucine rich repeat (LRR) family, regulates B-cell activation and apoptosis, loss of function is associated with increased disease activity in patients with systemic lupus erythematosis); 661 aa, pi: 6.20, MW: 74147 TM: 1 [P] Gene Chromosome: 5q12, Genbank accession No. NP_005573.1); US2002/193567; WO97/07198 (claim 11, pages 39-42); Miura et al (1996) Genomics 38(3):299-304; Miura et al (1998) Blood 92:2815-2822; WO2003/083047; WO97/44452 (claim 8, pages 57-61); WO2000/12130 (pages 24-26)
(34) FcRH1 (Fc receptor-like protein 1, a putative receptor for the immunoglobulin Fc domain that contains C2 type Ig-like and ITAM domains, may have a role in B-lymphocyte differentiation); 429 aa, pi: 5.28, MW: 46925 TM: 1 [P] Gene Chromosome: 1q21-1q22, Genbank accession No. NP_443170.1); WO2003/077836; WO2001/38490 (claim 6, Fig 18E-1-18-E-2); Davis et al (2001) Proc. Natl. Acad. Sci USA 98(17):9772-9777; WO2003/089624 (claim 8); EP1347046 (claim 1); WO2003/089624 (claim 7)
(35) IRTA2 (Immunoglobulin superfamily receptor translocation associated 2, a putative immunoreceptor with possible roles in B cell development and lymphomagenesis; deregulation of the gene by translocation occurs in some B cell malignancies); 977 aa, pi: 6.88, MW: 106468, TM: 1 [P] Gene Chromosome: 1q21, Genbank accession No. Human:AF343662, AF343663, AF343664, AF343665, AF369794, AF397453, AK090423, A K090475, AL834187, AY358085; Mouse:AK089756, AY158090, AY506558; NP_112571.1; WO2003/024392 (claim 2, Fig 97); Nakayama et al (2000) Biochem. Biophys. Res. Commun. 277(1):124-127; WO2003/077836; WO2001/38490 (claim 3, Fig 18B-1-18B-2)
(36) TENB2 (TMEFF2, tomoregulin, TPEF, HPP1, TR, putative transmembrane proteoglycan, related to the EGF/heregulin family of growth factors and follistatin); 374 aa, NCBI Accession: AAD55776, AAF91397, AAG49451, NCBI RefSeq: NP_057276; NCBI Gene: 23671; OMIM: 605734; SwissProt Q9UIK5; Genbank accession No. AF179274; AY358907, CAF85723, CQ782436; WO2004/074320; JP2004113151; WO2003/042661; WO2003/009814; EP1295944 (pages 69-70); WO2002/30268 (page 329); WO2001/90304; US2004/249130; US2004/022727; WO2004/063355; US2004/197325; US2003/232350; US2004/005563; US2003/124579; Horie et al (2000) Genomics 67:146-152; Uchida et al (1999) Biochem. Biophys. Res. Commun. 266:593-602; Liang et al (2000) Cancer Res. 60:4907-12; Glynne-Jones et al (2001) Int J Cancer. Oct 15; 94(2):178-84.
(37) PMEL17 (silver homolog; SILV; D12S53E; PMEL17; SI; SIL); ME20; gp100) BC001414; BT007202; M32295; M77348; NM_006928; McGlinchey, R.P. et al (2009) Proc. Natl. Acad. Sci. U.S.A. 106 (33), 13731-13736; Kummer, M.P. et al (2009) J. Biol. Chem. 284 (4), 2296-2306;
(38) TMEFF1 (transmembrane protein with EGF-like and two follistatin-like domains 1; Tomoregulin-1); H7365; C9orf2; C9ORF2; U19878; X83961; NM_080655; NM_003692; Harms, P.W. (2003) Genes Dev. 17 (21), 2624-2629; Gery, S. et al (2003) Oncogene 22 (18):2723-2727;
(39) GDNF-Ra1 (GDNF family receptor alpha 1; GFRA1; GDNFR; GDNFRA; RETL1; TRNR1; RET1L; GDNFR-alpha1; GFR-ALPHA-1); U95847; BC014962; NM_145793 NM_005264; Kim, M.H. et al (2009) Mol. Cell. Biol. 29 (8), 2264-2277; Treanor, J.J. et al (1996) Nature 382 (6586):80-83;
(40) Ly6E (lymphocyte antigen 6 complex, locus E; Ly67,RIG-E,SCA-2,TSA-1); NP_002337.1; NM_002346.2; de Nooij-van Dalen, A.G. et al (2003) Int. J. Cancer 103 (6), 768-774; Zammit, D.J. et al (2002) Mol. Cell. Biol. 22 (3):946-952;
(41) TMEM46 (shisa homolog 2 (Xenopus laevis); SHISA2); NP_001007539.1; NM_001007538.1; Furushima, K. et al (2007) Dev. Biol. 306 (2), 480-492; Clark, H.F. et al (2003) Genome Res. 13 (10):2265-2270;
(42) Ly6G6D (lymphocyte antigen 6 complex, locus G6D; Ly6-D, MEGT1); NP_067079.2; NM_021246.2; Mallya, M. et al (2002) Genomics 80 (1):113-123; Ribas, G. et al (1999) J. Immunol. 163 (1):278-287;
(43) LGR5 (leucine-rich repeat-containing G protein-coupled receptor 5; GPR49, GPR67); NP_003658.1; NM_003667.2; Salanti, G. et al (2009) Am. J. Epidemiol. 170 (5):537-545; Yamamoto, Y. et al (2003) Hepatology 37 (3):528-533;
(44) RET (ret proto-oncogene; MEN2A; HSCR1; MEN2B; MTC1; PTC; CDHF12; Hs.168114; RET51; RET-ELE1); NP_066124.1; NM_020975.4; Tsukamoto, H. et al (2009) Cancer Sci. 100 (10):1895-1901; Narita, N. et al (2009) Oncogene 28 (34):3058-3068;
(45) LY6K (lymphocyte antigen 6 complex, locus K; LY6K; HSJ001348; FLJ35226); NP_059997.3; NM_017527.3; Ishikawa, N. et al (2007) Cancer Res. 67 (24):11601-11611; de Nooij-van Dalen, A.G. et al (2003) Int. J. Cancer 103 (6):768-774;
(46) GPR19 (G protein-coupled receptor 19; Mm.4787); NP_006134.1; NM_006143.2; Montpetit, A. and Sinnett, D. (1999) Hum. Genet. 105 (1-2):162-164; O'Dowd, B.F. et al (1996) FEBS Lett. 394 (3):325-329;
(47) GPR54 (KISS1 receptor; KISS1 R; GPR54; HOT7T175; AXOR12); NP_115940.2; NM_032551.4; Navenot, J.M. et al (2009) Mol. Pharmacol. 75 (6):1300-1306; Hata, K. et al (2009) Anticancer Res. 29 (2):617-623;
(48) ASPHD1 (aspartate beta-hydroxylase domain containing 1; LOC253982); NP_859069.2; NM_181718.3; Gerhard, D.S. et al (2004) Genome Res. 14 (10B):2121-2127;
(49) Tyrosinase (TYR; OCAIA; OCA1A; tyrosinase; SHEP3); NP_000363.1; NM_000372.4; Bishop, D.T. et al (2009) Nat. Genet. 41 (8):920-925; Nan, H. et al (2009) Int. J. Cancer 125 (4):909-917;
(50) TMEM118 (ring finger protein, transmembrane 2; RNFT2; FLJ14627); NP_001103373.1; NM_001109903.1; Clark, H.F. et al (2003) Genome Res. 13 (10):2265-2270; Scherer, S.E. et al (2006) Nature 440 (7082):346-351
(51) GPR172A (G protein-coupled receptor 172A; GPCR41; FLJ11856; D15Ertd747e); NP_078807.1; NM_024531.3; Ericsson, T.A. et al (2003) Proc. Natl. Acad. Sci. U.S.A. 100 (11):6759-6764; Takeda, S. et al (2002) FEBS Lett. 520 (1-3):97-101.
(52) CD33, a member of the sialic acid binding, immunoglobulin-like lectin family, is a 67-kDa glycosylated transmembrane protein. CD33is expressed on most myeloid and monocytic leukemia cells in addition to committed myelomonocytic and erythroid progenitor cells. It is not seen on the earliest pluripotent stem cells, mature granulocytes, lymphoid cells, or nonhematopoietic cells (Sabbath et al., (1985) J. Clin. Invest. 75:756-56; Andrews et al., (1986) Blood 68:1030-5). CD33 contains two tyrosine residues on its cytoplasmic tail, each of which is followed by hydrophobic residues similar to the immunoreceptor tyrosine-based inhibitory motif (ITIM) seen in many inhibitory receptors.
(53) CLL-1 (CLEC12A, MICL, and DCAL2), encodes a member of the C-type lectin/C-type lectin-like domain (CTL/CTLD) superfamily. Members of this family share a common protein fold and have diverse functions, such as cell adhesion, cell-cell signaling, glycoprotein turnover, and roles in inflammation and immune response. The protein encoded by this gene is a negative regulator of granulocyte and monocyte function. Several alternatively spliced transcript variants of this gene have been described, but the full-length nature of some of these variants has not been determined. This gene is closely linked to other CTL/CTLD superfamily members in the natural killer gene complex region on chromosome 12p13 (Drickamer K (1999) Curr. Opin. Struct. Biol. 9 (5):585-90; van Rhenen A, et al., (2007) Blood 110 (7):2659-66; Chen CH, et al. (2006) Blood 107 (4):1459-67; Marshall AS, et al. (2006) Eur. J. Immunol. 36 (8):2159-69; Bakker AB, et al (2005) Cancer Res. 64 (22):8443-50; Marshall AS, et al (2004) J. Biol. Chem. 279 (15):14792-802). CLL-1 has been shown to be a type II transmembrane receptor comprising a single C-type lectin-like domain (which is not predicted to bind either calcium or sugar), a stalk region, a transmembrane domain and a short cytoplasmic tail containing an ITIM motif.

The parent antibody may also be a fusion protein comprising an albumin-binding peptide (ABP) sequence (Dennis et al. (2002) "Albumin Binding As A General Strategy For Improving The Pharmacokinetics Of Proteins" J Biol Chem. 277:35035-35043; WO 01/45746). Antibodies of the invention include fusion proteins with ABP sequences taught by: (i) Dennis et al (2002) J Biol Chem. 277:35035-35043 at Tables III and IV, page 35038; (ii) US 2004/0001827 at [0076]; and (iii) WO 01/45746 at pages 12-13, and all of which are incorporated herein by reference.

In one embodiment, the antibody has been raised to target specific the tumour related antigen αᵥβ₆.

In certain embodiments, the ADCs of the present invention comprise anti-HER2 antibodies. In one embodiment of the invention, an anti-HER2 antibody of an ADC of the invention comprises a humanized anti-HER2 antibody, e.g., huMAb4D5-1, huMAb4D5-2, huMAb4D5-3, huMAb4D5-4, huMAb4D5-5, huMAb4D5-6, huMAb4D5-7 and huMAb4D5-8, as described in Table 3 of US 5821337. Those antibodies contain human framework regions with the complementarity-determining regions of a murine antibody (4D5) that binds to HER2. The humanized antibody huMAb4D5-8 is also referred to as trastuzumab, commercially available as HERCEPTIN®. In another embodiment of the invention, an anti-HER2 antibody of an ADC of the invention comprises a humanized anti-HER2 antibody, e.g., humanized 2C4, as described in US7862817. An exemplary humanized 2C4 antibody is pertuzumab, commercially available as PERJETA®.

In another embodiment of the invention, an anti-HER2 antibody of an ADC of the invention comprises a humanized anti-HER2 antibody is 7C2.

In some embodiments, the cysteine-engineered THIOMAB™ antibodies have a cysteine residue introduced at the 149-lysine site of the light chain (LC K149C) according to the numbering of Kabat.

In other embodiments, the cysteine-engineered THIOMAB™ antibodies have a cysteine residue introduced at the 205-valine site of the light chain (LC V205C) according to the numbering of Kabat.

In other embodiments, the cysteine-engineered THIOMAB™ antibodies have a cysteine residue introduced at the 118-alanine site (EU numbering) of the heavy chain (HC A118C). This site is alternatively numbered 121 by Sequential numbering or 114 by Kabat numbering.

In other embodiments, the cysteine-engineered THIOMAB™ antibodies have a cysteine residue introduced at the 140-alanine site (EU numbering) of the heavy chain (HC A140C). This site is alternatively numbered 143 by Sequential numbering or 136 by Kabat numbering.

In other embodiments, the cysteine-engineered THIOMAB™ antibodies have a cysteine residue introduced at the 239-serine site (EU numbering) of the heavy chain (HC S239C). This site is alternatively numbered 242 by Sequential numbering or 235 by Kabat numbering.

The cell binding agent may be labelled, for example to aid detection or purification of the agent either prior to incorporation as a conjugate, or as part of the conjugate. The label may be a biotin label. In another embodiment, the cell binding agent may be labelled with a radioisotope.

### R and R'

In one embodiment, R is independently selected from optionally substituted C₁₋₁₂ alkyl, C₃₋₂₀ heterocyclyl and C₅₋₂₀ aryl groups. These groups are each defined in the substituents section below.

In one embodiment, R is independently optionally substituted C₁₋₁₂ alkyl.

In one embodiment, R is independently optionally substituted C₃₋₂₀ heterocyclyl.

In one embodiment, R is independently optionally substituted C₅₋₂₀ aryl.

In one embodiment, R is independently optionally substituted C₁₋₁₂ alkyl.

Described above in relation to R² are various embodiments relating to preferred alkyl and aryl groups and the identity and number of optional substituents. The preferences set out for R² as it applies to R are applicable, where appropriate, to all other groups R, for examples where R⁶, R⁷, R⁸ or R⁹ is R.

The preferences for R apply also to R'.

In some embodiments of the invention there is provided a compound having a substituent group -NRR'. In one embodiment, R and R' together with the nitrogen atom to which they are attached form an optionally substituted 4-, 5-, 6- or 7-membered heterocyclic ring. The ring may contain a further heteroatom, for example N, O or S.

In one embodiment, the heterocyclic ring is itself substituted with a group R. Where a further N heteroatom is present, the substituent may be on the N heteroatom.

### R"

R" is a C₃₋₁₂ alkylene group, which chain may be interrupted by one or more heteroatoms, e.g. O, S, N(H), NMe and/or aromatic rings, e.g. benzene or pyridine, which rings are optionally substituted.

In one embodiment, R" is a C₃₋₁₂ alkylene group, which chain may be interrupted by one or more heteroatoms and/or aromatic rings, e.g. benzene or pyridine.

In one embodiment, the alkylene group is optionally interrupted by one or more heteroatoms selected from O, S, and NMe and/or aromatic rings, which rings are optionally substituted.

In one embodiment, the aromatic ring is a C₅₋₂₀ arylene group, where arylene pertains to a divalent moiety obtained by removing two hydrogen atoms from two aromatic ring atoms of an aromatic compound, which moiety has from 5 to 20 ring atoms.

In one embodiment, R" is a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂.

In one embodiment, R" is a C₃₋₁₂ alkylene group.

In one embodiment, R" is selected from a C₃, C₅, C₇, C₉ and a C₁₁ alkylene group.

In one embodiment, R" is selected from a C₃, C₅ and a C₇ alkylene group.

In one embodiment, R" is selected from a C₃ and a C₅ alkylene group.

In one embodiment, R" is a C₃ alkylene group.

In one embodiment, R" is a C₅ alkylene group.

The alkylene groups listed above may be optionally interrupted by one or more heteroatoms and/or aromatic rings, e.g. benzene or pyridine, which rings are optionally substituted.

The alkylene groups listed above may be optionally interrupted by one or more heteroatoms and/or aromatic rings, e.g. benzene or pyridine.

The alkylene groups listed above may be unsubstituted linear aliphatic alkylene groups.

### X

In one embodiment, X is selected from O, S, or N(H).

Preferably, X is O.

### E

The compounds where one or both C rings is replaced by a ring of formula E, have a group R² which with either of R¹ or R³, together with carbon atoms of the C ring to which they are attached, form an optionally substituted benzene ring. The optionally substituted benzene ring may be regarded as fused to the C ring of the pyrrolobenzodiazepine. The fused benzene ring may be referred to as the D ring. The structure of the fused ring is illustrated below: 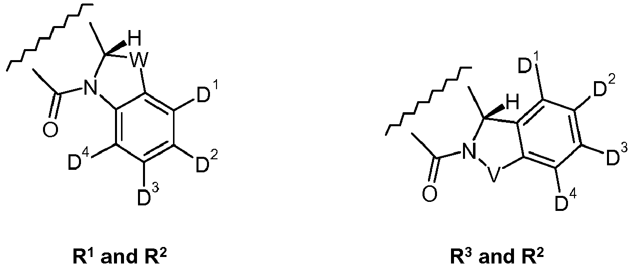 where each of D¹, D², D³ and D⁴ represents H or a substituent.

In one embodiment, the benzene ring is unsubstituted.

In one embodiment, the benzene ring is optionally substituted with one, two, three of four groups selected from OH, CN, R, OR, O-SO₂-R, CO₂R, COR, SH, SR, NH₂, NHR, NRR', NO₂, Me₃Sn and halo.

In one embodiment, the benzene ring is monosubstituted. The monosubstituent may be any one of D¹, D², D³ or D⁴ (the rest being H). In one embodiment the benzene ring is substituted at D², and D¹, D³ and D⁴ are each H. In one embodiment the benzene ring is substituted at D³, and D¹, D² and D⁴ are each H.

In one embodiment, R² with R¹, together with carbon atoms of the C ring to which they are attached, form an optionally substituted benzene ring.

The preferences for V and W are set out below.

### F

In the compounds where one or both C rings is replaced by a ring of formula F:
In one embodiment, U is CH₂ when T is NR, BH, SO, or SO₂.
In one embodiment, T is CH₂ or CO when U is NR, O or S.
In one embodiment, T is selected from CH₂ and CO.
In one embodiment, U is selected from NR, O and S.
In one embodiment, Y is (CH₂)ₙ, where n is 1 or 2.

In one embodiment, the C ring of the compound A-B has a structure selected from those shown below:

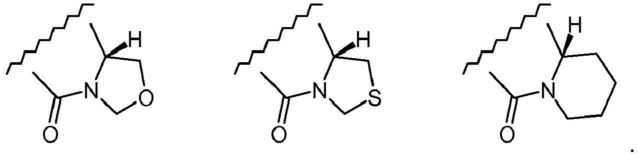

### V and W

V and W are each selected from (CH₂)ₙ, O, S, NR, CHR, and CRR' where n is 2,3 or 4, except that V is C when R¹ and R², together with carbon atoms of the C ring to which they are attached, form an optionally substituted benzene ring, and W is C when R³ and R², together with carbon atoms of the C ring to which they are attached, form an optionally substituted benzene ring.

In one embodiment, when one of V and W is C, the other of V and W is selected from CH₂ and NR.

In one embodiment, when one of V and W is C, the other of V and W is CH₂.

### Preferred Conjugate Compounds

The method of the second aspect of the present invention prepares conjugate compounds from the reaction between a cell binding agent and an intermediate compound of the present invention. The cell binding agent may be an antibody. In a third aspect of the invention, there are provided conjugates with LC K149C, LC V205C, HC A140C, or HC S239C cysteine-engineered antibody mutant (THIOMAB™), where CBA below represents Ab as defined above. Of these LC K149C cysteine-engineered antibody mutant (THIOMAB™) may be preferred.

In one embodiment, the conjugate is a dimer wherein each of the PBD moieties has a C2 methylene group i.e. each R² is =CH₂. It is preferred that the cell binding agent is an antibody.

In another embodiment, the conjugate is a dimer wherein each of the monomers has a C2 aryl group i.e. each R² is optionally substituted C₅₋₂₀ aryl, and there is a double bond between C2 and C3 in each PBD moiety. It is preferred that the cell binding agent is an antibody.

### C2 Alkylene

In one embodiment, the conjugate is a compound: 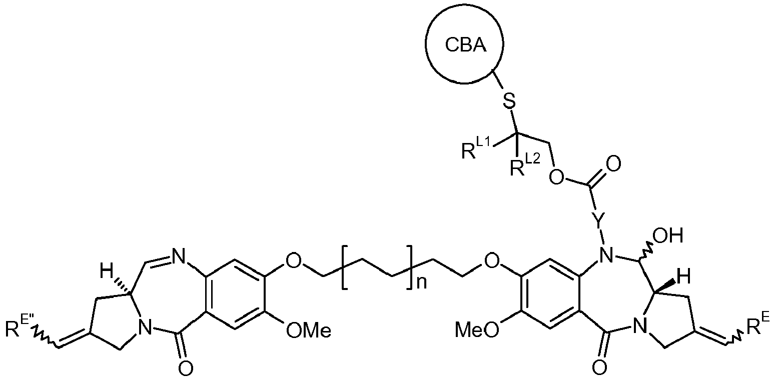 and more preferably: 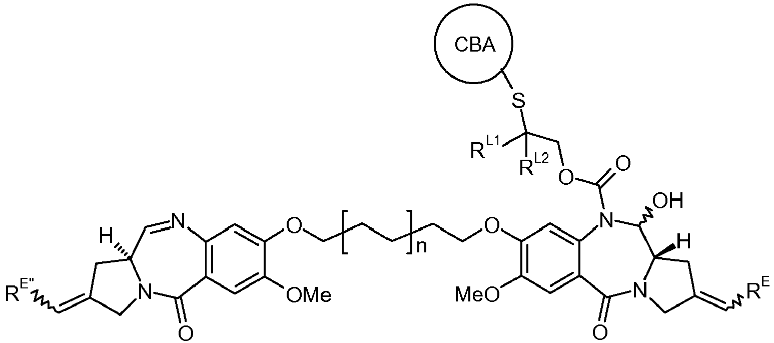 wherein CBA is a cell binding agent such as an antibody or a cyclic or linear peptide, and n is 0 or 1. Y, R^{L1} and R^{L2} are as previously defined, and R^{E} and R^{E}" are each independently selected from H or R^{D}.

### C2 Aryl

In one embodiment, the conjugate is a compound: 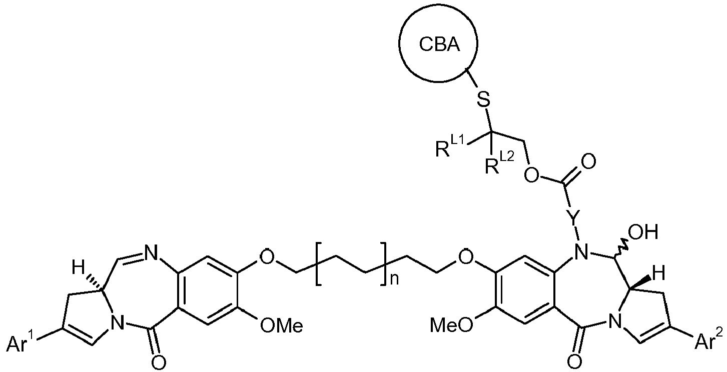 and more preferably: 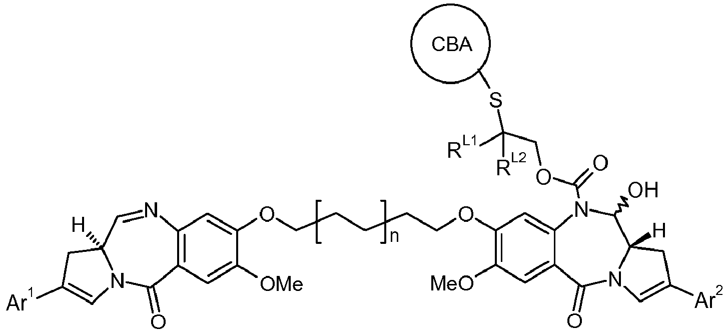 wherein CBA is a cell binding agent such as an antibody or a cyclic or linear peptide, Y, R^{L1} and R^{L2} are as previously defined; Ar¹ and Ar² are each independently optionally substituted C₅₋₂₀ aryl, and n is 0 or 1. Ar¹ and Ar² may be the same or different.

In one embodiment, Ar¹ and Ar² in each of the embodiments above are each independently selected from optionally substituted phenyl, furanyl, thiophenyl and pyridyl.

In one embodiment, Ar¹ and Ar² in each of the embodiments above is optionally substituted phenyl.

In one embodiment, Ar¹ and Ar² in each of the embodiments above is optionally substituted thien-2-yl or thien-3-yl.

In one embodiment, Ar¹ and Ar² in each of the embodiments above is optionally substituted quinolinyl or isoquinolinyl.

The quinolinyl or isoquinolinyl group may be bound to the PBD core through any available ring position. For example, the quinolinyl may be quinolin-2-yl, quinolin-3-yl, quinolin-4yl, quinolin-5-yl, quinolin-6-yl, quinolin-7-yl and quinolin-8-yl. Of these quinolin-3-yl and quinolin-6-yl may be preferred. The isoquinolinyl may be isoquinolin-1-yl, isoquinolin-3-yl, isoquinolin-4yl, isoquinolin-5-yl, isoquinolin-6-yl, isoquinolin-7-yl and isoquinolin-8-yl. Of these isoquinolin-3-yl and isoquinolin-6-yl may be preferred.

### C2 Vinyl

In one embodiment, the conjugate is a compound: 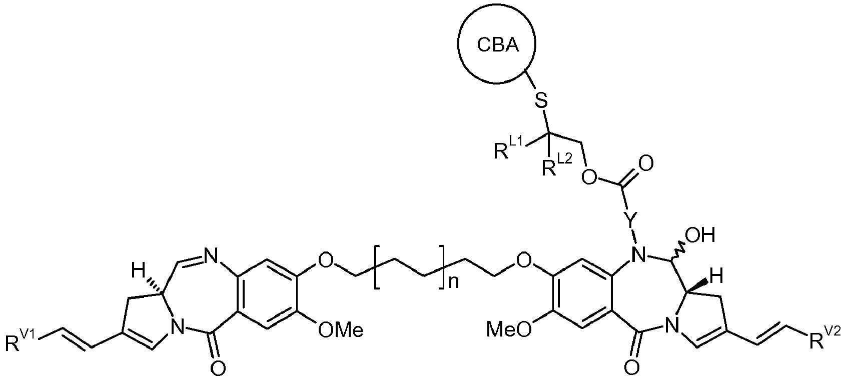 and more preferably: 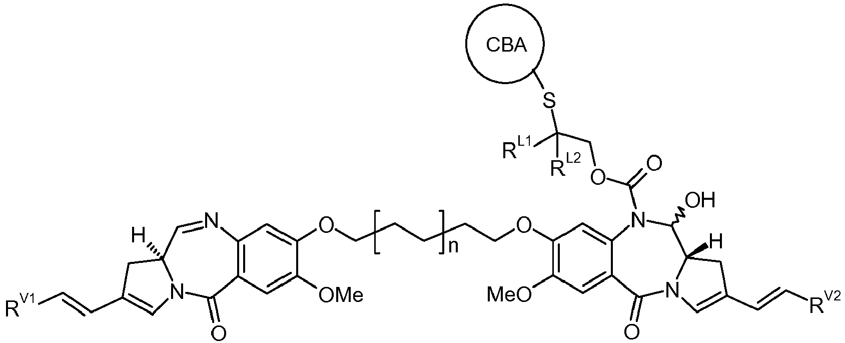 wherein CBA is a cell binding agent such as an antibody or a cyclic or linear peptide, Y, R^{L1} and R^{L2} are as previously defined, R^{V1} and R^{V2} are independently selected from H, methyl, ethyl and phenyl (which phenyl may be optionally substituted with fluoro, particularly in the 4 position) and C₅₋₆ heterocyclyl, and n is 0 or 1. R^{V1} and R^{V2} may be the same or different.

In some of the above embodiments, R^{V1} and R^{V2} may be independently selected from H, phenyl, and 4-fluorophenyl.

For each of the compounds above, the following preferences may apply, where appropriate:
n is 0;
n is 1;
R^{E} is H;
R^{E} is R^{D}, where R^{D} is optionally substituted alkyl;
R^{E} is R^{D}, where R^{D} is methyl;
CBA is an antibody, in particular a LC K149C, LC V205C, HC A140C, or HC S239C cysteine-engineered antibody mutant (THIOMAB™);
CBA is a cyclic peptide;
R^{L1} and R^{L2} are H;
R^{L1} and R^{L2} are Me.

### Substituents

The phrase "optionally substituted" as used herein, pertains to a parent group which may be unsubstituted or which may be substituted.

Unless otherwise specified, the term "substituted" as used herein, pertains to a parent group which bears one or more substituents. The term "substituent" is used herein in the conventional sense and refers to a chemical moiety which is covalently attached to, or if appropriate, fused to, a parent group. A wide variety of substituents are well known, and methods for their formation and introduction into a variety of parent groups are also well known.

In a preferred embodiment, the substituents described herein (which include optional substituents) are limited to those groups that are not reactive to a cell binding agent. The link to the cell binding agent in the present case is formed from the N10 position of the PBD compound through a linker group (comprising, for example, L¹, L² and A) to the cell binding agent. Reactive functional groups located at other parts of the PBD structure may be capable of forming additional bonds to the cell binding agent (this may be referred to as crosslinking). These additional bonds may alter transport and biological activity of the conjugate. Therefore, in some embodiment, the additional substituents are limited to those lacking reactive functionality.

In one embodiment, the substituents are selected from the group consisting of R, OR, SR, NRR', NO₂, halo, CO₂R, COR, CONH₂, CONHR, and CONRR'.

In one embodiment, the substituents are selected from the group consisting of R, OR, SR, NRR', NO₂, CO₂R, COR, CONH₂, CONHR, and CONRR'.

In one embodiment, the substituents are selected from the group consisting of R, OR, SR, NRR', NO₂, and halo.

In one embodiment, the substituents are selected from the group consisting of R, OR, SR, NRR', and NO₂.

Any one of the embodiment mentioned above may be applied to any one of the substituents described herein. Alternatively, the substituents may be selected from one or more of the groups listed below.

Examples of substituents are described in more detail below.

C₁₋₁₂ alkyl: The term "C₁₋₁₂ alkyl" as used herein, pertains to a monovalent moiety obtained by removing a hydrogen atom from a carbon atom of a hydrocarbon compound having from 1 to 12 carbon atoms, which may be aliphatic or alicyclic, and which may be saturated or unsaturated (e.g. partially unsaturated, fully unsaturated). Thus, the term "alkyl" includes the sub-classes alkenyl, alkynyl, cycloalkyl, etc., discussed below.

Examples of saturated alkyl groups include, but are not limited to, methyl (C₁), ethyl (C₂), propyl (C₃), butyl (C₄), pentyl (C₅), hexyl (C₆) and heptyl (C₇).

Examples of saturated linear alkyl groups include, but are not limited to, methyl (C₁), ethyl (C₂), n-propyl (C₃), n-butyl (C₄), n-pentyl (amyl) (C₅), n-hexyl (C₆) and n-heptyl (C₇).

Examples of saturated branched alkyl groups include iso-propyl (C₃), iso-butyl (C₄), sec-butyl (C₄), tert-butyl (C₄), iso-pentyl (C₅), and neo-pentyl (C₅).

An alkyl group may optionally be interrupted by one or more heteroatoms selected from O, N(H) and S. Such groups may be referred to as "heteroalkyl".

C₂₋₂₀ Heteroalkyl: The term "C₂₋₁₂ heteroalkyl" as used herein, pertains to a monovalent moiety obtained by removing a hydrogen atom from a carbon atom of a hydrocarbon compound having from 2 to 12 carbon atoms, and one or more heteroatoms selected from O, N(H) and S, preferably O and S.

Examples of heteroalkyl groups include, but are not limited to those comprising one or more ethylene glycol units of the type -(OCH₂CH₂)-. The terminal of a heteroalkyl group may be the primary form of a heteroatom, e.g. -OH, -SH or -NH₂. In a preferred embodiment, the terminal is -CH₃.

C₂₋₁₂ Alkenyl: The term "C₂₋₁₂ alkenyl" as used herein, pertains to an alkyl group having one or more carbon-carbon double bonds.

Examples of unsaturated alkenyl groups include, but are not limited to, ethenyl (vinyl, -CH=CH₂), 1-propenyl (-CH=CH-CH₃), 2-propenyl (allyl, -CH-CH=CH₂), isopropenyl (1-methylvinyl, -C(CH₃)=CH₂), butenyl (C₄), pentenyl (C₅), and hexenyl (C₆).

C₂₋₁₂ alkynyl: The term "C₂₋₁₂ alkynyl" as used herein, pertains to an alkyl group having one or more carbon-carbon triple bonds.

Examples of unsaturated alkynyl groups include, but are not limited to, ethynyl (-C≡CH) and 2-propynyl (propargyl, -CH₂-C≡CH).

C₃₋₁₂ cycloalkyl: The term "C₃₋₁₂ cycloalkyl" as used herein, pertains to an alkyl group which is also a cyclyl group; that is, a monovalent moiety obtained by removing a hydrogen atom from an alicyclic ring atom of a cyclic hydrocarbon (carbocyclic) compound, which moiety has from 3 to 7 carbon atoms, including from 3 to 7 ring atoms.

Examples of cycloalkyl groups include, but are not limited to, those derived from:
saturated monocyclic hydrocarbon compounds: cyclopropane (C₃), cyclobutane (C₄), cyclopentane (C₅), cyclohexane (C₆), cycloheptane (C₇), methylcyclopropane (C₄), dimethylcyclopropane (C₅), methylcyclobutane (C₅), dimethylcyclobutane (C₆), methylcyclopentane (C₆), dimethylcyclopentane (C₇) and methylcyclohexane (C₇);
unsaturated monocyclic hydrocarbon compounds: cyclopropene (C₃), cyclobutene (C₄), cyclopentene (C₅), cyclohexene (C₆), methylcyclopropene (C₄), dimethylcyclopropene (C₅), methylcyclobutene (C₅), dimethylcyclobutene (C₆), methylcyclopentene (C₆), dimethylcyclopentene (C₇) and methylcyclohexene (C₇); and
saturated polycyclic hydrocarbon compounds: norcarane (C₇), norpinane (C₇), norbornane (C₇).

C₃₋₂₀ heterocyclyl: The term "C₃₋₂₀ heterocyclyl" as used herein, pertains to a monovalent moiety obtained by removing a hydrogen atom from a ring atom of a heterocyclic compound, which moiety has from 3 to 20 ring atoms, of which from 1 to 10 are ring heteroatoms. Preferably, each ring has from 3 to 7 ring atoms, of which from 1 to 4 are ring heteroatoms.

In this context, the prefixes (e.g. C₃₋₂₀, C₃₋₇, C₅₋₆, etc.) denote the number of ring atoms, or range of number of ring atoms, whether carbon atoms or heteroatoms. For example, the term "C₅₋₆heterocyclyl", as used herein, pertains to a heterocyclyl group having 5 or 6 ring atoms.

Examples of monocyclic heterocyclyl groups include, but are not limited to, those derived from:
N₁: aziridine (C₃), azetidine (C₄), pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅), piperidine (C₆), dihydropyridine (C₆), tetrahydropyridine (C₆), azepine (C₇);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), piperazine (C₆);
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂O₁: oxadiazine (C₆);
O₁S₁: oxathiole (C₅) and oxathiane (thioxane) (C₆); and,
N₁O₁S₁: oxathiazine (C₆).

Examples of substituted monocyclic heterocyclyl groups include those derived from saccharides, in cyclic form, for example, furanoses (C₅), such as arabinofuranose, lyxofuranose, ribofuranose, and xylofuranse, and pyranoses (C₆), such as allopyranose, altropyranose, glucopyranose, mannopyranose, gulopyranose, idopyranose, galactopyranose, and talopyranose.

C₅₋₂₀ aryl: The term "C₅₋₂₀ aryl", as used herein, pertains to a monovalent moiety obtained by removing a hydrogen atom from an aromatic ring atom of an aromatic compound, which moiety has from 3 to 20 ring atoms. Preferably, each ring has from 5 to 7 ring atoms.

In this context, the prefixes (e.g. C₃₋₂₀, C₅₋₇, C₅₋₆, etc.) denote the number of ring atoms, or range of number of ring atoms, whether carbon atoms or heteroatoms. For example, the term "C₅₋₆ aryl" as used herein, pertains to an aryl group having 5 or 6 ring atoms.

The ring atoms may be all carbon atoms, as in "carboaryl groups".

Examples of carboaryl groups include, but are not limited to, those derived from benzene (i.e. phenyl) (C₆), naphthalene (C₁₀), azulene (C₁₀), anthracene (C₁₄), phenanthrene (C₁₄), naphthacene (C₁₈), and pyrene (C₁₆).

Examples of aryl groups which comprise fused rings, at least one of which is an aromatic ring, include, but are not limited to, groups derived from indane (e.g. 2,3-dihydro-1H-indene) (C₉), indene (C₉), isoindene (C₉), tetraline (1,2,3,4-tetrahydronaphthalene (C₁₀), acenaphthene (C₁₂), fluorene (C₁₃), phenalene (C₁₃), acephenanthrene (C₁₅), and aceanthrene (C₁₆).

Alternatively, the ring atoms may include one or more heteroatoms, as in "heteroaryl groups". Examples of monocyclic heteroaryl groups include, but are not limited to, those derived from:
N₁: pyrrole (azole) (C₅), pyridine (azine) (C₆);
O₁: furan (oxole) (C₅);
S₁: thiophene (thiole) (C₅);
N₁O₁: oxazole (C₅), isoxazole (C₅), isoxazine (C₆);
N₂O₁: oxadiazole (furazan) (C₅);
N₃O₁: oxatriazole (C₅);
N₁S₁: thiazole (C₅), isothiazole (C₅);
N₂: imidazole (1,3-diazole) (C₅), pyrazole (1,2-diazole) (C₅), pyridazine (1,2-diazine) (C₆), pyrimidine (1,3-diazine) (C₆) (e.g., cytosine, thymine, uracil), pyrazine (1,4-diazine) (C₆);
N₃: triazole (C₅), triazine (C₆); and,
N₄: tetrazole (C₅).

Examples of heteroaryl which comprise fused rings, include, but are not limited to:
C₉ (with 2 fused rings) derived from benzofuran (O₁), isobenzofuran (O₁), indole (N₁), isoindole (N₁), indolizine (N₁), indoline (N₁), isoindoline (N₁), purine (N₄) (e.g., adenine, guanine), benzimidazole (N₂), indazole (N₂), benzoxazole (N₁O₁), benzisoxazole (N₁O₁), benzodioxole (O₂), benzofurazan (N₂O₁), benzotriazole (N₃), benzothiofuran (S₁), benzothiazole (N₁S₁), benzothiadiazole (N₂S);
C₁₀ (with 2 fused rings) derived from chromene (O₁), isochromene (O₁), chroman (O₁), isochroman (O₁), benzodioxan (O₂), quinoline (N₁), isoquinoline (N₁), quinolizine (N₁), benzoxazine (N₁O₁), benzodiazine (N₂), pyridopyridine (N₂), quinoxaline (N₂), quinazoline (N₂), cinnoline (N₂), phthalazine (N₂), naphthyridine (N₂), pteridine (N₄);
C₁₁ (with 2 fused rings) derived from benzodiazepine (N₂);
C₁₃ (with 3 fused rings) derived from carbazole (N₁), dibenzofuran (O₁), dibenzothiophene (S₁), carboline (N₂), perimidine (N₂), pyridoindole (N₂); and,
C₁₄ (with 3 fused rings) derived from acridine (N₁), xanthene (O₁), thioxanthene (S₁), oxanthrene (O₂), phenoxathiin (O₁S₁), phenazine (N₂), phenoxazine (N₁O₁), phenothiazine (N₁S₁), thianthrene (S₂), phenanthridine (N₁), phenanthroline (N₂), phenazine (N₂).

The above groups, whether alone or part of another substituent, may themselves optionally be substituted with one or more groups selected from themselves and the additional substituents listed below.
Halo: -F, -Cl, -Br, and -I.
Hydroxy: -OH.

Ether: -OR, wherein R is an ether substituent, for example, a C₁₋₇ alkyl group (also referred to as a C₁₋₇ alkoxy group, discussed below), a C₃₋₂₀ heterocyclyl group (also referred to as a C₃₋₂₀ heterocyclyloxy group), or a C₅₋₂₀ aryl group (also referred to as a C₅₋₂₀ aryloxy group), preferably a C₁₋₇alkyl group.

Alkoxy: -OR, wherein R is an alkyl group, for example, a C₁₋₇ alkyl group. Examples of C₁₋₇ alkoxy groups include, but are not limited to, -OMe (methoxy), -OEt (ethoxy), -O(nPr) (n-propoxy), -O(iPr) (isopropoxy), -O(nBu) (n-butoxy), -O(sBu) (sec-butoxy), -O(iBu) (isobutoxy), and -O(tBu) (tert-butoxy).

Acetal: -CH(OR¹)(OR²), wherein R¹ and R² are independently acetal substituents, for example, a C₁₋₇ alkyl group, a C₃₋₂₀ heterocyclyl group, or a C₅₋₂₀ aryl group, preferably a C₁₋₇ alkyl group, or, in the case of a "cyclic" acetal group, R¹ and R², taken together with the two oxygen atoms to which they are attached, and the carbon atoms to which they are attached, form a heterocyclic ring having from 4 to 8 ring atoms. Examples of acetal groups include, but are not limited to, -CH(OMe)₂, -CH(OEt)₂, and -CH(OMe)(OEt).

Hemiacetal: -CH(OH)(OR¹), wherein R¹ is a hemiacetal substituent, for example, a C₁₋₇ alkyl, group, a C₃₋₂₀ heterocyclyl group, or a C₅₋₂₀ aryl group, preferably a C₁₋₇ alkyl group. Examples of hemiacetal groups include, but are not limited to, -CH(OH)(OMe) and - CH(OH)(OEt).

Ketal: -CR(OR¹)(OR²), where R¹ and R² are as defined for acetals, and R is a ketal substituent other than hydrogen, for example, a C₁₋₇ alkyl group, a C₃₋₂₀ heterocyclyl group, or a C₅₋₂₀ aryl group, preferably a C₁₋₇ alkyl group. Examples ketal groups include, but are not limited to, -C(Me)(OMe)₂, -C(Me)(OEt)₂, -C(Me)(OMe)(OEt), -C(Et)(OMe)₂, -C(Et)(OEt)₂, and -C(Et)(OMe)(OEt).

Hemiketal: -CR(OH)(OR¹), where R¹ is as defined for hemiacetals, and R is a hemiketal substituent other than hydrogen, for example, a C₁₋₇ alkyl group, a C₃₋₂₀ heterocyclyl group, or a C₅₋₂₀ aryl group, preferably a C₁₋₇ alkyl group. Examples of hemiacetal groups include, but are not limited to, -C(Me)(OH)(OMe), -C(Et)(OH)(OMe), -C(Me)(OH)(OEt), and -C(Et)(OH)(OEt).
Oxo (keto, -one): =O.
Thione (thioketone): =S.

Imino (imine): =NR, wherein R is an imino substituent, for example, hydrogen, C₁₋₇ alkyl group, a C₃₋₂₀ heterocyclyl group, or a C₅₋₂₀ aryl group, preferably hydrogen or a C₁₋₇ alkyl, group. Examples of ester groups include, but are not limited to, =NH, =NMe, =NEt, and =NPh.
Formyl (carbaldehyde, carboxaldehyde): -C(=O)H.

Acyl (keto): -C(=O)R, wherein R is an acyl substituent, for example, a C₁₋₇ alkyl group (also referred to as C₁₋₇ alkylacyl or C₁₋₇ alkanoyl), a C₃₋₂₀ heterocyclyl group (also referred to as C₃₋₂₀ heterocyclylacyl), or a C₅₋₂₀ aryl group (also referred to as C₅₋₂₀ arylacyl), preferably a C₁₋₇ alkyl group. Examples of acyl groups include, but are not limited to, -C(=O)CH₃ (acetyl), -C(=O)CH₂CH₃ (propionyl), -C(=O)C(CH₃)₃ (t-butyryl), and -C(=O)Ph (benzoyl, phenone).
Carboxy (carboxylic acid): -C(=O)OH.
Thiocarboxy (thiocarboxylic acid): -C(=S)SH.
Thiolocarboxy (thiolocarboxylic acid): -C(=O)SH.
Thionocarboxy (thionocarboxylic acid): -C(=S)OH.
Imidic acid: -C(=NH)OH.
Hydroxamic acid: -C(=NOH)OH.

Ester (carboxylate, carboxylic acid ester, oxycarbonyl): -C(=O)OR, wherein R is an ester substituent, for example, a C₁₋₇ alkyl group, a C₃₋₂₀ heterocyclyl group, or a C₅₋₂₀ aryl group, preferably a C₁₋₇ alkyl group. Examples of ester groups include, but are not limited to, -C(=O)OCH₃, -C(=O)OCH₂CH₃, -C(=O)OC(CH₃)₃, and -C(=O)OPh.

Acyloxy (reverse ester): -OC(=O)R, wherein R is an acyloxy substituent, for example, a C₁₋₇ alkyl group, a C₃₋₂₀ heterocyclyl group, or a C₅₋₂₀ aryl group, preferably a C₁₋₇ alkyl group. Examples of acyloxy groups include, but are not limited to, -OC(=O)CH₃ (acetoxy), -OC(=O)CH₂CH₃, -OC(=O)C(CH₃)₃, -OC(=O)Ph, and -OC(=O)CH₂Ph.

Oxycarboyloxy: -OC(=O)OR, wherein R is an ester substituent, for example, a C₁₋₇ alkyl, group, a C₃₋₂₀ heterocyclyl group, or a C₅₋₂₀ aryl group, preferably a C₁₋₇ alkyl group.

Examples of ester groups include, but are not limited to, -OC(=O)OCH₃, -OC(=O)OCH₂CH₃, -OC(=O)OC(CH₃)₃, and -OC(=O)OPh.

Amino: -NR¹R², wherein R¹ and R² are independently amino substituents, for example, hydrogen, a C₁₋₇ alkyl group (also referred to as C₁₋₇ alkylamino or di-C₁₋₇ alkylamino), a C₃₋₂₀ heterocyclyl group, or a C₅₋₂₀ aryl group, preferably H or a C₁₋₇ alkyl group, or, in the case of a "cyclic" amino group, R¹ and R², taken together with the nitrogen atom to which they are attached, form a heterocyclic ring having from 4 to 8 ring atoms. Amino groups may be primary (-NH₂), secondary (-NHR¹), or tertiary (-NHR¹R²), and in cationic form, may be quaternary (-⁺NR¹R²R³). Examples of amino groups include, but are not limited to, -NH₂, -NHCH₃, -NHC(CH₃)₂, -N(CH₃)₂, -N(CH₂CH₃)₂, and -NHPh. Examples of cyclic amino groups include, but are not limited to, aziridino, azetidino, pyrrolidino, piperidino, piperazino, morpholino, and thiomorpholino.

Amido (carbamoyl, carbamyl, aminocarbonyl, carboxamide): -C(=O)NR¹R², wherein R¹ and R² are independently amino substituents, as defined for amino groups. Examples of amido groups include, but are not limited
to, -C(=O)NH₂, -C(=O)NHCH₃, -C(=O)N(CH₃)₂, -C(=O)NHCH₂CH₃, and -C(=O)N(CH₂CH₃)₂, as well as amido groups in which R¹ and R², together with the nitrogen atom to which they are attached, form a heterocyclic structure as in, for example, piperidinocarbonyl, morpholinocarbonyl, thiomorpholinocarbonyl, and piperazinocarbonyl.

Thioamido (thiocarbamyl): -C(=S)NR¹R², wherein R¹ and R² are independently amino substituents, as defined for amino groups. Examples of amido groups include, but are not limited to, -C(=S)NH₂, -C(=S)NHCH₃, -C(=S)N(CH₃)₂, and -C(=S)NHCH₂CH₃.

Acylamido (acylamino): -NR¹C(=O)R², wherein R¹ is an amide substituent, for example, hydrogen, a C₁₋₇ alkyl group, a C₃₋₂₀ heterocyclyl group, or a C₅₋₂₀ aryl group, preferably hydrogen or a C₁₋₇ alkyl group, and R² is an acyl substituent, for example, a C₁₋₇ alkyl group, a C₃₋₂₀ heterocyclyl group, or a C₅₋₂₀aryl group, preferably hydrogen or a C₁₋₇ alkyl group. Examples of acylamide groups include, but are not limited
to, -NHC(=O)CH₃ , -NHC(=O)CH₂CH₃, and -NHC(=O)Ph. R¹ and R² may together form a cyclic structure, as in, for example, succinimidyl, maleimidyl, and phthalimidyl:

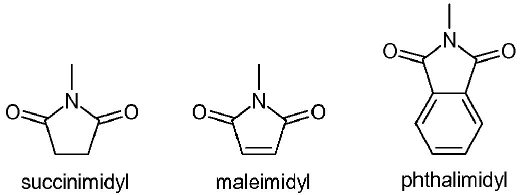

Aminocarbonyloxy: -OC(=O)NR¹R*²,* wherein R¹ and R² are independently amino substituents, as defined for amino groups. Examples of aminocarbonyloxy groups include, but are not limited to, -OC(=O)NH₂, -OC(=O)NHMe, -OC(=O)NMe₂, and -OC(=O)NEt₂.

Ureido: -N(R¹)CONR²R³ wherein R² and R³ are independently amino substituents, as defined for amino groups, and R¹ is a ureido substituent, for example, hydrogen, a C₁₋₇ alkyl, group, a C₃₋₂₀ heterocyclyl group, or a C₅₋₂₀ aryl group, preferably hydrogen or a C₁₋₇ alkyl, group. Examples of ureido groups include, but are not limited to, -NHCONH₂, - NHCONHMe, -NHCONHEt, -NHCONMe₂, -NHCONEt₂, -NMeCONH₂, - NMeCONHMe, -NMeCONHEt, -NMeCONMe₂, and -NMeCONEt₂.
Guanidino: -NH-C(=NH)NH₂.

Tetrazolyl: a five membered aromatic ring having four nitrogen atoms and one carbon atom,

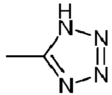

Imino: =NR, wherein R is an imino substituent, for example, for example, hydrogen, a C₁₋₇ alkyl group, a C₃₋₂₀ heterocyclyl group, or a C₅₋₂₀ aryl group, preferably H or a C₁₋₇alkyl group. Examples of imino groups include, but are not limited to, =NH, =NMe, and =NEt.

Amidine (amidino): -C(=NR)NR₂, wherein each R is an amidine substituent, for example, hydrogen, a C₁₋₇ alkyl group, a C₃₋₂₀ heterocyclyl group, or a C₅₋₂₀ aryl group, preferably H or a C₁₋₇ alkyl group. Examples of amidine groups include, but are not limited to, -C(=NH)NH₂, -C(=NH)NMe₂, and -C(=NMe)NMe₂.
Nitro: -NO₂.
Nitroso: -NO.
Azido: -N₃.
Cyano (nitrile, carbonitrile): -CN.
Isocyano: -NC.
Cyanato: -OCN.
Isocyanato: -NCO.
Thiocyano (thiocyanato): -SCN.
Isothiocyano (isothiocyanato): -NCS.
Sulfhydryl (thiol, mercapto): -SH.

Thioether (sulfide): -SR, wherein R is a thioether substituent, for example, a C₁₋₇ alkyl group (also referred to as a C₁₋₇alkylthio group), a C₃₋₂₀ heterocyclyl group, or a C₅₋₂₀ aryl group, preferably a C₁₋₇ alkyl group. Examples of C₁₋₇ alkylthio groups include, but are not limited to, -SCH₃ and -SCH₂CH₃.

Disulfide: -SS-R, wherein R is a disulfide substituent, for example, a C₁₋₇ alkyl group, a C₃₋₂₀ heterocyclyl group, or a C₅₋₂₀ aryl group, preferably a C₁₋₇ alkyl group (also referred to herein as C₁₋₇ alkyl disulfide). Examples of C₁₋₇ alkyl disulfide groups include, but are not limited to, -SSCH₃ and -SSCH₂CH₃.

Sulfine (sulfinyl, sulfoxide): -S(=O)R, wherein R is a sulfine substituent, for example, a C₁₋₇ alkyl group, a C₃₋₂₀ heterocyclyl group, or a C₅₋₂₀ aryl group, preferably a C₁₋₇ alkyl group. Examples of sulfine groups include, but are not limited to, -S(=O)CH₃ and -S(=O)CH₂CH₃.

Sulfone (sulfonyl): -S(=O)₂R, wherein R is a sulfone substituent, for example, a C₁₋₇ alkyl, group, a C₃₋₂₀ heterocyclyl group, or a C₅₋₂₀ aryl group, preferably a C₁₋₇ alkyl group, including, for example, a fluorinated or perfluorinated C₁₋₇ alkyl group. Examples of sulfone groups include, but are not limited to, -S(=O)₂CH₃ (methanesulfonyl, mesyl), -S(=O)₂CF₃ (triflyl), -S(=O)₂CH₂CH₃ (esyl), -S(=O)₂C₄F₉ (nonaflyl), -S(=O)₂CH₂CF₃ (tresyl), -S(=O)₂CH₂CH₂NH₂ (tauryl), -S(=O)₂Ph (phenylsulfonyl, besyl), 4-methylphenylsulfonyl (tosyl), 4-chlorophenylsulfonyl (closyl), 4-bromophenylsulfonyl (brosyl), 4-nitrophenyl (nosyl), 2-naphthalenesulfonate (napsyl), and 5-dimethylamino-naphthalen-1-ylsulfonate (dansyl).
Sulfinic acid (sulfino): -S(=O)OH, -SO₂H.
Sulfonic acid (sulfo): -S(=O)₂OH, -SO₃H.

Sulfinate (sulfinic acid ester): -S(=O)OR; wherein R is a sulfinate substituent, for example, a C₁₋₇ alkyl group, a C₃₋₂₀ heterocyclyl group, or a C₅₋₂₀ aryl group, preferably a C₁₋₇ alkyl group. Examples of sulfinate groups include, but are not limited to, -S(=O)OCH₃ (methoxysulfinyl; methyl sulfinate) and -S(=O)OCH₂CH₃ (ethoxysulfinyl; ethyl sulfinate).

Sulfonate (sulfonic acid ester): -S(=O)₂OR, wherein R is a sulfonate substituent, for example, a C₁₋₇ alkyl group, a C₃₋₂₀ heterocyclyl group, or a C₅₋₂₀ aryl group, preferably a C₁₋₇ alkyl, group. Examples of sulfonate groups include, but are not limited to, -S(=O)₂OCH₃ (methoxysulfonyl; methyl sulfonate) and -S(=O)₂OCH₂CH₃ (ethoxysulfonyl; ethyl sulfonate).

Sulfinyloxy: -OS(=O)R, wherein R is a sulfinyloxy substituent, for example, a C₁₋₇ alkyl group, a C₃₋₂₀ heterocyclyl group, or a C₅₋₂₀ aryl group, preferably a C₁₋₇ alkyl group. Examples of sulfinyloxy groups include, but are not limited to, -OS(=O)CH₃ and -OS(=O)CH₂CH₃.

Sulfonyloxy: -OS(=O)₂R, wherein R is a sulfonyloxy substituent, for example, a C₁₋₇ alkyl, group, a C₃₋₂₀ heterocyclyl group, or a C₅₋₂₀ aryl group, preferably a C₁₋₇ alkyl group. Examples of sulfonyloxy groups include, but are not limited to, -OS(=O)₂CH₃ (mesylate) and -OS(=O)₂CH₂CH₃ (esylate).

Sulfate: -OS(=O)₂OR; wherein R is a sulfate substituent, for example, a C₁₋₇ alkyl group, a C₃₋₂₀ heterocyclyl group, or a C₅₋₂₀ aryl group, preferably a C₁₋₇ alkyl group. Examples of sulfate groups include, but are not limited to, -OS(=O)₂OCH₃ and -SO(=O)₂OCH₂CH₃.

Sulfamyl (sulfamoyl; sulfinic acid amide; sulfinamide): -S(=O)NR¹R², wherein R¹ and R² are independently amino substituents, as defined for amino groups. Examples of sulfamyl groups include, but are not limited
to, -S(=O)NH₂, -S(=O)NH(CH₃), -S(=O)N(CH₃)₂, -S(=O)NH(CH₂CH₃), -S(=O)N(CH₂CH₃)2, and -S(=O)NHPh.

Sulfonamido (sulfinamoyl; sulfonic acid amide; sulfonamide): -S(=O)₂NR¹R², wherein R¹ and R² are independently amino substituents, as defined for amino groups. Examples of sulfonamido groups include, but are not limited
to, -S(=O)₂NH₂, -S(=O)₂NH(CH₃), -S(=O)₂N(CH₃)₂, -S(=O)₂NH(CH₂CH₃), -S(=O)₂N(CH₂CH₃)₂, and -S(=O)₂NHPh.

Sulfamino: -NR¹S(=O)₂OH, wherein R¹ is an amino substituent, as defined for amino groups. Examples of sulfamino groups include, but are not limited to, -NHS(=O)₂OH
and -N(CH₃)S(=O)₂OH.

Sulfonamino: -NR¹S(=O)₂R, wherein R¹ is an amino substituent, as defined for amino groups, and R is a sulfonamino substituent, for example, a C₁₋₇ alkyl group, a C₃₋₂₀ heterocyclyl group, or a C₅₋₂₀ aryl group, preferably a C₁₋₇ alkyl group. Examples of sulfonamino groups include, but are not limited to, -NHS(=O)₂CH₃ and -N(CH₃)S(=O)₂C₆H₅.

Sulfinamino: -NR¹S(=O)R, wherein R¹ is an amino substituent, as defined for amino groups, and R is a sulfinamino substituent, for example, a C₁₋₇ alkyl group, a C₃₋₂₀ heterocyclyl group, or a C₅₋₂₀ aryl group, preferably a C₁₋₇ alkyl group. Examples of sulfinamino groups include, but are not limited to, -NHS(=O)CH₃ and -N(CH₃)S(=O)C₆H₅.

Phosphino (phosphine): -PR₂, wherein R is a phosphino substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphino groups include, but are not limited to, -PH₂, -P(CH₃)₂, -P(CH₂CH₃)₂, -P(t-Bu)₂, and -P(Ph)₂.
Phospho: -P(=O)₂.

Phosphinyl (phosphine oxide): -P(=O)R₂, wherein R is a phosphinyl substituent, for example, a C₁₋₇ alkyl group, a C₃₋₂₀ heterocyclyl group, or a C₅₋₂₀ aryl group, preferably a C₁₋₇ alkyl, group or a C₅₋₂₀ aryl group. Examples of phosphinyl groups include, but are not limited to, -P(=O)(CH₃)₂, -P(=O)(CH₂CH₃)₂, -P(=O)(t-Bu)₂, and -P(=O)(Ph)₂.
Phosphonic acid (phosphono): -P(=O)(OH)₂.

Phosphonate (phosphono ester): -P(=O)(OR)₂, where R is a phosphonate substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphonate groups include, but are not limited to, -P(=O)(OCH₃)₂, -P(=O)(OCH₂CH₃)₂, -P(=O)(O-t-Bu)₂, and -P(=O)(OPh)₂.
Phosphoric acid (phosphonooxy): -OP(=O)(OH)₂.

Phosphate (phosphonooxy ester): -OP(=O)(OR)₂, where R is a phosphate substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphate groups include, but are not limited to, -OP(=O)(OCH₃)₂, -OP(=O)(OCH₂CH₃)₂, -OP(=O)(O-t-Bu)₂, and -OP(=O)(OPh)₂.
Phosphorous acid: -OP(OH)₂.

Phosphite: -OP(OR)₂, where R is a phosphite substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphite groups include, but are not limited
to, -OP(OCH₃)₂, -OP(OCH₂CH₃)₂, -OP(O-t-Bu)₂, and -OP(OPh)₂.

Phosphoramidite: -OP(OR¹)-NR²₂, where R¹ and R² are phosphoramidite substituents, for example, -H, a (optionally substituted) C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphoramidite groups include, but are not limited to, -OP(OCH₂CH₃)-N(CH₃)₂, -OP(OCH₂CH₃)-N(i-Pr)₂, and -OP(OCH₂CH₂CN)-N(i-Pr)₂.

Phosphoramidate: -OP(=O)(OR¹)-NR²₂, where R¹ and R² are phosphoramidate substituents, for example, -H, a (optionally substituted) C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphoramidate groups include, but are not limited to, -OP(=O)(OCH₂CH₃)-N(CH₃)₂, -OP(=O)(OCH₂CH₃)-N(i-Pr)₂, and -OP(=O)(OCH₂CH₂CN)-N(i-Pr)₂.

### Alkylene

C₃₋₁₂ alkylene: The term "C₃₋₁₂ alkylene", as used herein, pertains to a bidentate moiety obtained by removing two hydrogen atoms, either both from the same carbon atom, or one from each of two different carbon atoms, of a hydrocarbon compound having from 3 to 12 carbon atoms (unless otherwise specified), which may be aliphatic or alicyclic, and which may be saturated, partially unsaturated, or fully unsaturated. Thus, the term "alkylene" includes the sub-classes alkenylene, alkynylene, cycloalkylene, etc., discussed below.

Examples of linear saturated C₃₋₁₂ alkylene groups include, but are not limited to, -(CH₂)ₙ-where n is an integer from 3 to 12, for example, -CH₂CH₂CH₂-(propylene), -CH₂CH₂CH₂CH₂- (butylene), -CH₂CH₂CH₂CH₂CH₂- (pentylene)
and -CH₂CH₂CH₂CH₂CH₂CH₂CH₂- (heptylene).

Examples of branched saturated C₃₋₁₂ alkylene groups include, but are not limited
to, -CH(CH₃)CH₂-, -CH(CH₃)CH₂CH₂-, -CH(CH₃)CH₂CH₂CH₂-, -CH₂CH(CH₃)CH₂-, -CH₂CH(C H₃)CH₂CH₂-, -CH(CH₂CH₃)-, -CH(CH₂CH₃)CH₂-, and -CH₂CH(CH₂CH₃)CH₂-.

Examples of linear partially unsaturated C₃₋₁₂ alkylene groups (C₃₋₁₂ alkenylene, and alkynylene groups) include, but are not limited to, -CH=CH-CH₂-, -CH₂-CH=CH₂-, -CH=CH-CH₂-CH₂-, -CH=CH-CH₂-CH₂-CH₂-, -CH=CH-CH=CH-, -CH=CH-CH=CH-CH₂-, -CH=CH-CH=CH-CH₂-CH₂-, -CH=CH-CH₂-CH=CH-, -CH=CH-CH₂-CH₂-CH=CH-, and -CH₂-C≡C-CH₂-.

Examples of branched partially unsaturated C₃₋₁₂ alkylene groups (C₃₋₁₂ alkenylene and alkynylene groups) include, but are not limited to, -C(CH₃)=CH-, -C(CH₃)=CH-CH₂-, -CH=CH-CH(CH₃)- and -C≡C-CH(CH₃)-.

Examples of alicyclic saturated C₃₋₁₂ alkylene groups (C₃₋₁₂ cycloalkylenes) include, but are not limited to, cyclopentylene (e.g. cyclopent-1,3-ylene), and cyclohexylene (e.g. cyclohex-1,4-ylene).

Examples of alicyclic partially unsaturated C₃₋₁₂ alkylene groups (C₃₋₁₂ cycloalkylenes) include, but are not limited to, cyclopentenylene (e.g. 4-cyclopenten-1,3-ylene), cyclohexenylene (e.g. 2-cyclohexen-1,4-ylene; 3-cyclohexen-1,2-ylene; 2,5-cyclohexadien-1,4-ylene).

### Includes Other Forms

Unless otherwise specified, included in the above are the well-known ionic, salt, solvate, and protected forms of these substituents. For example, a reference to carboxylic acid (-COOH) also includes the anionic (carboxylate) form (-COO⁻), a salt or solvate thereof, as well as conventional protected forms. Similarly, a reference to an amino group includes the protonated form (-N⁺HR¹R²), a salt or solvate of the amino group, for example, a hydrochloride salt, as well as conventional protected forms of an amino group. Similarly, a reference to a hydroxyl group also includes the anionic form (-O⁻), a salt or solvate thereof, as well as conventional protected forms.

### Salts

It may be convenient or desirable to prepare, purify, and/or handle a corresponding salt of the drug linker compound, for example, a pharmaceutically-acceptable salt. Examples of pharmaceutically acceptable salts are discussed in Berge, et al., J. Pharm. Sci., 66, 1-19 (1977).

For example, if the compound is anionic, or has a functional group which may be anionic (e.g. -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al⁺³. Examples of suitable organic cations include, but are not limited to, ammonium ion (i.e. NH₄⁺) and substituted ammonium ions (e.g. NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group which may be cationic (e.g. -NH₂ may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, trifluoroacetic acid and valeric. Examples of suitable polymeric organic anions include, but are not limited to, those derived from the following polymeric acids: tannic acid, carboxymethyl cellulose.

### Solvates

It may be convenient or desirable to prepare, purify, and/or handle a corresponding solvate of the drug linker compound. The term "solvate" is used herein in the conventional sense to refer to a complex of solute (e.g. active compound, salt of active compound) and solvent. If the solvent is water, the solvate may be conveniently referred to as a hydrate, for example, a mono-hydrate, a di-hydrate, a tri-hydrate, etc.

The invention includes compounds where a solvent adds across the imine bond of the PBD moiety, which is illustrated below where the solvent is water or an alcohol (R^{A}OH, where R^{A} is C₁₋₄ alkyl):

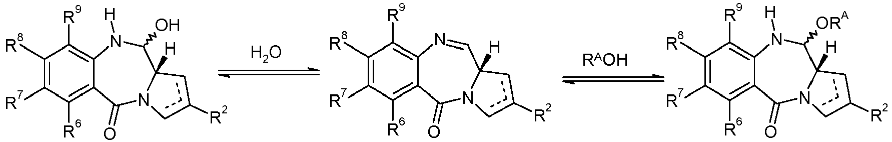

These forms can be called the carbinolamine and carbinolamine ether forms of the PBD (as described in the section relating to R¹⁰ above). The balance of these equilibria depend on the conditions in which the compounds are found, as well as the nature of the moiety itself.

These particular compounds may be isolated in solid form, for example, by lyophilisation.

### Isomers

Certain compounds of the invention may exist in one or more particular geometric, optical, enantiomeric, diasteriomeric, epimeric, atropic, stereoisomeric, tautomeric, conformational, or anomeric forms, including but not limited to, cis- and trans-forms; E- and Z-forms; c-, t-, and r- forms; endo- and exo-forms; R-, S-, and meso-forms; D- and L-forms; d- and I-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms").

The term "chiral" refers to molecules which have the property of non-superimposability of the mirror image partner, while the term "achiral" refers to molecules which are superimposable on their mirror image partner.

The term "stereoisomers" refers to compounds which have identical chemical constitution, but differ with regard to the arrangement of the atoms or groups in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds of the invention may contain asymmetric or chiral centers, and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes d and I or (+) and (-) are employed to designate the sign of rotation of plane-polarized light by the compound, with (-) or I meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

Note that, except as discussed below for tautomeric forms, specifically excluded from the term "isomers", as used herein, are structural (or constitutional) isomers (i.e. isomers which differ in the connections between atoms rather than merely by the position of atoms in space). For example, a reference to a methoxy group, -OCH₃, is not to be construed as a reference to its structural isomer, a hydroxymethyl group, -CH₂OH. Similarly, a reference to ortho-chlorophenyl is not to be construed as a reference to its structural isomer, meta-chlorophenyl. However, a reference to a class of structures may well include structurally isomeric forms falling within that class (e.g. C₁₋₇ alkyl includes n-propyl and iso-propyl; butyl includes n-, iso-, sec-, and tert-butyl; methoxyphenyl includes ortho-, meta-, and para-methoxyphenyl).

The above exclusion does not pertain to tautomeric forms, for example, keto-, enol-, and enolate-forms, as in, for example, the following tautomeric pairs: keto/enol (illustrated below), imine/enamine, amide/imino alcohol, amidine/amidine, nitroso/oxime, thioketone/enethiol, N-nitroso/hyroxyazo, and nitro/aci-nitro.

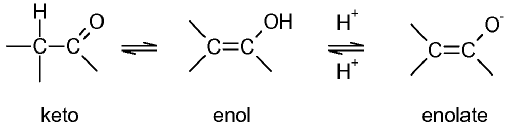

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

Note that specifically included in the term "isomer" are compounds with one or more isotopic substitutions. For example, H may be in any isotopic form, including ¹H, ²H (D), and ³H (T); C may be in any isotopic form, including ¹²C, ¹³C, and ¹⁴C; O may be in any isotopic form, including ¹⁶O and ¹⁸O; and the like.

Examples of isotopes that can be incorporated into compounds of the invention include isotopes of hydrogen, carbon, nitrogen, oxygen, phosphorous, fluorine, and chlorine, such as, but not limited to ²H (deuterium, D), ³H (tritium), ¹¹C, ¹³C ¹⁴C ¹⁵N ¹⁸F, ³¹P, ³²P, ³⁵S, ³⁶Cl, and ¹²⁵I. Various isotopically labeled compounds of the present invention, for example those into which radioactive isotopes such as 3H, 13C, and 14C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays, or in radioactive treatment of patients. Deuterium labelled or substituted therapeutic compounds of the invention may have improved DMPK (drug metabolism and pharmacokinetics) properties, relating to distribution, metabolism, and excretion (ADME). Substitution with heavier isotopes such as deuterium may afford certain therapeutic advantages resulting from greater metabolic stability, for example increased in vivo half-life or reduced dosage requirements. An 18F labeled compound may be useful for PET or SPECT studies. Isotopically labeled compounds of this invention and prodrugs thereof can generally be prepared by carrying out the procedures disclosed in the schemes or in the examples and preparations described below by substituting a readily available isotopically labeled reagent for a non-isotopically labeled reagent. Further, substitution with heavier isotopes, particularly deuterium (i.e., 2H or D) may afford certain therapeutic advantages resulting from greater metabolic stability, for example increased in vivo half-life or reduced dosage requirements or an improvement in therapeutic index. It is understood that deuterium in this context is regarded as a substituent. The concentration of such a heavier isotope, specifically deuterium, may be defined by an isotopic enrichment factor. In the compounds of this invention any atom not specifically designated as a particular isotope is meant to represent any stable isotope of that atom.

Unless otherwise specified, a reference to a particular compound includes all such isomeric forms, including (wholly or partially) racemic and other mixtures thereof. Methods for the preparation (e.g. asymmetric synthesis) and separation (e.g. fractional crystallisation and chromatographic means) of such isomeric forms are either known in the art or are readily obtained by adapting the methods taught herein, or known methods, in a known manner.

Exemplary drug linker compounds of formula I include:

| | |
|---|---|
| 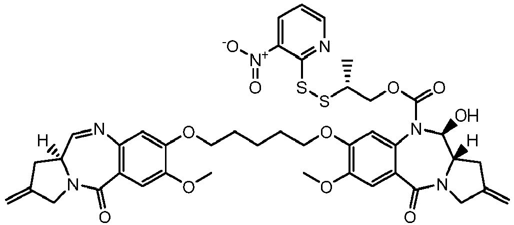 | (11S,11aS)-((R)-2-((3-nitropyridin-2-yl)disulfanyl)propyl) 11-hydroxy-7-methoxy-8-(5-((S)-7-methoxy-2-methylene-5-oxo-2,3,5,11a-tetrahydro-1 H-benzo[e]pyrrolo[1,2-a][1,4]diazepin-8-yloxy)pentyloxy)-2-methylene-5-oxo-2,3,11,11a-tetrahydro-1H-benzo[e]pyrrolo[1,2-a][1,4]diazepine-10(5H)-carboxylate |
| 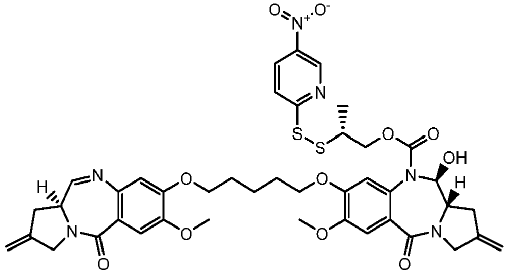 | (11S,11aS)-((R)-2-((5-nitropyridin-2-yl)disulfanyl)propyl) 11-hydroxy-7-methoxy-8-(5-((S)-7-methoxy-2-methylene-5-oxo-2,3,5,11a-tetrahydro-1H-benzo[e]pyrrolo[1,2-a][1,4]diazepin-8-yloxy)pentyloxy)-2-methylene-5-oxo-2,3,11,11a-tetrahydro-1H-benzo[e]pyrrolo[1,2-a][1,4]diazepine-10(5H)-carboxylate |
| 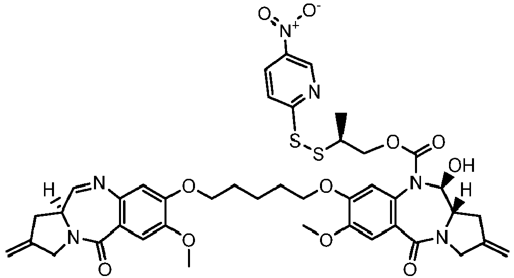 | (11S,11aS)-((S)-2-((5-nitropyridin-2-yl)disulfanyl)propyl) 11-hydroxy-7-methoxy-8-(5-((S)-7-methoxy-2-methylene-5-oxo-2,3,5,11a-tetrahydro-1H-benzo[e]pyrrolo[1,2-a][1,4]diazepin-8-yloxy)pentyloxy)-2-methylene-5-oxo-2,3,11,11a-tetrahydro-1H-benzo[e]pyrrolo[1,2-a][1,4]diazepine-10(5H)-carboxylate |
| 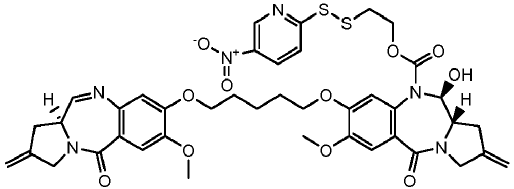 | (11S,11aS)-2-((5-nitropyridin-2-yl)disulfanyl)ethyl 11-hydroxy-7-methoxy-8-(5-((S)-7-methoxy-2-methylene-5-oxo-2,3,5,11a-tetrahydro-1H-benzo[e]pyrrolo[1,2-a][1,4]diazepin-8-yloxy)pentyloxy)-2-methylene-5-oxo-2,3,11,11a-tetrahydro-1H-benzo[e]pyrrolo[1,2-a][1,4]diazepine-10(5H)-carboxylate |

### Biological Activity

### In vitro cell proliferation assays

Generally, the cytotoxic or cytostatic activity of an antibody-drug conjugate (ADC) is measured by: exposing mammalian cells having receptor proteins, e.g. HER2, to the antibody of the ADC in a cell culture medium; culturing the cells for a period from about 6 hours to about 5 days; and measuring cell viability. Cell-based *in vitro* assays are used to measure viability (proliferation), cytotoxicity, and induction of apoptosis (caspase activation) of an ADC of the invention.

The *in vitro* potency of antibody-drug conjugates can be measured by a cell proliferation assay. The CeIITiter-Glo^{®} Luminescent Cell Viability Assay is a commercially available (Promega Corp., Madison, WI), homogeneous assay method based on the recombinant expression of *Coleoptera* luciferase (US Patent Nos. 5583024; 5674713 and 5700670). This cell proliferation assay determines the number of viable cells in culture based on quantitation of the ATP present, an indicator of metabolically active cells (Crouch et al (1993) J. Immunol. Meth. 160:81-88; US 6602677). The CellTiter-Glo^{®} Assay is conducted in 96 well format, making it amenable to automated high-throughput screening (HTS) (Cree et al (1995) AntiCancer Drugs 6:398-404). The homogeneous assay procedure involves adding the single reagent (CellTiter-Glo^{®} Reagent) directly to cells cultured in serum-supplemented medium. Cell washing, removal of medium and multiple pipetting steps are not required. The system detects as few as 15 cells/well in a 384-well format in 10 minutes after adding reagent and mixing. The cells may be treated continuously with ADC, or they may be treated and separated from ADC. Generally, cells treated briefly, i.e. 3 hours, showed the same potency effects as continuously treated cells.

The homogeneous "add-mix-measure" format results in cell lysis and generation of a luminescent signal proportional to the amount of ATP present. The amount of ATP is directly proportional to the number of cells present in culture. The CellTiter-Glo^{®} Assay generates a "glow-type" luminescent signal, produced by the luciferase reaction, which has a half-life generally greater than five hours, depending on cell type and medium used. Viable cells are reflected in relative luminescence units (RLU). The substrate, Beetle Luciferin, is oxidatively decarboxylated by recombinant firefly luciferase with concomitant conversion of ATP to AMP and generation of photons.

### In vivo efficacy

The *in vivo* efficacy of antibody-drug conjugates (ADC) of the invention can be measured by tumor xenograft studies in mice. For example, the in vivo efficacy of an anti-HER2 ADC of the invention can be measured by a high expressing HER2 transgenic explant mouse model. An allograft is propagated from the Fo5 mmtv transgenic mouse which does not respond to, or responds poorly to, HERCEPTIN® therapy. Subjects can be treated once with ADC at certain dose levels (mg/kg) and PBD drug exposure (µg/m²); and placebo buffer control (Vehicle) and monitored over two weeks or more to measure the time to tumor doubling, log cell kill, and tumor shrinkage.

### Use

The conjugates described herein may be used to provide a PBD compound at a target location.

The target location is preferably a proliferative cell population. The antibody is an antibody for an antigen present in a proliferative cell population.

In one embodiment the antigen is absent or present at a reduced level in a non-proliferative cell population compared to the amount of antigen present in the proliferative cell population, for example a tumour cell population.

The linker may be cleaved by an enzyme present at the target location.

The target location may be *in vitro, in vivo* or *ex vivo.*

The antibody-drug conjugate (ADC) compounds described herein include those with utility for anticancer activity. In particular, the compounds include an antibody conjugated, i.e. covalently attached by a linker, to a PBD drug moiety, i.e. toxin. When the drug is not conjugated to an antibody, the PBD drug has a cytotoxic effect. The biological activity of the PBD drug moiety is thus modulated by conjugation to an antibody. The antibody-drug conjugates (ADC) of the invention selectively deliver an effective dose of a cytotoxic agent to tumor tissue whereby greater selectivity, i.e. a lower efficacious dose, may be achieved.

Thus, described herein is a conjugate compound for use in therapy.

Also described herein is a conjugate compound for use in the treatment of a proliferative disease. Also described herein is the use of a conjugate compound in the manufacture of a medicament for treating a proliferative disease.

One of ordinary skill in the art is readily able to determine whether or not a candidate conjugate treats a proliferative condition for any particular cell type. For example, assays which may conveniently be used to assess the activity offered by a particular compound are described in the examples below.

The term "proliferative disease" pertains to an unwanted or uncontrolled cellular proliferation of excessive or abnormal cells which is undesired, such as, neoplastic or hyperplastic growth, whether *in vitro* or *in vivo.*

Examples of proliferative conditions include, but are not limited to, benign, pre-malignant, and malignant cellular proliferation, including but not limited to, neoplasms and tumours (e.g. histocytoma, glioma, astrocyoma, osteoma), cancers (e.g. lung cancer, small cell lung cancer, gastrointestinal cancer, bowel cancer, colon cancer, breast carcinoma, ovarian carcinoma, prostate cancer, testicular cancer, liver cancer, kidney cancer, bladder cancer, pancreas cancer, brain cancer, sarcoma, osteosarcoma, Kaposi's sarcoma, melanoma), leukemias, psoriasis, bone diseases, fibroproliferative disorders (e.g. of connective tissues), and atherosclerosis. Cancers of particular interest include, but are not limited to, leukemias and ovarian cancers.

Any type of cell may be treated, including but not limited to, lung, gastrointestinal (including, e.g. bowel, colon), breast (mammary), ovarian, prostate, liver (hepatic), kidney (renal), bladder, pancreas, brain, and skin.

In one embodiment, the treatment is of a pancreatic cancer.

In one embodiment, the treatment is of a tumour having αᵥβ₆ integrin on the surface of the cell.

It is contemplated that the antibody-drug conjugates (ADC) described herein may be used to treat various diseases or disorders, e.g. characterized by the overexpression of a tumor antigen. Exemplary conditions or hyperproliferative disorders include benign or malignant tumors; leukemia, haematological, and lymphoid malignancies. Others include neuronal, glial, astrocytal, hypothalamic, glandular, macrophagal, epithelial, stromal, blastocoelic, inflammatory, angiogenic and immunologic, including autoimmune, disorders.

Generally, the disease or disorder to be treated is a hyperproliferative disease such as cancer. Examples of cancer to be treated herein include, but are not limited to, carcinoma, lymphoma, blastoma, sarcoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

Autoimmune diseases for which the ADC compounds may be used in treatment include rheumatologic disorders (such as, for example, rheumatoid arthritis, Sjögren's syndrome, scleroderma, lupus such as SLE and lupus nephritis, polymyositis/dermatomyositis, cryoglobulinemia, anti-phospholipid antibody syndrome, and psoriatic arthritis), osteoarthritis, autoimmune gastrointestinal and liver disorders (such as, for example, inflammatory bowel diseases (e.g. ulcerative colitis and Crohn's disease), autoimmune gastritis and pernicious anemia, autoimmune hepatitis, primary biliary cirrhosis, primary sclerosing cholangitis, and celiac disease), vasculitis (such as, for example, ANCA-associated vasculitis, including Churg-Strauss vasculitis, Wegener's granulomatosis, and polyarteriitis), autoimmune neurological disorders (such as, for example, multiple sclerosis, opsoclonus myoclonus syndrome, myasthenia gravis, neuromyelitis optica, Parkinson's disease, Alzheimer's disease, and autoimmune polyneuropathies), renal disorders (such as, for example, glomerulonephritis, Goodpasture's syndrome, and Berger's disease), autoimmune dermatologic disorders (such as, for example, psoriasis, urticaria, hives, pemphigus vulgaris, bullous pemphigoid, and cutaneous lupus erythematosus), hematologic disorders (such as, for example, thrombocytopenic purpura, thrombotic thrombocytopenic purpura, post-transfusion purpura, and autoimmune hemolytic anemia), atherosclerosis, uveitis, autoimmune hearing diseases (such as, for example, inner ear disease and hearing loss), Behcet's disease, Raynaud's syndrome, organ transplant, and autoimmune endocrine disorders (such as, for example, diabetic-related autoimmune diseases such as insulin-dependent diabetes mellitus (IDDM), Addison's disease, and autoimmune thyroid disease (e.g. Graves' disease and thyroiditis)). More preferred such diseases include, for example, rheumatoid arthritis, ulcerative colitis, ANCA-associated vasculitis, lupus, multiple sclerosis, Sjögren's syndrome, Graves' disease, IDDM, pernicious anemia, thyroiditis, and glomerulonephritis.

### Methods of Treatment

The conjugates described herein may be used in a method of therapy. Also provided is a method of treatment, comprising administering to a subject in need of treatment a therapeutically-effective amount of a conjugate compound described herein. The term "therapeutically effective amount" is an amount sufficient to show benefit to a patient. Such benefit may be at least amelioration of at least one symptom. The actual amount administered, and rate and time-course of administration, will depend on the nature and severity of what is being treated. Prescription of treatment, e.g. decisions on dosage, is within the responsibility of general practitioners and other medical doctors.

A compound may be administered alone or in combination with other treatments, either simultaneously or sequentially dependent upon the condition to be treated. Examples of treatments and therapies include, but are not limited to, chemotherapy (the administration of active agents, including, e.g. drugs, such as chemotherapeutics); surgery; and radiation therapy.

A "chemotherapeutic agent" is a chemical compound useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, antibodies, photosensitizers, and kinase inhibitors. Chemotherapeutic agents include compounds used in "targeted therapy" and conventional chemotherapy.

Examples of chemotherapeutic agents include: erlotinib (TARCEVA®, Genentech/OSI Pharm.), docetaxel (TAXOTERE®, Sanofi-Aventis), 5-FU (fluorouracil, 5-fluorouracil, CAS No. 51-21-8), gemcitabine (GEMZAR®, Lilly), PD-0325901 (CAS No. 391210-10-9, Pfizer), cisplatin (cis-diamine, dichloroplatinum(II), CAS No. 15663-27-1), carboplatin (CAS No. 41575-94-4), paclitaxel (TAXOL®, Bristol-Myers Squibb Oncology, Princeton, N.J.), trastuzumab (HERCEPTIN®, Genentech), temozolomide (4-methyl-5-oxo- 2,3,4,6,8-pentazabicyclo [4.3.0] nona-2,7,9-triene- 9-carboxamide, CAS No. 85622-93-1, TEMODAR®, TEMODAL®, Schering Plough), tamoxifen ((*Z*)-2-[4-(1,2-diphenylbut-1-enyl)phenoxy]-*N,N-*dimethylethanamine, NOLVADEX®, ISTUBAL®, VALODEX®), and doxorubicin (ADRIAMYCIN®), Akti-1/2, HPPD, and rapamycin.
More examples of chemotherapeutic agents include: oxaliplatin (ELOXATIN®, Sanofi), bortezomib (VELCADE®, Millennium Pharm.), sutent (SUNITINIB®, SU11248, Pfizer), letrozole (FEMARA®, Novartis), imatinib mesylate (GLEEVEC®, Novartis), XL-518 (Mek inhibitor, Exelixis, WO 2007/044515), ARRY-886 (Mek inhibitor, AZD6244, Array BioPharma, Astra Zeneca), SF-1126 (PI3K inhibitor, Semafore Pharmaceuticals), BEZ-235 (PI3K inhibitor, Novartis), XL-147 (PI3K inhibitor, Exelixis), PTK787/ZK 222584 (Novartis), fulvestrant (FASLODEX®, AstraZeneca), leucovorin (folinic acid), rapamycin (sirolimus, RAPAMUNE®, Wyeth), lapatinib (TYKERB®, GSK572016, Glaxo Smith Kline), lonafarnib (SARASAR™, SCH 66336, Schering Plough), sorafenib (NEXAVAR®, BAY43-9006, Bayer Labs), gefitinib (IRESSA®, AstraZeneca), irinotecan (CAMPTOSAR®, CPT-11, Pfizer), tipifarnib (ZARNESTRA™, Johnson & Johnson), ABRAXANE™ (Cremophor-free), albumin-engineered nanoparticle formulations of paclitaxel (American Pharmaceutical Partners, Schaumberg, II), vandetanib (rINN, ZD6474, ZACTIMA®, AstraZeneca), chloranmbucil, AG1478, AG1571 (SU 5271; Sugen), temsirolimus (TORISEL®, Wyeth), pazopanib (GlaxoSmithKline), canfosfamide (TELCYTA®, Telik), thiotepa and cyclosphosphamide (CYTOXAN®, NEOSAR®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including the synthetic analog topotecan); bryostatin; callystatin; CC-1065 (including its adozelesin, carzelesin and bizelesin synthetic analogs); cryptophycins (particularly cryptophycin 1 and cryptophycin 8); dolastatin; duocarmycin (including the synthetic analogs, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g. calicheamicin, calicheamicin gamma1I, calicheamicin omegaI1 (Angew Chem. Intl. Ed. Engl. (1994) 33:183-186); dynemicin, dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, morpholino-doxorubicin, cyanomorpholino-doxorubicin, 2-pyrrolino-doxorubicin and deoxydoxorubicin), epirubicin, esorubicin, idarubicin, nemorubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; 6-thioguanine; mercaptopurine; methotrexate; platinum analogs such as cisplatin and carboplatin; vinblastine; etoposide (VP-16); ifosfamide; mitoxantrone; vincristine; vinorelbine (NAVELBINE®); novantrone; teniposide; edatrexate; daunomycin; aminopterin; capecitabine (XELODA®, Roche); ibandronate; CPT-11; topoisomerase inhibitor RFS 2000; difluoromethylornithine (DMFO); retinoids such as retinoic acid; and pharmaceutically acceptable salts, acids and derivatives of any of the above.

Also included in the definition of "chemotherapeutic agent" are: (i) anti-hormonal agents that act to regulate or inhibit hormone action on tumors such as anti-estrogens and selective estrogen receptor modulators (SERMs), including, for example, tamoxifen (including NOLVADEX®; tamoxifen citrate), raloxifene, droloxifene, 4-hydroxytamoxifen, trioxifene, keoxifene, LY117018, onapristone, and FARESTON® (toremifine citrate); (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, RIVISOR® (vorozole), FEMARA® (letrozole; Novartis), and ARIMIDEX® (anastrozole; AstraZeneca); (iii) anti-androgens such as flutamide, nilutamide, bicalutamide, leuprolide, and goserelin; as well as troxacitabine (a 1,3-dioxolane nucleoside cytosine analog); (iv) protein kinase inhibitors such as MEK inhibitors (WO 2007/044515); (v) lipid kinase inhibitors; (vi) antisense oligonucleotides, particularly those which inhibit expression of genes in signaling pathways implicated in aberrant cell proliferation, for example, PKC-alpha, Raf and H-Ras, such as oblimersen (GENASENSE®, Genta Inc.); (vii) ribozymes such as VEGF expression inhibitors (e.g., ANGIOZYME®) and HER2 expression inhibitors; (viii) vaccines such as gene therapy vaccines, for example, ALLOVECTIN®, LEUVECTIN®, and VAXID®; PROLEUKIN® rIL-2; topoisomerase 1 inhibitors such as LURTOTECAN®; ABARELIX® rmRH; (ix) anti-angiogenic agents such as bevacizumab (AVASTIN®, Genentech); and pharmaceutically acceptable salts, acids and derivatives of any of the above.
Also included in the definition of "chemotherapeutic agent" are therapeutic antibodies such as alemtuzumab (Campath), bevacizumab (AVASTIN®, Genentech); cetuximab (ERBITUX®, Imclone); panitumumab (VECTIBIX®, Amgen), rituximab (RITUXAN®, Genentech/Biogen Idec), pertuzumab (OMNITARG™, 2C4, Genentech), trastuzumab (HERCEPTIN®, Genentech), tositumomab (Bexxar, Corixia), and the antibody drug conjugate, gemtuzumab ozogamicin (MYLOTARG®, Wyeth).

Humanized monoclonal antibodies with therapeutic potential as chemotherapeutic agents in combination with the conjugates of the invention include: alemtuzumab, apolizumab, aselizumab, atlizumab, bapineuzumab, bevacizumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pertuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, trastuzumab, tucotuzumab celmoleukin, tucusituzumab, umavizumab, urtoxazumab, and visilizumab.

Pharmaceutical compositions described herein, and for use as described herein, may comprise, in addition to the active ingredient, i.e. a conjugate compound, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. cutaneous, subcutaneous, or intravenous.

Pharmaceutical compositions for oral administration may be in tablet, capsule, powder or liquid form. A tablet may comprise a solid carrier or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. A capsule may comprise a solid carrier such a gelatin.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

### Formulations

While it is possible for the conjugate compound to be used (e.g., administered) alone, it is often preferable to present it as a composition or formulation.

In one embodiment, the composition is a pharmaceutical composition (e.g., formulation, preparation, medicament) comprising a conjugate compound, as described herein, and a pharmaceutically acceptable carrier, diluent, or excipient.

In one embodiment, the composition is a pharmaceutical composition comprising at least one conjugate compound, as described herein, together with one or more other pharmaceutically acceptable ingredients well known to those skilled in the art, including, but not limited to, pharmaceutically acceptable carriers, diluents, excipients, adjuvants, fillers, buffers, preservatives, anti-oxidants, lubricants, stabilisers, solubilisers, surfactants (e.g., wetting agents), masking agents, colouring agents, flavouring agents, and sweetening agents.

In one embodiment, the composition further comprises other active agents, for example, other therapeutic or prophylactic agents.

Suitable carriers, diluents, excipients, etc. can be found in standard pharmaceutical texts. See, for example, Handbook of Pharmaceutical Additives, 2nd Edition (eds. M. Ash and I. Ash), 2001 (Synapse Information Resources, Inc., Endicott, New York, USA), Remington's Pharmaceutical Sciences, 20th edition, pub. Lippincott, Williams & Wilkins, 2000; and Handbook of Pharmaceutical Excipients, 2nd edition, 1994.

Also described herein are methods of making a pharmaceutical composition comprising admixing at least one [¹¹C]-radiolabelled conjugate or conjugate-like compound, as defined herein, together with one or more other pharmaceutically acceptable ingredients well known to those skilled in the art, e.g., carriers, diluents, excipients, etc. If formulated as discrete units (e.g., tablets, etc.), each unit contains a predetermined amount (dosage) of the active compound.

The term "pharmaceutically acceptable," as used herein, pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, diluent, excipient, etc. must also be "acceptable" in the sense of being compatible with the other ingredients of the formulation.

The formulations may be prepared by any methods well known in the art of pharmacy. Such methods include the step of bringing into association the active compound with a carrier which constitutes one or more accessory ingredients. In general, the formulations are prepared by uniformly and intimately bringing into association the active compound with carriers (e.g., liquid carriers, finely divided solid carrier, etc.), and then shaping the product, if necessary.

The formulation may be prepared to provide for rapid or slow release; immediate, delayed, timed, or sustained release; or a combination thereof.

Formulations suitable for parenteral administration (e.g., by injection), include aqueous or non-aqueous, isotonic, pyrogen-free, sterile liquids (e.g., solutions, suspensions), in which the active ingredient is dissolved, suspended, or otherwise provided (e.g., in a liposome or other microparticulate). Such liquids may additional contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection. Typically, the concentration of the active ingredient in the liquid is from about 1 ng/ml to about 10 µg/ml, for example from about 10 ng/ml to about 1 µg/ml. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets.

### Dosage

It will be appreciated by one of skill in the art that appropriate dosages of the conjugate compound, and compositions comprising the conjugate compound, can vary from patient to patient. Determining the optimal dosage will generally involve the balancing of the level of therapeutic benefit against any risk or deleterious side effects. The selected dosage level will depend on a variety of factors including, but not limited to, the activity of the particular compound, the route of administration, the time of administration, the rate of excretion of the compound, the duration of the treatment, other drugs, compounds, and/or materials used in combination, the severity of the condition, and the species, sex, age, weight, condition, general health, and prior medical history of the patient. The amount of compound and route of administration will ultimately be at the discretion of the physician, veterinarian, or clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

In general, a suitable dose of the active compound is in the range of about 100 ng to about 25 mg (more typically about 1 µg to about 10 mg) per kilogram body weight of the subject per day. Where the active compound is a salt, an ester, an amide, a prodrug, or the like, the amount administered is calculated on the basis of the parent compound and so the actual weight to be used is increased proportionately.

In one embodiment, the active compound is administered to a human patient according to the following dosage regime: about 100 mg, 3 times daily.

In one embodiment, the active compound is administered to a human patient according to the following dosage regime: about 150 mg, 2 times daily.

In one embodiment, the active compound is administered to a human patient according to the following dosage regime: about 200 mg, 2 times daily.

However in one embodiment, the conjugate compound is administered to a human patient according to the following dosage regime: about 50 or about 75 mg, 3 or 4 times daily.

In one embodiment, the conjugate compound is administered to a human patient according to the following dosage regime: about 100 or about 125 mg, 2 times daily.

The dosage amounts described above may apply to the conjugate (including the PBD moiety and the linker to the antibody) or to the effective amount of PBD compound provided, for example the amount of compound that is releasable after cleavage of the linker.

For the prevention or treatment of disease, the appropriate dosage of an ADC of the invention will depend on the type of disease to be treated, as defined above, the severity and course of the disease, whether the molecule is administered for preventive or therapeutic purposes, previous therapy, the patient's clinical history and response to the antibody, and the discretion of the attending physician. The molecule is suitably administered to the patient at one time or over a series of treatments. Depending on the type and severity of the disease, about 1 µg/kg to 15 mg/kg (e.g. 0.1-20 mg/kg) of molecule is an initial candidate dosage for administration to the patient, whether, for example, by one or more separate administrations, or by continuous infusion. A typical daily dosage might range from about 1 µg/kg to 100 mg/kg or more, depending on the factors mentioned above. An exemplary dosage of ADC to be administered to a patient is in the range of about 0.1 to about 10 mg/kg of patient weight. For repeated administrations over several days or longer, depending on the condition, the treatment is sustained until a desired suppression of disease symptoms occurs. An exemplary dosing regimen comprises a course of administering an initial loading dose of about 4 mg/kg, followed by additional doses every week, two weeks, or three weeks of an ADC. Other dosage regimens may be useful. The progress of this therapy is easily monitored by conventional techniques and assays.

### Treatment

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, regression of the condition, amelioration of the condition, and cure of the condition. Treatment as a prophylactic measure (i.e., prophylaxis, prevention) is also included.

The term "therapeutically-effective amount," as used herein, pertains to that amount of an active compound, or a material, composition or dosage from comprising an active compound, which is effective for producing some desired therapeutic effect, commensurate with a reasonable benefit/risk ratio, when administered in accordance with a desired treatment regimen.

Similarly, the term "prophylactically-effective amount," as used herein, pertains to that amount of an active compound, or a material, composition or dosage from comprising an active compound, which is effective for producing some desired prophylactic effect, commensurate with a reasonable benefit/risk ratio, when administered in accordance with a desired treatment regimen.

### Preparation of Antibody drug conjugates

The second aspect of the present invention relates to a method of preparing a conjugate, comprising the step of reacting a cell binding agent with a drug linker compound of the present invention, such as a formula I compound.

Antibody drug conjugates may be prepared by several routes, employing organic chemistry reactions, conditions, and reagents known to those skilled in the art, including: (1) reaction of a nucleophilic group of an antibody with a bivalent linker reagent, to form antibody-linker intermediate Ab-L, via a covalent bond, followed by reaction with an activated drug moiety reagent; and (2) reaction of a drug moiety reagent with a linker reagent, to form drug-linker reagent D-L, via a covalent bond, followed by reaction with the nucleophilic group of an antibody. According to the present invention, conjugation method (2) may be employed with a variety of antibodies and linkers to prepare the antibody-drug conjugates described herein.

Nucleophilic groups on antibodies include, but are not limited to side chain thiol groups, e.g. cysteine. Thiol groups are nucleophilic and capable of reacting to form covalent bonds with electrophilic groups on linker moieties such as those of the present invention. Certain antibodies have reducible interchain disulfides, i.e. cysteine bridges. Antibodies may be made reactive for conjugation with linker reagents by treatment with a reducing agent such as DTT (Cleland's reagent, dithiothreitol) or TCEP (tris(2-carboxyethyl)phosphine hydrochloride; Getz et al (1999) Anal. Biochem. Vol 273:73-80; Soltec Ventures, Beverly, MA). Each cysteine disulfide bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol.

### Synthesis

One possible synthesis route to a dimer intermediate of formula VIII is shown below:

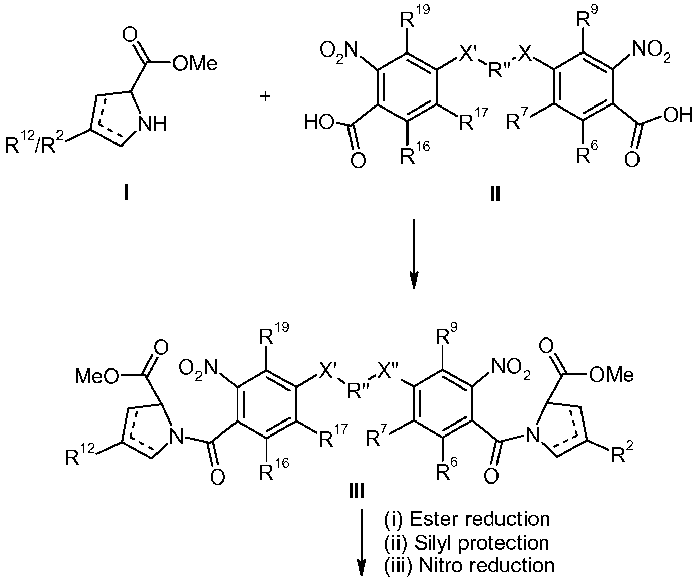

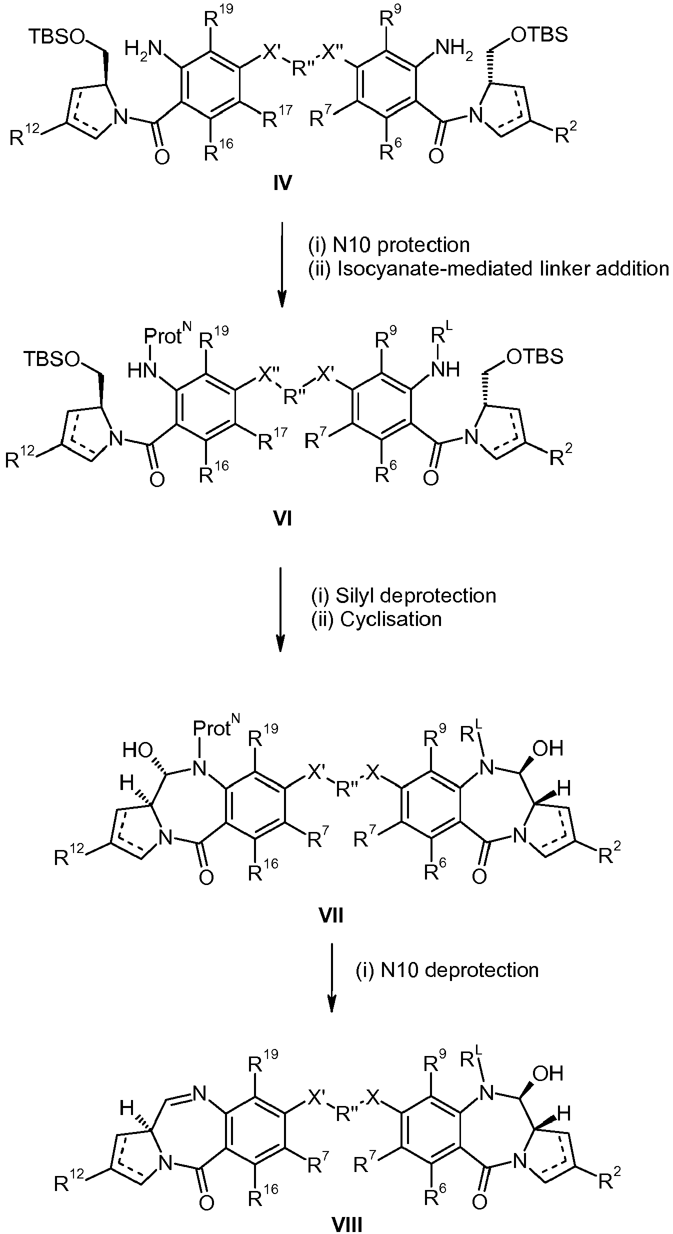

In the above scheme, R^{L} represents:

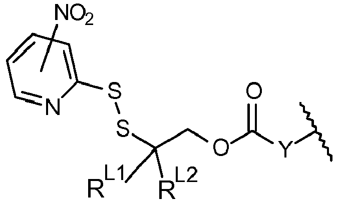

In general, unsymmetrical dimers, with respect to their N10-C11 bonds, may be prepared by treating bis-amino compounds of formula **IV** with one equivalent of a commercially available (or readily prepared) chloroformate reagent in order to break the symmetry of the molecules. The remaining free amine can then be functionalised independently to introduce the linking group precursor (R^{L}). Further functional group manipulation to close the PBD B-ring, remove protecting groups affords the target molecule.

Compounds of formula **IV** are typically prepared by coupling a suitably functionalised C-ring fragment (I) to an A-ring containing dimer core of formula **II.** C-ring fragments may be prepared from known carbamate protected methyl 4-oxoprolinate building blocks. Olefination under Wittig or Horner-Emmons conditions can be employed to furnish *endo-* or exo-unsaturated alkenes. C-ring and A-ring fragments can be coupled under standard conditions in the presence of triethylamine, using acid chloride derivatives of the A-ring fragments to give molecules of formula **III.** Symmetry may also be broken at this stage by introducing different C-rings. Compounds of type **III** can be reduced, without affecting *endo* or exo C-ring unsaturation, with zinc in acetic or formic acid to afford molecules of formula **IV**.

Alternatively, a suitable 4-hydroxy pyrrolidine building block may be coupled to a dimer core of formula II. The hydroxyl groups can be oxidized to ketones and then converted to enol triflates. Suzuki coupling can be used to introduce the pro C2 substituents (e.g. aryl, alkenyl etc). The nitro groups can then be reduced to amines, one amine is protected leaving the other free to bear the linker group.

Unsymmetrical carbamates of type **VI** can be prepared by treating bis-amines of type **IV** with a single equivalent of a commercially available (or readily prepared) chloroformates in the presence of pyridine or triethylamine. Chloroformates may be selected to afford appropriate carbamate based nitrogen protecting groups (Prot^{N}) which are orthogonal to those used in the pro-linker group (R^{L}). The R^{L} carbamate may be introduced by converting the remaining amino group to an isocyanate and quenching it with the R^{L} alcohol. Alternatively the R^{L} alcohol can be converted to a chloroformate or functional equivalent (fluoroformate, p-nitrocarbonate, pentafluorocarbonate or hydroxybenzotriazole carbonate). Finally, the remaining amino group can be converted to a reactive p-nitrocarbamate, pentafluorocarbamate or hydroxybenzotriazole carbamate which can be displaced with the R^{L} alcohol to afford molecules of formula **VI.**

Molecules of formula **VII** can be prepared from molecules of formula **VI** by removing the silyl protecting groups, with, for example, aqueous acetic acid. Oxidation with Dess-Martin periodinane (or alternatively TPAP/NMO, PDC or under Swern conditions) affords the ring closed product.

Conjugates of formula **V** may be prepared from molecules of formula **VII** by removal of the carbamate based nitrogen protection group.

### Compound II

The synthesis of compounds of formula **II** is described in WO 2006/111759 and is also described by Gregson et al. (J. Med. Chem. 2001, 44, 1161-1174). The preparation of compound (II) as described therein is specifically incorporated by reference herein.

Reference is also made to the known methods of synthesising PBD dimers, including those reviewed in Antonow, D. and Thurston, D.E., Chem. Rev. 2011 111 (4), 2815-2864.

Further relevant disclosure may be found in WO 2010/091150. The intermediate compounds described in WO 2010/091150 may also be employed in the methods described above.

For example, the dimer compound (15) shown in paragraph [164] may be used as compound **III** in Scheme I above. This, and further adaptations, would be apparent to one of skill in the art.

### Examples

### General Information

Reaction progress was monitored by thin-layer chromatography (TLC) using Merck Kieselgel 60 F254 silica gel, with fluorescent indicator on aluminium plates. Visualisation of TLC was achieved with UV light or iodine vapour unless otherwise stated. Flash chromatography was performed using VWR silica gel for flash chromatography. Extraction and chromatography solvents were bought and used without further purification from Fisher Scientific, U.K. All fine chemicals were purchased from Sigma-Aldrich or TCI Europe unless otherwise stated.

¹H and ¹³C NMR spectra were obtained on a Bruker Avance® 400 spectrometer. Coupling constants are quoted in hertz (Hz). Chemical shifts are recorded in parts per million (ppm) downfield from tetramethylsilane. Spin multiplicities are described as s (singlet), bs (broad singlet), d (doublet), t (triplet), q (quartet), p (pentuplet) and m (multiplet).

The analytical LC/MS conditions were as follows: Positive mode electrospray mass spectrometry was performed using a Shimadzu Nexera®/Prominence® LCMS-2020. Mobile phases used were solvent A (H₂O with 0.1 % formic acid) and solvent B (CH₃CN with 0.1 % formic acid). Gradient for routine 3-minute run: Initial composition 5% B held over 0.25 minutes, then increased from 5% B to 100% B over a 2 minute period. The composition was held for 0.50 minutes at 100% B, then returned to 5% B in 0.05 minutes and held there for 0.05 minutes. The total duration of the gradient run was 3.0 minutes. Gradient for 15-minute run: Initial composition 5% B held over 1 minute, then increased from 5% B to 100% B over a 10 minute period. The composition was held for 2 minutes at 100% B, then returned to 5% B in 0.1 minute and held there for 2.9 minutes. The total duration of the gradient run was 15.0 minutes. Flow rate was 0.8 mL/minute and 0.6 mL/minute (for 15-minute run). Detection was at 214 and 254 nm. Columns: Waters Acquity UPLC® BEH Shield RP18 1.7µm 2.1 x 50 mm at 50 °C fitted with Waters Acquity UPLC® BEH Shield RP18 VanGuard Pre-column, 130A, 1.7µm, 2.1 mm x 5 mm (routine 3-minute run); and Phenomenex® Gemini® 3 µm NX-C18 110 A, LC Column 100 x 2 mm (15-minute run).

### Example 1

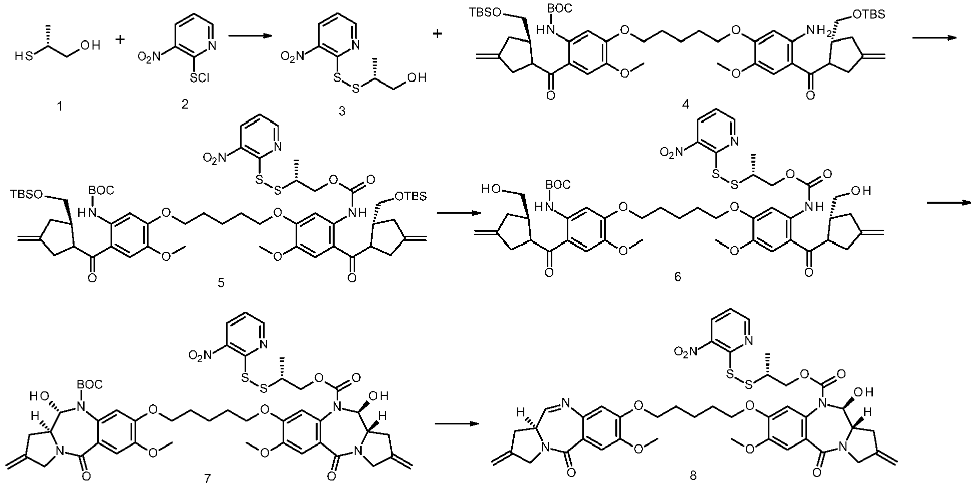

### (a) (R)-2-((3-Nitropyridin-2-yl)disulfanyl)propan-1-ol (3)

A solution of (R)-2-mercaptopropan-1-ol **1** (0.4 g, 4.35 mmol, 1.0 eq.) in dry DCM (14 mL) was added drop wise to a solution of 3-nitropyridin-2-yl hypochlorothioite **2** (1.0 g 5.22 mmol, 1.2 eq) in dry DCM (40 mL) under an argon atmosphere at 0°C with stirring. The mixture was stirred at room temperature for 3 hours. The reaction mixture was evaporated under reduced pressure to give a yellow gum. The gum was re-dissolved in water and the solution was basified with ammonium hydroxide solution (pH12), extracted with DCM (4 x 50 mL) and the combined extracts were washed with saturated brine (100 mL), dried (MgSO₄) and evaporated to give an orange oil/solid mixture. Purification by flash column chromatography [gradient elution DCM/MeOH 0% to 1%] gave the product as a yellow semi-solid (0.745 g, 70%). Analytical Data: RT 1.41 min; MS (ES⁺) *m*/*z* (relative intensity) 247 ([M + H]^{+.}, 100).

### (b) tert-Butyl (2-((S)-2-(((tert-butyldimethylsilyl)oxy)methyl)-4-methylenepyrrolidine-1-carbonyl)-5-((5-(4-((S)-2-(((tert-butyldimethylsilyl)oxy)methyl)-4-methylenepyrrolidine-1-carbonyl)-2-methoxy-5-((((R)-2-((3-nitropyridin-2-yl)disulfanyl)propoxy)carbonyl)amino)phenoxy)pentyl)oxy)-4-methoxyphenyl)carbamate (5)

Triethylamine (0.25 g, 0.34 mL, 2.5 mmol, 2.2 eq.) was added to a stirred solution of the mono-boc protected *bis*-aniline **4** (106 g, 1.11 mmol, 1.0 eq.) and triphosgene (0.12 g, 0.4 mmol, 0.36 eq.) in dry THF (15 mL) under an argon atmosphere at room temperature. The reaction mixture was heated to 40°C and after 5 minutes a sample was treated with methanol and analysed by LCMS as the methyl carbamate.

A solution of (*R*)-2-((3-Nitropyridin-2-yl)disulfanyl)propan-1-ol **3** (0.334 g, 1.36 mmol, 1.5 eq.) and triethylamine (0.17 g, 0.23 mL, 1.67 mmol, 1.5 eq.) in dry THF (15 mL) was added drop wise to the freshly prepared isocyanate. The reaction mixture was heated at 40°C for 4 hours and then stirred at room temperature for 18 hours. The reaction mixture was filtered to remove triethylamine hydrochloride and the filtrate was evaporated to dryness to afford the crude product. Purification by flash column chromatography [55% *n*-hexane/45%] gave the desired product as a yellow foam (0.44 g, 32%). Analytical Data: RT 2.42 min; MS (ES⁺) *m*/*z* (relative intensity) 1225 ([*M* + H]^{+.}, 70), 1247 ([*M* + Na]^{+.}, 100).

### (c) tert-butyl (2-((S)-2-(hydroxymethyl)-4-methylenepyrrolidine-1-carbonyl)-5-((5-(4-((S)-2-(hydroxymethyl)-4-methylenepyrrolidine-1-carbonyl)-2-methoxy-5-((((R)-2-((3-nitropyridin-2-yl)disulfanyl)propoxy)carbonyl)amino)phenoxy)pentyl)oxy)-4-methoxyphenyl)carbamate (6)

Acetic acid/H₂O (3/1, 16 mL) was added to a solution, of the *bis*-silyl ether **5** (0.41 g, 0.33 mmol, 1.0 eq.) in THF (4 mL). The resultant solution was stirred at room temperature for 6.5 hours. The reaction mixture was basified to pH8 with saturated sodium bicarbonate solution. The mixture was extracted with ethylacetate (4 x 100 mL) and the combined extracts were washed with saturated sodium bicarbonate solution (2 x 200 mL), water (200 mL), saturated brine (200 mL), dried (MgSO₄) and evaporated under reduced pressure. Purification by flash column chromatography [EtOAc] gave the product as a yellow foam (0.235 g, 71%). Analytical Data: RT 1.8 min; MS (ES⁺) *m*/*z* (relative intensity) 997 ([*M* + H]^{+.}, 100)

### (d) tert-butyl (11S,11aS)-11-hydroxy-8-((5-(((11S,11aS)-11-hydroxy-7-methoxy-2-methylene-10-(((R)-2-((3-nitropyridin-2-yl)disulfanyl)propoxy)carbonyl)-5-oxo-2,3,5,10,11,11a-hexahydro-1H-pyrrolo[2,1-c][1,4]benzodiazepin-8-yl)oxy)pentyl)oxy)-7-methoxy-2-methylene-5-oxo-2,3,11,11a-tetrahydro-1H-pyrrolo[2,1-c][1,4]benzodiazepine-10(5H)-carboxylate (7)

A solution of dry DMSO (79 mg, 72 µL, 1.01 mmol, 4.4 eq.) in dry DCM (5 mL) was added drop wise to a solution of oxalyl chloride (2.0M in DCM, 276 µL, 0.55 mmol, 2.4 eq.) in anhydrous DCM (5 mL) at -40°C under an argon atmosphere. The solution was stirred at - 40°C for 15 minutes. A solution of *bis*-alcohol **6** (0.23 g, 0.23 mmol, 1.0 eq.) in dry DCM (10 mL) was added drop wise and the resultant mixture stirred at -40°C for 45 min. During this time the temperature was allowed to reach -25°C. The temperature was lowered to -40°C and triethylamine (0.23 g, 0.32 mL, 2.3 mmol, 10 eq.) was added drop wise. After 5 minutes the temperature was allowed to reach room temperature. After a further 30 minutes the reaction mixture was diluted with DCM (50 mL) and extracted with 1 M citric acid solution (2 x 100 mL), saturated sodium bicarbonate solution (200 mL), water (200 mL), brine (200 mL), dried (MgSO₄) and evaporated under reduced pressure to give a yellow foam. Purification by flash column chromatography [chloroform/methanol 0% to 2% in 0.5% increments] gave the product as a white foam (0.085 g, 37%). Analytical Data: RT 1.69 min; MS (ES⁺) *m*/*z* (relative intensity) 993 ([*M* + H]^{+.}, 60).

### Example 2

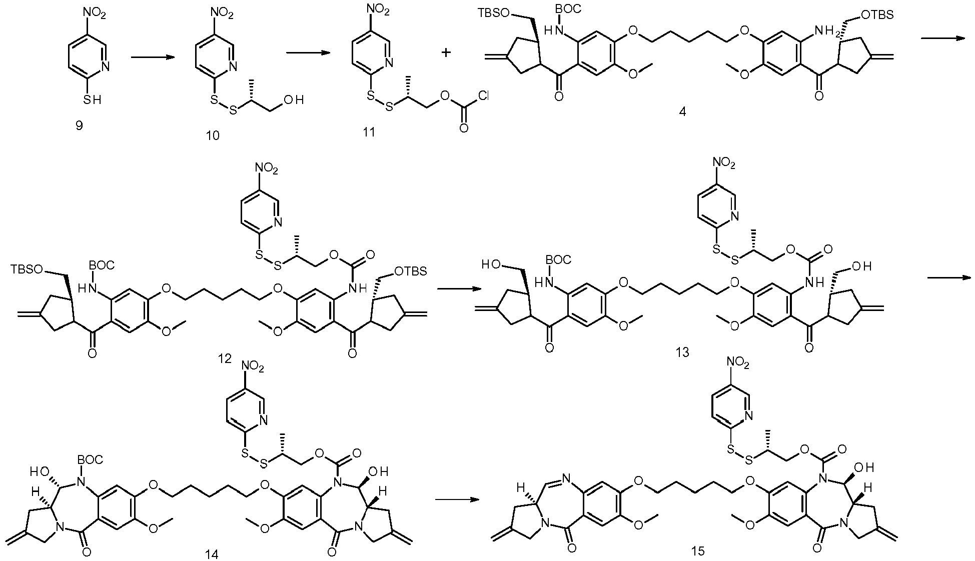

### (a) (R)-2-((5-nitropyridin-2-yl)disulfanyl)propan-1-ol (10)

Sulfuryl chloride (2.35 mL of a 1.0M solution in DCM, 2.35 mmol) was added drop-wise to a stirred suspension of 5-nitropyridine-2-thiol **9** (334 mg, 2.14 mmol) in dry DCM (7.5 mL) at 0°C (ice/acetone) under an argon atmosphere. The reaction mixture turned from a yellow suspension to a yellow solution and was allowed to warm to room temperature then stirred for 2 hours after which time the solvent was removed by evaporation *in vacuo* to provide a yellow solid. The solid was re-dissolved in DCM (15 mL) and treated drop-wise with a solution of (*R*)-2-mercaptopropan-1-ol (213 mg, 2.31 mmol) in dry DCM (7.5 mL) at 0°C under an argon atmosphere. The reaction mixture was allowed to warm to room temperature and stirred for 20 hours at which point analysis by LC/MS revealed substantial product formation at retention time 1.41 minutes (ES+) *m*/*z* 247 *([M+* H]^{+.}, ∼100% relative intensity). The precipitate was removed by filtration and the filtrate evaporated *in vacuo* to give an orange solid which was treated with H₂O (20 mL) and basified with ammonium hydroxide solution. The mixture was extracted with DCM (3 x 25 mL) and the combined extracts washed with H₂O (20 mL), brine (20 mL), dried (MgSO₄), filtered and evaporated *in vacuo* to give the crude product. Purification by flash chromatography (gradient elution in 1% increments: 100% DCM to 98:2 v/v DCM/MeOH) gave the dilsulfide **10** as an oil (111 mg, 21% yield).

### (b) (R)-2-((5-nitropyridin-2-yl)disulfanyl)propyl carbonochloridate (11)

Triphosgene (48 mg, 0.16 mmol) was added to a stirred solution of (*R*)-2-((5-nitropyridin-2-yl)disulfanyl)propan-1-ol **10** (111 mg, 0.45 mmol) and pyridine (34 µL, 33.5 mg, 0.42 mmol) in dry DCM (5 mL). The reaction mixture was allowed to stir under an argon atmosphere for 45 minutes after which time the solvent was removed by evaporation *in vacuo* to provide the crude chloroformate **11** as a yellow film. The product was carried through to the next step without purification or analysis.

### (c) tert-Butyl (2-((S)-2-(((tert-butyldimethylsilyl)oxy)methyl)-4-methylenepyrrolidine-1-carbonyl)-5-((5-(4-((S)-2-(((tert-butyldimethylsiyl)oxy)methyl)-4-methylenepyrrolidine-1-carbonyl)-2-methoxy-5-((((R)-2-((5-nitropyridin-2-yl)disulfanyl)propoxy)carbonyl)amino)phenoxy)pentyl)oxy)-4-methoxyphenyl)carbamate (12)

A solution of **11** (∼139 mg, 0.45 mmol) in dry DCM (5 mL) was added drop-wise to a stirred solution of aniline **4** (430 mg, -0.45 mmol) and pyridine (40 µL, 39 mg, 0.49 mmol) in dry DCM (12 mL) at room temperature. The reaction mixture was allowed to stir under an argon atmosphere for 2.5 hours at which point analysis by LC/MS revealed substantial product formation at retention time 2.42 minutes (ES+) *m*/*z* 1226 *([M+* H]^{+.}, ∼20% relative intensity), 1248 ([*M*+ Na]^{+.}, ∼60% relative intensity). The mixture was diluted with DCM (20 mL) and treated with SiO₂ and the solvent removed by evaporation *in vacuo.* The resulting residue was subjected to purification by flash chromatography (gradient elution in 10% increments: 80:20 v/v hexane/EtOAc to 70:30 v/v hexane/EtOAc) to give the pure carbamate **12** as a yellow foam (419 mg, 76% yield).

### (d) tert-Butyl (2-((S)-2-(hydroxymethyl)-4-methylenepyrrolidine-1-carbonyl)-5-((5-(4-((S)-2-(hydroxymethyl)-4-methylenepyrrolidine-1-carbonyl)-2-methoxy-5-((((R)-2-((5-nitropyridin-2-yl)disulfanyl)propoxy)carbonyl)amino)phenoxy)pentyl)oxy)-4-methoxyphenyl)carbamate (13)

Glacial acetic acid (24 mL) was added to a stirred solution of the TBS-protected compound **12** (419 mg, 0.34 mmol) in THF (8 mL) and H₂O (8 mL). The reaction mixture was allowed to stir for 16 hours at which point analysis by LC/MS revealed reaction completion with desired product observed at retention time 1.82 minutes (ES+) *m*/*z* 997 ([*M*+ H]^{+.}, ∼100% relative intensity), 1019 ([*M*+ Na]^{+.}, ∼45% relative intensity). The reaction mixture was added drop-wise to a chilled (0-5°C) saturated solution of NaHCO₃ (400 mL). The neutral solution was allowed to warm to room temperature and extracted with EtOAc (4 x 100 mL), the combined organic layers were washed with H₂O (80 mL), brine (100 mL), dried (MgSO₄), filtered and evaporated *in vacuo* to give the crude product. Purification by flash chromatography (gradient elution in 1% increments: 100% DCM to 98:2 v/v DCM/MeOH) gave the *bis*-alcohol **13** as a yellowish foam (341 mg, 100% yield).

### (e) tert-Butyl (11S,11aS)-11-hydroxy-8-((5-(((11S,11aS)-11-hydroxy-7-methoxy-2-methylene-10-(((R)-2-((5-nitropyridin-2-yl)disulfanyl)propoxy)carbonyl)-5-oxo-2,3,5,10,11,11a-hexahydro-1H-pyrrolo[2,1-c][1,4]benzodiazepin-8-yl)oxy)pentyl)oxy)-7-methoxy-2-methylene-5-oxo-2,3,11,11a-tetrahydro-1H-pyrrolo[2,1-c][1,4]benzodiazepine-10(5H)-carboxylate (14)

A solution of anhydrous DMSO (107 µL, 188 mg, 1.50 mmol) in dry DCM (7.5 mL) was added drop-wise to a stirred solution of oxalyl chloride (410 µL of a 2.0M solution in DCM, 0.82 mmol) in dry DCM (7.5 mL) at -45 °C (dry ice/CH₃CN) under an argon atmosphere. After 15 minutes stirring at -45°C, the reaction mixture was treated drop-wise with a solution of the *bis*-alcohol **13** (341 mg, 0.34 mmol) in dry DCM (15 mL). After stirring at -45 °C for a further 1 hour, the reaction mixture was treated drop-wise with a solution of TEA (476 µL, 342 mg, 3.42 mmol) in dry DCM (7.5 mL). The reaction mixture was allowed to warm to room temperature over a period of 1.5 hours and diluted with DCM (50 mL) then washed with saturated NH₄Cl (15 mL), saturated NaHCO₃ (15 mL), brine (15 mL), dried (MgSO₄), filtered and evaporated *in vacuo* to give the crude product. Purification by flash chromatography (gradient elution in 0.4% increments: 100% DCM to 98.4:1.6 v/v DCM/MeOH) gave the cyclised compound **14** as a yellowish foam (227 mg, 67% yield): LC/MS **14** retention time 1.69 minutes (ES+) *m*/*z* 993 ([M+ H]^{+.}, ∼80% relative intensity), 1015 ([*M*+ Na]^{+.}, ∼20% relative intensity).

### (f) (R)-2-((5-nitropyridin-2-yl)disulfanyl)propyl(11S,11aS)-11-hydroxy-7-methoxy-8-((5-(((S)-7-methoxy-2-methylene-5-oxo-2,3,5,11a-tetrahydro-1H-pyrrolo[2,1-c][1,4]benzo diazepin-8-yl)oxy)pentyl)oxy)-2-methylene-5-oxo-2,3,11,11a-tetrahydro-1H-pyrrolo[2,1-c][1,4]benzodiazepine-10(5H)-carboxylate (15)

A solution of 95:5 v/v TFA/H₂O (4 mL) was added to a crude sample of the Boc/THP-protected compound **14** (216 mg, 0.22 mmol) at 0°C (ice/acetone). After stirring at 0°C for 30 minutes the reaction was deemed complete as judged by LC/MS, desired product peak at retention time 1.60 minutes (ES+) *m*/*z* 875 *([M+* H]^{+.}, ∼100% relative intensity). The reaction mixture was kept cold and added drop-wise to a chilled saturated aqueous solution of NaHCO₃ (100 mL). The mixture was extracted with DCM (3 x 30 mL) and the combined organic layers washed with brine (50 mL), dried (MgSO₄), filtered and evaporated *in vacuo* to provide the crude product. Purification by flash chromatography (gradient elution in 0.4% increments: 100% CHCl₃ to 98.4:1.6 v/v CHCl₃/MeOH) gave **15** as a yellow foam (127 mg, 66% yield): LC/MS (15-minute run), retention time 6.18 minutes (ES+) *m*/*z* 875 ([*M*+ H]^{+.}, ∼100% relative intensity); ¹H NMR (400 MHz, CDCl₃) δ 9.21 (s, 1H), 8.30 (d, 1 H, *J* = 8.8 Hz), 7.69 (d, 1H, *J* = 4.5 Hz), 7.62 (d, 1 H, *J* = 8.9 Hz), 7.49 (s, 1H), 7.25 (s, 1H), 6.79 (s, 1H), 6.74 (s, 1H), 5.58 (dd, 1H, *J* = 4.4, 9.8 Hz), 5.22-5.10 (m, 4H), 4.43 (d, 1H, *J* = 3.7 Hz), 4.33-4.25 (m, 4H), 4.15-3.98 (m, 5H), 3.95-3.80 (m, 7H), 3.68-3.59 (m, 1H), 3.20-3.07 (m, 2H), 2.99-2.87 (m, 2H), 2.76-2.68 (m, 2H), 1.99-1.83 (m, 4H), 1.72-1.57 (m, 2H), 1.19 (d, 3H, *J* = 6.6 Hz).

### Example 3

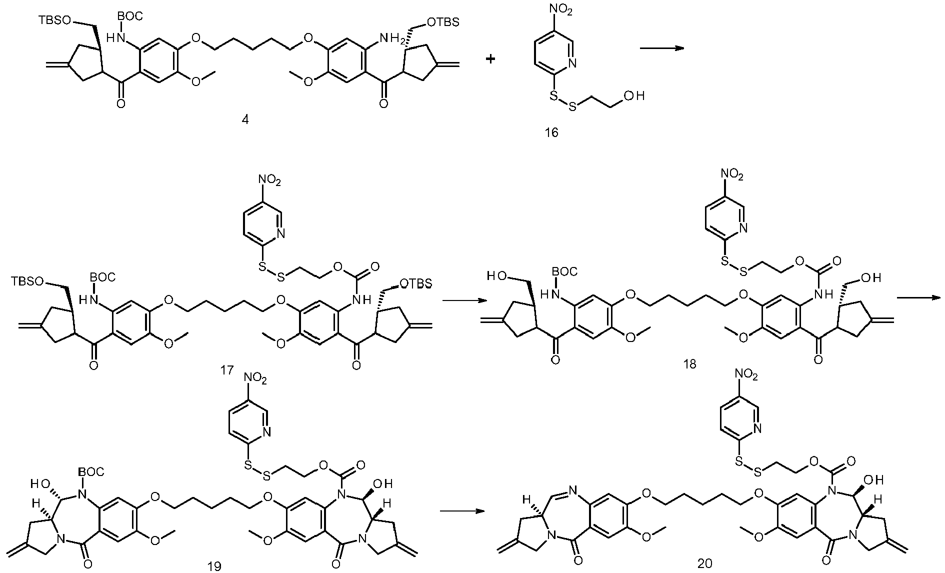

(a) A solution of triphosgene (210 mg, 0.71 mmol) in dry THF (30.0 mL) was added to a solution of compound **4** (1.5 g, 1.57 mmol) and Et₃N (475 mg, 4.69 mmol) in dry THF (5.0 mL) dropwise in ice bath. It was stirred at 20°C for 1.0 hour under N₂. A sample of reaction mixture was treated with MeOH and analyzed by LCMS and methyl carbamate was found. Then a solution of compound **16** (401 mg, 1.73 mmol) and Et₃N (436 mg, 4.31 mmol) in THF (5.0 mL) was added to the freshly the prepared isocyanate. The mixture was stirred at 40°C for 1.5 hours and extra triphosgene (93 mg, 0.31 mmol) was added. After 30 minutes the reaction mixture was cooled to room temperature, filtered to remove triethylamine hydrochloride and the filtrate was extracted with EtOAc (3 x 200 mL). The combined organic layer was dried over Na₂SO₄, concentrated and purified flash column chromatography (PE: EtOAc=1:1) to give the desired product **17** as a yellow oil (810 mg yield: 43%). LCMS: (10-80, AB, 1.5 min), RT=1.20 min, *m*/*z* = 1212.3[M+1]⁺.
(b) A mixture of HOAc and H₂O (3/1) (12.0 mL) was added to a solution of compound **17** (810 mg, 0.67 mmol) in THF (4.5 mL). The solution was stirred at 20°C for 18.0 hours. The pH of the reaction mixture was adjusted to pH = 8.0 with saturated NaHCO₃ solution. The mixture was extracted with EtOAc (3 x 100 mL) and the combined extracts were washed with saturated NaHCO₃ solution (100 mL), water (100 mL), brine (100 mL), dried over MgSO₄ and concentrated under reduced pressure. The residue was purified by flash chromatography (DCM : MeOH = 15:1) to give the desired product **18** as a yellow solid (410 mg, 62.3%). LCMS: (10-80, AB, 1.5 min), RT=0.867 min, *m*/*z* = 983.2[M+1]⁺.
(c) To a solution of compound **18** (130 mg, 0.13 mmol) in DCM (20.0 mL) was added DMP (224 mg, 0.53 mmol). The mixture was stirred at 20°C for 2.0 hours. LCMS showed about 57% of desired product. The mixture was filtered, the filtrate was washed with water (2 x 15 mL), dried over MgSO₄, and concentrated. It was purified by pre-TLC (DCM: MeOH = 20:1) to give the desired product **19** as a yellow solid (70 mg, 54%). LCMS: (10-80, AB, 1.5 min), RT = 0.811 min, *m*/*z* = 1002.2[M+23]⁺.
(d) TFA (3.0 mL) was added dropwise to compound **19** (100 mg, 0.1 mmol) at 0°C and the mixture was stirred at 0°C for 20 minutes. The mixture was added to a saturated NaHCO₃ solution at 0°C and extracted with DCM (3 x 100 mL). The organic layer was dried over Na₂SO₄, concentrated and purified by pre-TLC (DCM:MeOH=16:1) to give the desired product **20** as a white solid (70 mg, yield: 80%). LCMS: (10-80, AB, 1.5 min), RT = 0.715 min, *m*/*z* = 861.2[M+1]⁺; ¹H NMR (400MHz, CDCl₃) *δ* 9.16 (s, 1 H), 8.25-8.23 (d, *J* = 9.2 Hz, 1 H), 7.63-7.62 (d, *J* = 4.4 Hz, 1 H), 7.56-7.54(d, *J* = 8.8 Hz, 1 H), 7.42 (s, 1 H), 7.18 (s, 1 H), 6.71 (s, 1H), 6.64 (s, 1 H), 5.51-5.49 (d, *J* = 10 Hz, 1 H), 5.23 (s, 1 H), 5.14-5.08 (m, 3H), 4.33-4.0 (m, 9H), 3.98-3.85 (m, 9H), 3.10-2.64 (m, 7 H), 1.85-1.84 (m, 6H).

### Example 4

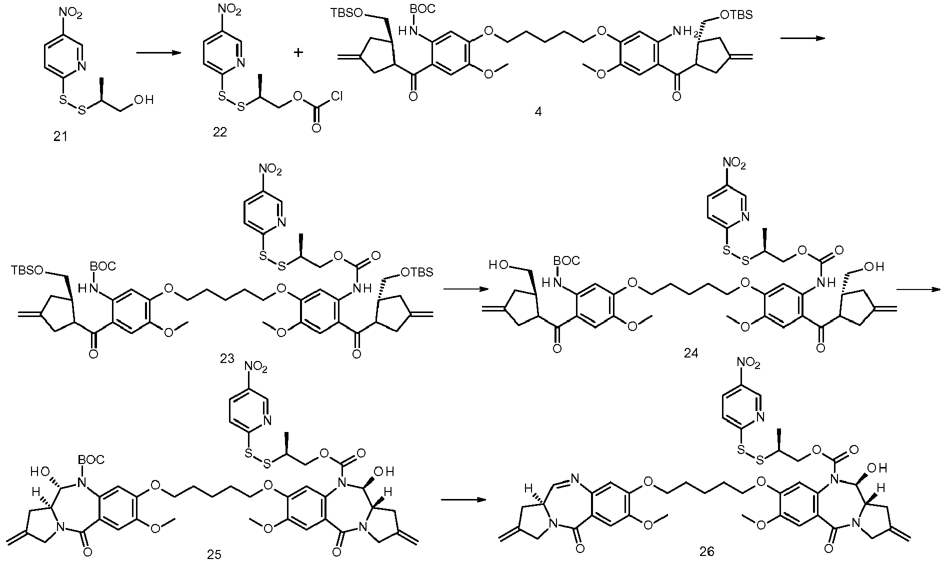

(a) Triphosgene (72.29 mg, 0.243 mmol) was added to a stirred solution of compound **21** (150 mg, 0.609 mmol) and pyridine (45.76 mg, 0.577 mmol) in DCM (5 mL) at 20°C. The reaction mixture was stirred at 20°C for 40 minutes. The solvent was removed and the residue was used directly in the next step.
(b) A solution of compound **22** (168 mg, 0.545 mmol) in DCM (5 mL) was added drop-wise to a solution of compound **4** (400 mg, 0.419 mmol) and pyridine (43 mg, 0.545 mmol) in DCM (5 mL) at 20°C. The reaction mixture was stirred at 20°C for 2 hours. Solvent was removed and the residue was purified by pre-TLC (PE: EtOAc=3:2) to give the desired product **23** (160 mg, 31%) as a yellow solid. LCMS: (5-95, AB, 1.5 min), 1.199 min, *m*/*z* = 1225.4 (M+1).
(c) To a solution of compound **23** (160 mg, 0.13 mmol) in THF/H₂O (3 mL/3 mL) was added HOAc (5 mL) at 20°C. The reaction mixture was stirred at 20°C for 16 hours. The reaction mixture was diluted with EtOAc (20 mL), washed with water (2 x 10 mL), saturated aq. NaHCO₃ (2 x 10 mL) and brine (10 mL). It was dried and concentrated to give the crude product which was purified by pre-TLC (DCM:MeOH = 15:1) to give the pure desired product **24** (110 mg, 85%) as a yellow foam.
(d) To a solution of compound **24** (110 mg, 0.11 mmol) in DCM (5 mL) was added DMP (187 mg, 0.441 mmol) at 0°C. After the reaction mixture was stirred at 20°C for 2 hours, it was quenched with a NaHCO₃/Na₂SO₃ saturate solution (5 mL/5 mL) and extracted with DCM (3 x 10 mL). The combined organic layer was washed with a NaHCO₃/ Na₂SO₃ solution (5 mL/5 mL), brine (10 mL), dried and concentrated. The residue was purified by pre-TLC (DCM:MeOH = 15:1) to give the desired product **25** (65 mg, 59 %) as a yellow foam. LCMS: (5-95, AB, 1.5 min), 0.772 min, *m*/*z* = 1015.4 (M+23).
(e) Cold TFA (10 mL) was added to compound **25** (65 mg, 0.065 mmol) at 0°C. After the reaction mixture was stirred at 0°C for 30 minutes, it was added dropwise to a cold saturate aq. NaHCO₃ (20 mL) at 0°C and extracted with DCM (4 x 20 mL). The combined organic layer was washed with brine (30 mL), dried and concentrated to give the crude product which was purified by pre-TLC (DCM:MeOH=15:1) to give the pure desired product **26** (32 mg, 55.88%) as a yellow foam. LCMS: (5-95, AB, 1.5 min), 0.877 min, *m*/*z* = 875.4 (M+1); ¹H NMR (400 MHz, CDCl₃) *δ* 9.19 (s, 1 H), 8.32 (d, *J* = 8.8 Hz, 1 H), 7.67 (d, *J* = 4.4 Hz, 1 H), 7.55 (d, *J* = 8.8 Hz, 1 H), 7.47 (s, 1 H), 6.77 (s, 1 H), 6.69 (s, 1 H), 5.57 (d, *J* = 9.6 Hz, 1 H), 5.19-5.13 (m, 4H), 4.40-4.20 (m, 4H), 4.15-3.90 (m, 14H), 3.61 (m, 1 H), 3.47 (s, 1 H), 3.20-2.63 (m, 4H), 1.89 (t, *J* = 6.8 Hz, 2H), 1.71-1.50 (m, 4H), 1.25-1.21 (m, 3H).

### Reduction/Oxidation of cysteine-engineered antibody mutants (THIOMAB™) for Conjugation

Full length, cysteine engineered monoclonal antibodies (cysteine-engineered antibody mutants (THIOMAB™)- Junutula, et al., 2008b Nature Biotech., 26(8):925-932; Dornan et al (2009) Blood 114(13):2721-2729; US 7521541; US 7723485; WO2009/052249, Shen et al (2012) Nature Biotech., 30(2):184-191; Junutula et al (2008) Jour of Immun. Methods 332:41-52) expressed in CHO cells were reduced with about a 20-40 fold excess of TCEP (tris(2-carboxyethyl)phosphine hydrochloride or DTT (dithiothreitol) in 50 mM Tris pH 7.5 with 2 mM EDTA for 3 hrs at 37°C or overnight at room temperature.(Getz et al (1999) Anal. Biochem. Vol 273:73-80; Soltec Ventures, Beverly, MA). The reduced cysteine-engineered antibody mutants (THIOMAB™) were diluted and loaded onto a HiTrap S® column in 10 mM sodium acetate, pH 5, and eluted with PBS containing 0.3M sodium chloride. Alternatively, the antibody was acidified by addition of 1/20^{th} volume of 10% acetic acid, diluted with 10 mM succinate pH 5, loaded onto the column and then washed with 10 column volumes of succinate buffer. The column was eluted with 50 mM Tris pH7.5, 2 mM EDTA.

The eluted reduced cysteine-engineered antibody mutants (THIOMAB™) was treated with 15 fold molar excess of DHAA (dehydroascorbic acid) or 200 nM aqueous copper sulfate (CuSO₄). Oxidation of the interchain disulfide bonds was complete in about three hours or more. Ambient air oxidation was also effective. The re-oxidized antibody was dialyzed into 20 mM sodium succinate pH 5, 150 mM NaCl, 2 mM EDTA and stored frozen at -20°C.

### Conjugation of cysteine-engineered antibodies with Compounds to prepare antibody-drug conjugates

The deblocked, reoxidized, thio-antibodies (cysteine-engineered antibody mutants (THIOMAB™)) were reacted with 6-8 fold molar excess of the compounds above (from a DMSO stock at a concentration of 20 mM) in 50 mM Tris, pH 8, until the reaction was complete (16-24 hours) as determined by LC-MS analysis of the reaction mixture.

The crude antibody-drug conjugates (ADC) were then applied to a cation exchange column after dilution with 20 mM sodium succinate, pH 5. The column was washed with at least 10 column volumes of 20 mM sodium succinate, pH 5, and the antibody was eluted with PBS. The antibody drug conjugates were formulated into 20 mM His/acetate, pH 5, with 240 mM sucrose using gel filtration columns. The antibody-drug conjugates were characterized by UV spectroscopy to determine protein concentration, analytical SEC (size-exclusion chromatography) for aggregation analysis and LC-MS before and after treatment with Lysine C endopeptidase.

Size exclusion chromatography was performed using a Shodex KW802.5 column in 0.2M potassium phosphate pH 6.2 with 0.25 mM potassium chloride and 15% IPA at a flow rate of 0.75 ml/min. Aggregation state of the conjugate was determined by integration of eluted peak area absorbance at 280 nm.

LC-MS analysis was performed using an Agilent QTOF 6520 ESI instrument. As an example, an antibody-drug conjugate generated using this chemistry was treated with 1:500 w/w Endoproteinase Lys C (Promega) in Tris, pH 7.5, for 30 min at 37°C. The resulting cleavage fragments were loaded onto a 1000A, 8 um PLRP-S column heated to 80°C and eluted with a gradient of 30% B to 40% B in 5 minutes. Mobile phase A was H₂O with 0.05% TFA and mobile phase B was acetonitrile with 0.04% TFA. The flow rate was 0.5ml/min. Protein elution was monitored by UV absorbance detection at 280nm prior to electrospray ionization and MS analysis. Chromatographic resolution of the unconjugated Fc fragment, residual unconjugated Fab and drugged Fab was usually achieved. The obtained m/z spectra were deconvoluted using Mass Hunter™ software (Agilent Technologies) to calculate the mass of the antibody fragments.

| | **ADC** | **Antigen** | **Linker-drug SG** | **DAR** |
|---|---|---|---|---|
| 101 | Thio Hu anti-Her2 7C2 HC A118C | Her2 7C2 | 8 | 1.9 |
| 102 | Thio Hu anti-Her2 7C2 LC K149C | Her2 7C2 | 8 | |
| 103 | Thio Hu anti-CD33 HC A118C | CD33 | 8 | 1.9 |
| 104 | Thio Hu anti-CD33 LC K149C | CD33 | 8 | |
| 105 | Thio Hu anti-Her2 7C2 LC K149C | Her2 7C2 | 15 | 1.8 |
| 106 | Thio Hu anti-CD33 LC K149C | CD33 | 15 | 1.8 |
| 107 | Thio Hu anti-CD33 HC A118C | CD33 | 8 | 1.8 |
| 108 | Thio Hu anti-CD33 LC K149C | CD33 | 15 | 1.9 |
| 109 | Thio Hu anti-CLL-1 HC A118C | CLL-1 | 8 | |
| 110 | Thio Hu anti-CLL-1 HC A118C | CLL-1 | 8 | |
| 111 | Thio Hu anti-CLL-1 LC K149C | CLL-1 | 15 | |
| 112 | Thio Hu anti-CLL-1 LC K149C | CLL-1 | 15 | 1.9 |
| 113 | Thio Hu Anti-Her2 4D5 LC K149C | Her2 4D5 (trastuzumab) | 15 | 1.7 |
| 114 | Thio Hu anti-Her2 4D5 HC A140C | Her2 4D5 (trastuzumab) | 15 | 1.7 |
| 115 | Thio Hu Anti-CD33 HC A140C | CD33 | 15 | 1.9 |
| 116 | Thio Hu anti-NaPi2b LC V205C | NaPi2b | 15 | 1.8 |
| 117 | Thio Hu anti-Her2 7C2 LC K149C | Her2 7C2 | 15 | 1.84 |
| 118 | Thio Hu anti-CD33 HC S239C | CD33 | 15 | 1.8 |
| 119 | Thio Hu anti-CD33 S239C | CD33 | 15 | 1.7 |
| 120 | Thio Hu anti-CLL-1 LC K149C | CLL-1 | 15 | 2.0 |
| 121 | Thio Hu Anti-CD22 LC K149C | CD22 | 15 | 1.8 |
| 122 | Thio Hu Anti-gD 5B6 LC K149C | gD | 15 | 1.9 |
| 123 | Thio Hu anti-CD33 LC | CD33 | 15 | 1.9 |
| 124 | Thio Hu Anti-Her2 4D5 LC K149C | Her2 4D5 (trastuzumab) | 15 | 1.7 |
| 125 | Thio Hu Anti-Napi3b LC K149C | NaPi3b | 15 | 1.9 |
| 126 | Thio Hu anti-CLL-1 LC K149C | CLL-1 | 15 | |
| 127 | Thio anti-Her2 7C2 LC K149C | Her2 7C2 | 20 | |
| 128 | Thio anti-Her2 7C2 LC K149C | Her2 7C2 | 26 | |

The following *in vitro* assay is also described in Phillips et al (2008) Cancer Res. 68(22):9280-9290.

### In vitro cell proliferation assay

Efficacy of ADC were measured by a cell proliferation assay employing the following protocol (CellTiter Glo® Luminescent Cell Viability Assay, Promega Corp. Technical Bulletin TB288; Mendoza et al (2002) Cancer Res. 62:5485-5488). All cell lines were obtained from American Type Culture Collection:
1. An aliquot of 100 µl of cell culture containing about 10⁴ cells (for example, KPL-4, a human breast cancer cell line, Kurebayashi et al (1999) Brit. Jour. Cancer 79(5-6):707-717), SKBR-3, or MCF7) in medium was deposited in each well of a 96-well, opaque-walled plate.
2. Control wells were prepared containing medium and without cells.
3. ADC was added to the experimental wells and incubated for 3-5 days.
4. The plates were equilibrated to room temperature for approximately 30 minutes.
5. A volume of CellTiter-Glo Reagent equal to the volume of cell culture medium present in each well was added.
6. The contents were mixed for 2 minutes on an orbital shaker to induce cell lysis.
7. The plate was incubated at room temperature for 10 minutes to stabilize the luminescence signal.
8. Luminescence was recorded and reported in graphs as RLU = relative luminescence units.

Certain cells are seeded at 1000-2000/well or 2000-3000/well in a 96-well plate, 50 uL/well. After one or two days, ADC are added in 50 µL volumes to final concentration of 9000, 3000, 1000, 333, 111, 37, 12.4, 4.1, or 1.4 ng/mL, with "no ADC" control wells receiving medium alone. Conditions are in duplicate or triplicate After 3-5 days, 100 µL/well Cell TiterGlo II is added (luciferase-based assay; proliferation measured by ATP levels) and cell counts are determined using a luminometer. Data are plotted as the mean of luminescence for each set of replicates, with standard deviation error bars. The protocol is a modification of the CellTiter Glo Luminescent Cell Viability Assay (Promega):
1. Plate 1000 cells/ well in 50 µL/well of FBS/glutamine media. Allow cells to attach overnight.
2. ADC is serially diluted 1:3 in media beginning at working concentration 18 µg/ml (this results in a final concentration of 9 µg/ml). 50 µL of diluted ADC is added to the 50 µL of cells and media already in the well.
3. Incubate 72-96 hrs (the standard is 72 hours, but watch the 0 ug/mL concentration to stop assay when the cells are 85-95% confluent).
4. Add 100 µL/well of Promega Cell Titer Glo reagent, shake 3 min. and read on luminometer

### Results

Antibody-drug conjugates Thio Hu anti-Her2 7C2 HC A118C-**8** (**101**), Thio Hu anti-CD33 15G15.3 HC A118C-**8** (**103**), Thio Hu anti-Her2 7C2 LC K149C-**15** (**105**), Thio Hu anti-CD33 15G15.3 LC K149C-**15** (**106**) were tested against SK-BR-3 (Levenson et al (1997) Cancer Res. 57(15):3071-3078) cells to measure *in vitro* cell viability in five day studies. SK-BR-3 cells are HER2+ expressing. Both **101** and **105** were active against these cells, whereas both **103** and **106** were effectively inactive.

| **CNJ** | **IC₅₀ (ng/mL) SK-BR-3** |
|---|---|
| 101 | 5.9 |
| 103 | 1900 |
| 105 | 5.5 |
| 106 | 3000 |

The same four conjugates were tested against EOL1 and HL-60 Levenson et al (1997) Cancer Res. 57(15):3071-3078) cells to measure *in vitro* cell viability in five day studies. EOL1 and HL-60 cells are CD33 expressing. Both **103** and **106** were active against these cells, whereas both **101** and **105** were effectively inactive.

| **CNJ** | **IC₅₀ (µg/mL) EOL1** | **IC₅₀ (µg/mL) HL-60** |
|---|---|---|
| 103 | 0.7 | 6.3 |
| 101 | 28.9 | 196 |
| 106 | 2.8 | 18.6 |
| 105 | 44.8 | 271 |

In combination, these results that conjugates 101, 103, 105 and 106 exhibit targeted cell killing.

Antibody-drug conjugates Thio Hu anti-Her2 7C2 LC K149C-15 (**105**), Thio Hu anti-Her2 7C2 LC K149C-15 (**117**), Thio anti-Her2 7C2 LC K149C-20 (**127**), and Thio anti-Her2 7C2 LC K149C-26 (**128**) were also tested against SK-BR-3 (Levenson et al (1997) Cancer Res. 57(15):3071-3078) cells to measure *in vitro* cell viability in five day studies. SK-BR-3 cells are HER2+ expressing.

| **CNJ** | **IC₅₀ (ng/mL) SK-BR-3** |
|---|---|
| 105 | 1.4 |
| 117 | 2.0 |
| 127 | 20.0 |
| 128 | 2.5 |

### Tumor growth inhibition, in vivo efficacy, in transgenic explant mice

Conjugates of the invention were tested in appropriate in vivo models and shown to be active. Appropriate *in vivo* assays are described in Phillips et al (2008) Cancer Res. 68(22):9280-9290.

### Abbreviations

- Ac: acetyl
- Acm: acetamidomethyl
- Alloc: allyloxycarbonyl
- Boc: di-*tert*-butyl dicarbonate
- t-Bu: tert-butyl
- Bzl: benzyl, where Bzl-OMe is methoxybenzyl and Bzl-Me is methylbenzene
- Cbz or Z: benzyloxy-carbonyl, where Z-Cl and Z-Br are chloro- and bromobenzyloxy carbonyl respectively
- DMF: *N,N*-dimethylformamide
- Dnp: dinitrophenyl
- DTT: dithiothreitol
- Fmoc: 9*H*-fluoren-9-ylmethoxycarbonyl
- imp: *N*-10 imine protecting group: 3-(2-methoxyethoxy)propanoate-Val-Ala-PAB
- MC-OSu: maleimidocaproyl-O-*N*-succinimide
- Moc: methoxycarbonyl
- MP: maleimidopropanamide
- Mtr: 4-methoxy-2,3,6-trimethtylbenzenesulfonyl
- PAB: para-aminobenzyloxycarbonyl
- PEG: ethyleneoxy
- PNZ: *p*-nitrobenzyl carbamate
- Psec: 2-(phenylsulfonyl)ethoxycarbonyl
- TBDMS: tert-butyldimethylsilyl
- TBDPS: tert-butyldiphenylsilyl
- Teoc: 2-(trimethylsilyl)ethoxycarbonyl
- Tos: tosyl
- Troc: 2,2,2-trichlorethoxycarbonyl chloride
- Trt: trityl
- Xan: xanthyl

The following numbered clauses, describing aspects of our proposals, are part of the description.
1. A compound of formula I: 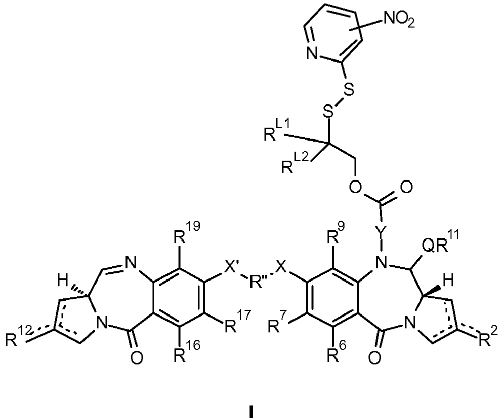
   wherein the dotted lines indicate the optional presence of a double bond between C1 and C2 or C2 and C3;
   R² is independently selected from H, OH, =O, =CH₂, CN, R, OR, =CH-R^{D}, =C(R^{D})₂, O-SO₂-R, CO₂R and COR, and optionally further selected from halo or dihalo;
   where R^{D} is independently selected from R, CO₂R, COR, CHO, CO₂H, and halo;
   R⁶ and R⁹ are independently selected from H, R, OH, OR, SH, SR, NH₂, NHR, NRR', NO₂, Me₃Sn and halo;
   R⁷ is independently selected from H, R, OH, OR, SH, SR, NH₂, NHR, NRR', NO₂, Me₃Sn and halo;
   Y is selected from a single bond, and a group of formulae A1 or A2: 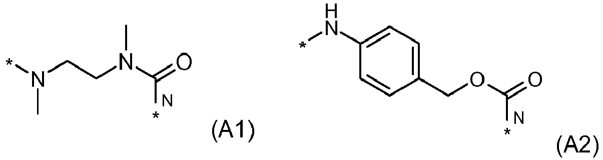
   where N shows where the group binds to the N10 of the PBD moiety;
   R^{L1} and R^{L2} are independently selected from H and methyl, or together with the carbon atom to which they are bound form a cyclopropylene group;
   Q is independently selected from O, S and NH;
   R¹¹ is either H, or R or, where Q is O, SO₃M, where M is a metal cation;
   R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
   wherein R¹², R¹⁶, R¹⁹ and R¹⁷ are as defined for R², R⁶, R⁹ and R⁷ respectively;
   wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted; and
   X and X' are independently selected from O, S and N(H).
2. The compound of clause 1, which is of formula **II**: 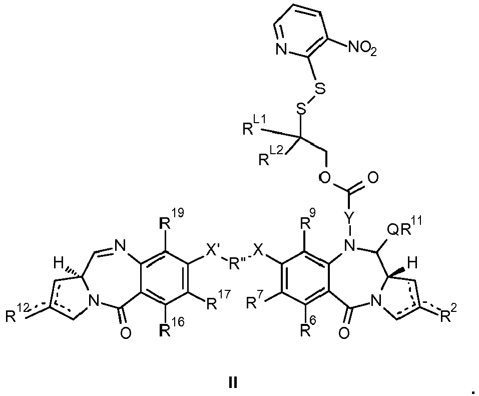
3. The compound of clause 1, which is of formula **III:** 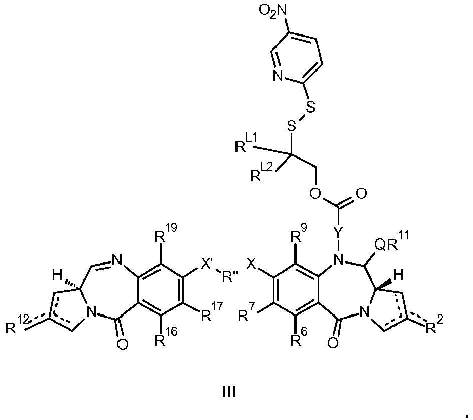
4. The compound according to any one of clauses 1 to 3, wherein R^{L1} and R^{L2} are both H.
5. The compound according to any one of clauses 1 to 3, wherein R^{L1} and R^{L2} are both methyl.
6. The compound according to any one of clauses 1 to 3, wherein one of R^{L1} and R^{L2} is H and the other is methyl.
7. The compound according to any one of clauses 1 to 6, wherein Y is a single bond.
8. The compound according to any one of clauses 1 to 6, wherein Y is: 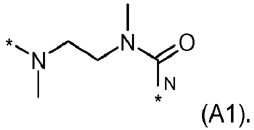
9. The compound according to any one of clauses 1 to 6, wherein Y is: 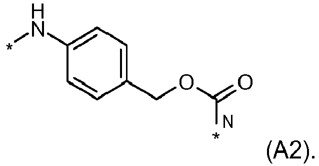
10. The compound according to any one of clauses 1 to 9, wherein R⁹ and R¹⁹ are H.
11. The compound according to any one of clauses 1 to 10, wherein R⁶ and R¹⁶ are H.
12. The compound according to any one of clauses 1 to 11, wherein R⁷ are R¹⁷ are both OR^{7A}, where R^{7A} is optionally substituted C₁₋₄ alkyl.
13. The compound of clause 12, wherein R^{7A} is Me.
14. The compound according to any one of clauses 1 to 13, wherein X is O.
15. The compound according to any one of clauses 1 to 14, wherein R¹¹ is H.
16. The compound according to any one of clauses 1 to 15, wherein there is a double bond between C2 and C3 in each monomer unit.
17. The compound according to clause 16, wherein R² and R¹² are independently selected from H and R.
18. The compound according to clause 17, wherein R² and R¹² are independently R.
19. The compound according to clause 18, wherein R² and R¹² are independently optionally substituted C₅₋₂₀ aryl.
20. The compound according to any one of clauses 1 to 15, wherein R² and R¹² are independently selected from =O, =CH₂, =CH-R^{D}, and =C(R^{D})₂.
21. The compound according to clause 20, wherein R² and R¹² are =CH₂.
22. The compound according to any one of clauses 1 to 21, wherein R" is a C₃ alkylene group or a C₅ alkylene group.
23. A method of making a conjugate of formula A: 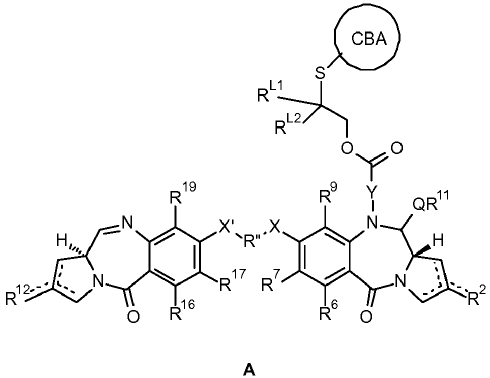
   wherein the dotted lines indicate the optional presence of a double bond between C1 and C2 or C2 and C3;
   CBA represents a cell binding agent;
      by reacting a compound according to any one of clauses 1 to 22 with a cell binding agent, wherein the groups Y, R^{L1}, R^{L2}, R², R⁶, R⁷, R⁹, Q, R¹¹, X, X', R", R¹²,R¹⁶, R¹⁹ are as defined in any one of clauses 1 to 22.
24. The method of clause 23 wherein the cell binding agent is an antibody or an active fragment thereof.
25. The method of clause 24, wherein the antibody or antibody fragment is an antibody or antibody fragment for a tumour-associated antigen.
26. The method of clause 24 wherein the antibody or antibody fragment is an antibody which binds to one or more tumor-associated antigens or cell-surface receptors selected from (1)-(53):
   (1) BMPR1 B (bone morphogenetic protein receptor-type IB);
   (2) E16 (LAT1, SLC7A5);
   (3) STEAP1 (six transmembrane epithelial antigen of prostate);
   (4) 0772P (CA125, MUC16);
   (5) MPF (MPF, MSLN, SMR, megakaryocyte potentiating factor, mesothelin);
   (6) Napi3b (NAPI-3B, NPTIIb, SLC34A2, solute carrier family 34 (sodium phosphate), member 2, type II sodium-dependent phosphate transporter 3b);
   (7) Sema 5b (FLJ10372, KIAA1445, Mm.42015, SEMA5B, SEMAG, Semaphorin 5b Hlog, sema domain, seven thrombospondin repeats (type 1 and type 1-like), transmembrane domain (TM) and short cytoplasmic domain, (semaphorin) 5B);
   (8) PSCA hlg (2700050C12Rik, C530008O16Rik, RIKEN cDNA 2700050C12, RIKEN cDNA 2700050C12 gene);
   (9) ETBR (Endothelin type B receptor);
   (10) MSG783 (RNF124, hypothetical protein FLJ20315);
   (11) STEAP2 (HGNC_8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein);
   (12) TrpM4 (BR22450, FLJ20041, TRPM4, TRPM4B, transient receptor potential cation channel, subfamily M, member 4);
   (13) CRIPTO (CR, CR1, CRGF, CRIPTO, TDGF1, teratocarcinoma-derived growth factor);
   (14) CD21 (CR2 (Complement receptor 2) or C3DR (C3d/Epstein Barr virus receptor) or Hs 73792);
   (15) CD79b (CD79B, CD79β, IGb (immunoglobulin-associated beta), B29);
   (16) FcRH2 (IFGP4, IRTA4, SPAP1A (SH2 domain containing phosphatase anchor protein 1a), SPAP1B, SPAP1C);
   (17) HER2;
   (18) NCA;
   (19) MDP;
   (20) IL20Rα;
   (21) Brevican;
   (22) EphB2R;
   (23) ASLG659;
   (24) PSCA;
   (25) GEDA;
   (26) BAFF-R (B cell -activating factor receptor, BLyS receptor 3, BR3);
   (27) CD22 (B-cell receptor CD22-B isoform);
   (28) CD79a (CD79A, CD79β, immunoglobulin-associated alpha);
   (29) CXCR5 (Burkitt's lymphoma receptor 1);
   (30) HLA-DOB (Beta subunit of MHC class II molecule (Ia antigen));
   (31) P2X5 (Purinergic receptor P2X ligand-gated ion channel 5);
   (32) CD72 (B-cell differentiation antigen CD72, Lyb-2);
   (33) LY64 (Lymphocyte antigen 64 (RP105), type I membrane protein of the leucine rich repeat (LRR) family);
   (34) FcRH1 (Fc receptor-like protein 1);
   (35) IRTA2 (Immunoglobulin superfamily receptor translocation associated 2); and
   (36) TENB2 (putative transmembrane proteoglycan)
   (37) PMEL17 (silver homolog; SILV; D12S53E; PMEL17; SI; SIL);
   (38) TMEFF1 (transmembrane protein with EGF-like and two follistatin-like domains 1; Tomoregulin-1);
   (39) GDNF-Ra1 (GDNF family receptor alpha 1; GFRA1; GDNFR; GDNFRA; RETL1; TRNR1; RET1L; GDNFR-alpha1; GFR-ALPHA-1);
   (40) Ly6E (lymphocyte antigen 6 complex, locus E; Ly67,RIG-E,SCA-2,TSA-1);
   (41) TMEM46 (shisa homolog 2 (Xenopus laevis); SHISA2);
   (42) Ly6G6D (lymphocyte antigen 6 complex, locus G6D; Ly6-D, MEGT1);
   (43) LGR5 (leucine-rich repeat-containing G protein-coupled receptor 5; GPR49, GPR67);
   (44) RET (ret proto-oncogene; MEN2A; HSCR1; MEN2B; MTC1; PTC; CDHF12; Hs.168114; RET51; RET-ELE1);
   (45) LY6K (lymphocyte antigen 6 complex, locus K; LY6K; HSJ001348; FLJ35226);
   (46) GPR19 (G protein-coupled receptor 19; Mm.4787);
   (47) GPR54 (KISS1 receptor; KISS1R; GPR54; HOT7T175; AXOR12);
   (48) ASPHD1 (aspartate beta-hydroxylase domain containing 1; LOC253982);
   (49) Tyrosinase (TYR; OCAIA; OCA1A; tyrosinase; SHEP3);
   (50) TMEM118 (ring finger protein, transmembrane 2; RNFT2; FLJ14627);
   (51) GPR172A (G protein-coupled receptor 172A; GPCR41; FLJ11856; D15Ertd747e);
   (52) CD33; and
   (53) CLL-1.
27. The method of clause 24 wherein the antibody or antibody fragment is a cysteine-engineered antibody.
28. The method of either clause 24 or clause 27 wherein Ab is anti-HER2 4D5, anti-CD22, anti-CD33, anti-Napi3b, anti-HER2 7C2, or anti-CLL-1 antibody.
29. The method according to clause 24 wherein the drug loading (p) of drugs (D) to antibody (Ab) is an integer from 1 to about 8.
30. The method according to clause 29, wherein p is 1, 2, 3, or 4.
31. A conjugate of formula A1: 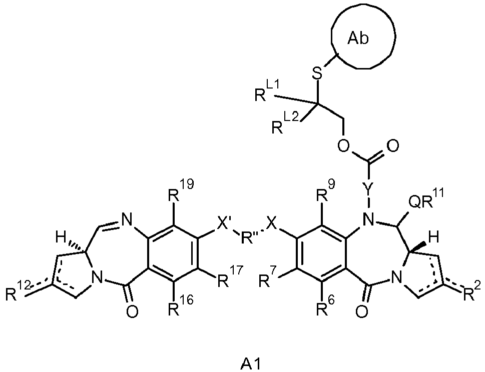
   wherein the dotted lines indicate the optional presence of a double bond between C1 and C2 or C2 and C3;
   Ab represents a cysteine-engineered antibody mutant (THIOMAB™) selected from the group consisiting of:
      (a) LC K149C cysteine-engineered antibody mutant (THIOMAB™);
      (b) HC A140C cysteine-engineered antibody mutant (THIOMAB™);
      (c) LC V205C cysteine-engineered antibody mutant (THIOMAB™); and
      (d) HC S239C cysteine-engineered antibody mutant (THIOMAB™);
         wherein the groups Y, R^{L1}, R^{L2}, R², R⁶, R⁷, R⁹, Q, R¹¹, X, X', R", R¹²,R¹⁶, , R¹⁹ are as defined in any one of clauses 1 to 22.
32. The conjugate of clause 31, wherein the antibody mutant is an antibody for a tumour-associated antigen.
33. The conjugate of clause 32 wherein the antibody mutant is an antibody which binds to one or more tumor-associated antigens or cell-surface receptors selected from (1)-(53):
   (1) BMPR1 B (bone morphogenetic protein receptor-type IB);
   (2) E16 (LAT1, SLC7A5);
   (3) STEAP1 (six transmembrane epithelial antigen of prostate);
   (4) 0772P (CA125, MUC16);
   (5) MPF (MPF, MSLN, SMR, megakaryocyte potentiating factor, mesothelin);
   (6) Napi3b (NAPI-3B, NPTIIb, SLC34A2, solute carrier family 34 (sodium phosphate), member 2, type II sodium-dependent phosphate transporter 3b);
   (7) Sema 5b (FLJ10372, KIAA1445, Mm.42015, SEMA5B, SEMAG, Semaphorin 5b Hlog, sema domain, seven thrombospondin repeats (type 1 and type 1-like), transmembrane domain (TM) and short cytoplasmic domain, (semaphorin) 5B);
   (8) PSCA hlg (2700050C12Rik, C530008O16Rik, RIKEN cDNA 2700050C12, RIKEN cDNA 2700050C12 gene);
   (9) ETBR (Endothelin type B receptor);
   (10) MSG783 (RNF124, hypothetical protein FLJ20315);
   (11) STEAP2 (HGNC_8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein);
   (12) TrpM4 (BR22450, FLJ20041, TRPM4, TRPM4B, transient receptor potential cation channel, subfamily M, member 4);
   (13) CRIPTO (CR, CR1, CRGF, CRIPTO, TDGF1, teratocarcinoma-derived growth factor);
   (14) CD21 (CR2 (Complement receptor 2) or C3DR (C3d/Epstein Barr virus receptor) or Hs 73792);
   (15) CD79b (CD79B, CD79β, IGb (immunoglobulin-associated beta), B29);
   (16) FcRH2 (IFGP4, IRTA4, SPAP1A (SH2 domain containing phosphatase anchor protein 1 a), SPAP1B, SPAP1C);
   (17) HER2;
   (18) NCA;
   (19) MDP;
   (20) IL20Rα;
   (21) Brevican;
   (22) EphB2R;
   (23) ASLG659;
   (24) PSCA;
   (25) GEDA;
   (26) BAFF-R (B cell -activating factor receptor, BLyS receptor 3, BR3);
   (27) CD22 (B-cell receptor CD22-B isoform);
   (28) CD79a (CD79A, CD79β, immunoglobulin-associated alpha);
   (29) CXCR5 (Burkitt's lymphoma receptor 1);
   (30) HLA-DOB (Beta subunit of MHC class II molecule (Ia antigen));
   (31) P2X5 (Purinergic receptor P2X ligand-gated ion channel 5);
   (32) CD72 (B-cell differentiation antigen CD72, Lyb-2);
   (33) LY64 (Lymphocyte antigen 64 (RP105), type I membrane protein of the leucine rich repeat (LRR) family);
   (34) FcRH1 (Fc receptor-like protein 1);
   (35) IRTA2 (Immunoglobulin superfamily receptor translocation associated 2); and
   (36) TENB2 (putative transmembrane proteoglycan)
   (37) PMEL17 (silver homolog; SILV; D12S53E; PMEL17; SI; SIL);
   (38) TMEFF1 (transmembrane protein with EGF-like and two follistatin-like domains 1; Tomoregulin-1);
   (39) GDNF-Ra1 (GDNF family receptor alpha 1; GFRA1; GDNFR; GDNFRA; RETL1; TRNR1; RET1L; GDNFR-alpha1; GFR-ALPHA-1);
   (40) Ly6E (lymphocyte antigen 6 complex, locus E; Ly67,RIG-E,SCA-2,TSA-1);
   (41) TMEM46 (shisa homolog 2 (Xenopus laevis); SHISA2);
   (42) Ly6G6D (lymphocyte antigen 6 complex, locus G6D; Ly6-D, MEGT1);
   (43) LGR5 (leucine-rich repeat-containing G protein-coupled receptor 5; GPR49, GPR67);
   (44) RET (ret proto-oncogene; MEN2A; HSCR1; MEN2B; MTC1; PTC; CDHF12; Hs.168114; RET51; RET-ELE1);
   (45) LY6K (lymphocyte antigen 6 complex, locus K; LY6K; HSJ001348; FLJ35226);
   (46) GPR19 (G protein-coupled receptor 19; Mm.4787);
   (47) GPR54 (KISS1 receptor; KISS1R; GPR54; HOT7T175; AXOR12);
   (48) ASPHD1 (aspartate beta-hydroxylase domain containing 1; LOC253982);
   (49) Tyrosinase (TYR; OCAIA; OCA1A; tyrosinase; SHEP3);
   (50) TMEM118 (ring finger protein, transmembrane 2; RNFT2; FLJ14627);
   (51) GPR172A (G protein-coupled receptor 172A; GPCR41; FLJ11856; D15Ertd747e);
   (52) CD33; and
   (53) CLL-1.
35. The conjugate of either clause 33 or clause 34 wherein the antibody mutant is an antibody which is anti-HER2 4D5, anti-CD22, anti-CD33, anti-Napi3b, anti-HER2 7C2, or anti-CLL-1 antibody.
35. The conjugate of according to clause 31 wherein the drug loading (p) of drugs (D) to antibody (Ab) is an integer from 1 to about 8.
36. The conjugate of according to clause 35, wherein p is 1, 2, 3, or 4.
37. A composition comprising a mixture of the antibody-drug conjugate compounds according to any one of clauses 31 to 36, wherein the average drug loading per antibody in the mixture of antibody-drug conjugate compounds is about 2 to about 5.
38. The conjugate according to any one of clauses 31 to 36 or the composition according to clause 37, for use in therapy.
39. The conjugate according to any one of clauses 31 to 36 or the composition according to clause 37, for use in the treatment of a proliferative disease in a subject.
40. The conjugate or composition according to clause 39, wherein the disease is cancer.
41. A pharmaceutical composition comprising the conjugate according to any one of clauses 31 to 36 or the composition according to clause 37, and a pharmaceutically acceptable diluent, carrier or excipient.
42. The pharmaceutical composition of clause 41 further comprising a therapeutically effective amount of a chemotherapeutic agent.
43. Use of a conjugate according to any one of clauses 31 to 36 or a composition according to clause 37 in the preparation of a medicament for use in the treatment of a proliferative disease in a subject.
44. A method of treating cancer comprising administering to a patient the pharmaceutical composition of clause 43.
45. The method of clause 44 wherein the patient is administered a chemotherapeutic agent, in combination with the conjugate or composition.

## Claims

1. A conjugate of formula CON 1: 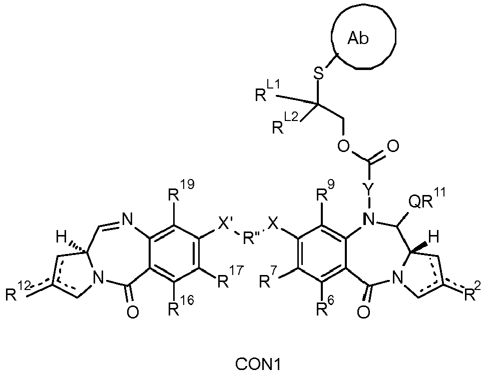
wherein the dotted lines indicate the optional presence of a double bond between C1 and C2 or C2 and C3;
Ab represents a cysteine-engineered antibody mutant (THIOMAB™) which binds to CLL-1 selected from the group consisiting of:
(a) LC K149C cysteine-engineered antibody mutant (THIOMAB™);
(b) HC A140C cysteine-engineered antibody mutant (THIOMAB™);
(c) LC V205C cysteine-engineered antibody mutant (THIOMAB™); and
(d) HC S239C cysteine-engineered antibody mutant (THIOMAB™);
wherein R² is independently selected from H, OH, =O, =CH₂, CN, R, OR, =CH-R^{D}, =C(R^{D})₂, O-SO₂-R, CO₂R and COR, and optionally further selected from halo or dihalo;
where R^{D} is independently selected from R, CO₂R, COR, CHO, CO₂H, and halo;
R⁶ and R⁹ are independently selected from H, R, OH, OR, SH, SR, NH₂, NHR, NRR', NO₂, Me₃Sn and halo;
R⁷ is independently selected from H, R, OH, OR, SH, SR, NH₂, NHR, NRR', NO₂, Me₃Sn and halo;
Y is selected from a single bond, and a group of formulae A1 or A2: 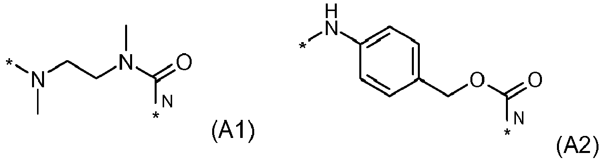
where N shows where the group binds to the N10 of the PBD moiety;
R^{L1} and R^{L2} are independently selected from H and methyl, or together with the carbon atom to which they are bound form a cyclopropylene group;
Q is independently selected from O, S and NH;
R¹¹ is either H, or R or, where Q is O, SO₃M, where M is a metal cation;
R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
wherein R¹², R¹⁶, R¹⁹ and R¹⁷ are as defined for R², R⁶, R⁹ and R⁷ respectively;
wherein R" is a C₃₋₁₂ alkylene group, which chain may be interrupted by one or more heteroatoms, e.g. O, S, N(H), NMe and/or aromatic rings, e.g. benzene or pyridine, which rings are optionally substituted; and
X and X' are independently selected from O, S and N(H).

2. The conjugate according to claim 1, wherein
(a) R^{L1} and R^{L2} are both H; or
(b) R^{L1} and R^{L2} are both methyl; or
(c) one of R^{L1} and R^{L2} is H and the other is methyl.

3. The conjugate according to either claim 1 or claim 2, wherein Y is a single bond.

4. The conjugate according to any one of claims 1 to 3, wherein R⁹ and R¹⁹ are H, R⁶ and R¹⁶ are H, R⁷ are R¹⁷ are both OR^{7A}, where R^{7A} is optionally substituted C₁₋₄ alkyl.

5. The conjugate according to any one of claims 1 to 4, wherein X is O.

6. The conjugate according to any one of claims 1 to 5, wherein R¹¹ is H.

7. The conjugate according to any one of claims 1 to 6, wherein there is a double bond between C2 and C3 in each monomer unit.

8. The conjugate according to claim 7, wherein R² and R¹² are independently selected from H and R.

9. The conjugate according to claim 8, wherein R² and R¹² are independently optionally substituted C₅₋₂₀ aryl.

10. The conjugate according to any one of claims 1 to 6, wherein R² and R¹² are =CH₂.

11. The conjugate according to any one of claims 1 to 10, wherein R" is a C₃ alkylene group or a C₅ alkylene group.

12. A conjugate of formula CON2: 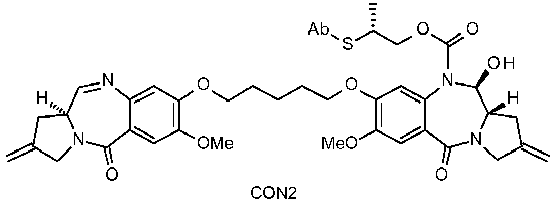 Ab represents a HC A118C or LC K149C cysteine-engineered antibody mutant (THIOMAB™) which binds to CLL-1.

13. The conjugate according to any one of claims 1 to 12 for use in therapy.

14. The conjugate according to any one of claims 1 to 12 for use in the treatment of a proliferative disease in a subject.

15. The conjugate according to claim 14, wherein the disease is cancer.

16. A pharmaceutical composition comprising the conjugate according to any one of claims 1 to 12, and a pharmaceutically acceptable diluent, carrier or excipient.

17. The pharmaceutical composition of claim 16 further comprising a therapeutically effective amount of a chemotherapeutic agent.


SEQUENCE LISTING

<110>  Genentech, Inc.

<120>  PYRROLOBENZODIAZEPINES AND ANTIBODY DISULFIDE CONJUGATES THEREOF

<130>  065435-9247-WO00

<140>  New PCT International Patent Application
<141>  2015-09-17

<150>  US 62/051,387
<151>  2014-09-17

<160>  5

<170>  PatentIn version 3.3

<210>  1
<211>  11
<212>  PRT
<213>  Artificial

<220>
<223>  Synthetic Peptide (FIG. 1a, LC K149C)

<400>  1

Ala Lys Val Gln Trp Cys Val Asp Asn Ala Leu
1               5                   10


<210>  2
<211>  11
<212>  PRT
<213>  Artificial

<220>
<223>  Synthetic Peptide (FIG. 1b, LC V205C)

<400>  2

Gly Leu Ser Ser Pro Cys Thr Lys Ser Phe Asn
1               5                   10


<210>  3
<211>  11
<212>  PRT
<213>  Artificial

<220>
<223>  Synthetic Peptide (FIG. 1c, HC A140C)

<400>  3

Thr Ser Gly Gly Thr Cys Ala Leu Gly Cys Leu
1               5                   10


<210>  4
<211>  11
<212>  PRT

<213>  Artificial

<220>
<223>  Synthetic Peptide (FIG. 1d, HC S239C)

<400>  4

Leu Leu Gly Gly Pro Cys Val Phe Leu Phe Pro
1               5                   10


<210>  5
<211>  21
<212>  PRT
<213>  Artificial

<220>
<223>  Synthetic Peptide

<400>  5

Asn Ala Val Pro Asn Leu Arg Gly Asp Leu Gln Val Leu Ala Gln Lys
1               5                   10                  15


Val Ala Arg Thr Cys
            20